# EUROPEAN PATENT SPECIFICATION

(11) **EP 4 174 062 B1**
(45) Date of publication and mention of the grant of the patent: **18.03.2026**
(21) Application number: 21832185.9
(22) Date of filing: 29.06.2021
(51) Int. Cl.: C07D 405/14, C09K 11/06, C07D 209/86, C07D 487/04, H10K 50/11, H10K 101/30, H10K 101/40, H10K 85/60

(54) **ORGANIC ELECTROLUMINESCENT ELEMENT**
ORGANISCHES ELEKTROLUMINESZENZELEMENT
ÉLÉMENT ÉLECTROLUMINESCENT ORGANIQUE

(30) Priority: 30.06.2020 JP 2020112859
(43) Date of publication of application: 03.05.2023
(73) Proprietor: NIPPON STEEL Chemical & Material Co., Ltd., Tokyo 103-0027 (JP)
(72) Inventor: KAI Takahiro, Tokyo 103-0027 (JP); HAYASHI Kentaro, Tokyo 103-0027 (JP); OGAWA Junya, Tokyo 103-0027 (JP); IKENAGA Yuji, Tokyo 103-0027 (JP); TERADA Ayaka, Tokyo 103-0027 (JP); YOSHIDA Kazunari, Tokyo 103-0027 (JP); KITAHARA Ikumi, Tokyo 103-0027 (JP); IZUMI Haruka, Tokyo 103-0027 (JP); NOGUCHI Katsuhide, Tokyo 103-0027 (JP); KAWADA Atsushi, Tokyo 103-0027 (JP)
(74) Representative: Vossius & Partner Patentanwälte Rechtsanwälte mbB
(86) International application number: PCT/JP2021/024526
(87) International publication number: WO 2022/004709

(56) References cited:
- WO-A1-2016/158191
- WO-A1-2018/198844
- CN-A- 110 492 009
- KR-A- 20200 017 727
- KR-B1- 102 054 806
- US-A1- 2014 374 728
- US-A1- 2015 041 785
- US-A1- 2017 098 778
- US-A1- 2017 098 778
- LI YILANG ET AL: "High efficiency red phosphorescent organic light-emitting diodes with low dopant concentration, low roll-off and long lifetime based on a novel host material with thermally activated delayed fluorescent properties", ORGANIC ELECTRONICS, ELSEVIER, AMSTERDAM, NL, vol. 57, 13 February 2018 (2018-02-13), pages 53 - 59, XP085378284, ISSN: 1566-1199, DOI: 10.1016/J.ORGEL.2018.02.017

## Description

### Technical Field

The present invention relates to an organic electroluminescent device (organic EL device).

### Background Art

Application of a voltage to an organic electroluminescent device allows injection of holes and electrons from an anode and a cathode, respectively, into a light-emitting layer. Then, in the light-emitting layer, injected holes and electrons recombine to generate excitons. At this time, according to statistical rules of electron spins, singlet excitons and triplet excitons are generated at a ratio of 1:3. Regarding a fluorescence-emitting organic electroluminescent device using light emission from singlet excitons, it is said that the internal quantum efficiency thereof has a limit of 25%. Meanwhile, regarding a phosphorescent organic electroluminescent device using light emission from triplet excitons, it is known that intersystem crossing is efficiently performed from singlet excitons, the internal quantum efficiency is enhanced to 100%.

Recently, highly efficient organic electroluminescent devices utilizing delayed fluorescence have been developed. There is known, for example, an organic electroluminescent device utilizing a TTF (Triplet-Triplet Fusion) mechanism, which is one of delayed fluorescence mechanisms. The TTF mechanism utilizes a phenomenon in which singlet excitons are generated due to collision of two triplet excitons, and it is thought that the internal quantum efficiency can be theoretically raised to 40%. However, since the efficiency is lower compared to phosphorescent organic electroluminescent devices, further improvement in efficiency is required.

Patent Literature 1 discloses an organic electroluminescent device utilizing a TADF (Thermally Activated Delayed Fluorescence) mechanism. The TADF mechanism utilizes a phenomenon in which reverse intersystem crossing from triplet excitons to singlet excitons is generated in a material having a small energy difference between a singlet level and a triplet level, and it is thought that the internal quantum efficiency can be theoretically raised to 100%. Devices utilizing fluorescent materials or phosphorescent materials, and devices utilizing TADF mechanism, which are currently in practical use, are respectively demanded to be further enhanced in efficiency characteristics, voltage characteristics and driving lifetime, and demanded to realize characteristics at practical levels.

### Citation List

### Patent Literature

Patent Literature 1: WO2011/070963A
Patent Literature 2: WO2013/062075A
Patent Literature 3: US2014/0374728A
Patent Literature 4: WO2011/136755A
Patent Literature 5: WO2016/194604A
Patent Literature 6: WO2018/0198844A
Patent Literature 7: US2017/098778A
Patent Literature 8: KR2020-017727A

### Non Patent Literature

Non Patent Literature 1: Adv. Mater, 2011, 23, 3590-3596
Patent Literatures 2 and 3 disclose use of a biscarbazole compound as a mixed host material.
Patent Literature 4 discloses use of a host material in which a plurality of host materials containing an indolocarbazole compound is premixed.
Patent Literatures 5 and 6 disclose use of a mixed host material containing an indolocarbazole compound and a biscarbazole compound.
Patent Literatures 7 and 8, and Non Patent Literature 1 disclose use of a mixed host material containing three host materials.

However, none of these can be said to be sufficient, and further improvement is desired.

### Summary of Invention

In order to apply organic electroluminescent devices to display devices such as flat panel displays, or light sources, it is necessary to improve the driving voltage and the luminous efficiency of devices and at the same time, to sufficiently secure the stability during driving. An object of the present invention is to provide an organic electroluminescent device exhibiting long driving lifetime characteristics while having a low driving voltage and high efficiency, and a material for organic electroluminescent device suitable therefor.

It is effective for enhancements in lifetime characteristics of organic electroluminescent devices to not only enhance the stability in a more active state than the ground state, for example, the charged state or excited state of materials used, but also reduce the frequency of the active state per molecule, namely, to disperse the active state to many molecules. It is important particularly for dispersion of the excited state to suitably adjust the injection ability and the transport ability of electrons and holes into light-emitting layers, namely, adjust the charge balance. Furthermore, the charge balance is adjusted to thereby reduce leakage of electrons, holes and excitons to surrounding layers, also leading to an enhancement in lifetime and furthermore allowing the effect of enhancing efficiency to be obtained.

In order to realize them, a procedure is currently adopted mainly for a phosphorescent layer, in which the charge balance is optimized by mixing two host materials of a combination of an electron injecting/transporting host and a hole injecting/transporting host, and adjusting the mixing ratio. On the other hand, mixing of two host materials, while results in enhancements in lifetime characteristics, has the problem of causing deterioration in charge injection/transport ability and thus an increase in driving voltage.

As a result of intensive studies, the present inventors have found that use of a specific first host material, a second host material, and a third host material provides an organic electroluminescent device exhibiting excellent characteristics, and have completed the present invention.

The present invention is an organic electroluminescent device including one or more light-emitting layers between an anode and a cathode opposed to each other, wherein at least one of the light-emitting layers contains a host material including a first host material, a second host material and a third host material, and a light-emitting dopant material, the LUMO energy of the first host material is -1.95 eV or less, the LUMO energy of the second host material is -1.54 eV or more, the LUMO energy of the third host material is -1.94 to -1.77 eV and LM2 ≥ LM3 ≥ LM1 is satisfied under the assumption that LUMO energies of the first host material, the second host material and the third host material are LM1, LM2 and LM3, respectively.

In a preferred aspect, the triplet excitation (T1) energies of the first host material, the second host material and the third host material are each 2.55 eV or more.

In a preferred aspect, at least three materials of the first host material, the second host material and the third host material, and the light-emitting dopant material are vapor deposited from one vapor deposition source, or the first host material, the second host material and the third host material are vapor deposited from one vapor deposition source.

Examples of the light-emitting dopant material include a material emitting fluorescence including thermally activated delayed fluorescence, or a phosphorescence-emitting material.

The first host material can be a compound represented by the following general formula (1).

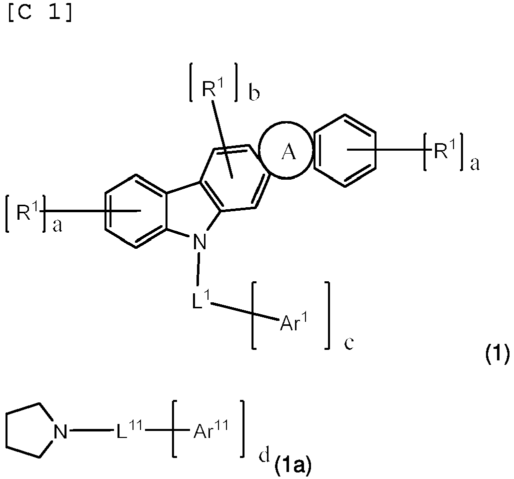

In the formula, a ring A is a heterocycle represented by formula (1a) and the ring A is fused to an adjacent ring at any position,
R¹ independently represents deuterium, an aliphatic hydrocarbon group having 1 to 10 carbon atoms, a substituted or unsubstituted aromatic hydrocarbon group having 6 to 30 carbon atoms, a substituted or unsubstituted aromatic heterocyclic group having 3 to 17 carbon atoms, or a substituted or unsubstituted linked aromatic group in which two to five of these aromatic rings are linked to each other,
a, c and d each independently represent an integer of 0 to 4, and b represents an integer of 0 to 2, and L¹ and L¹¹ independently represent a substituted or unsubstituted aromatic hydrocarbon group having 6 to 30 carbon atoms, or a substituted or unsubstituted aromatic heterocyclic group having 3 to 17 carbon atoms, and at least one thereof represents the aromatic heterocyclic group.
Ar¹ and Ar¹¹ each independently represent a substituted or unsubstituted aromatic hydrocarbon group having 6 to 30 carbon atoms, a substituted or unsubstituted aromatic heterocyclic group having 3 to 17 carbon atoms, or a substituted or unsubstituted linked aromatic group in which two to seven of these aromatic rings are linked to each other.

It is preferred that at least one of L¹ and L¹¹ represents a substituted or unsubstituted nitrogen-containing 6-membered ring group, or a substituted or unsubstituted ring-fused aromatic heterocyclic group containing a nitrogen-containing 6-membered ring.

The compound represented by the general formula (1) is a compound represented by the following formula (11), formula (12) or formula (13).

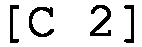

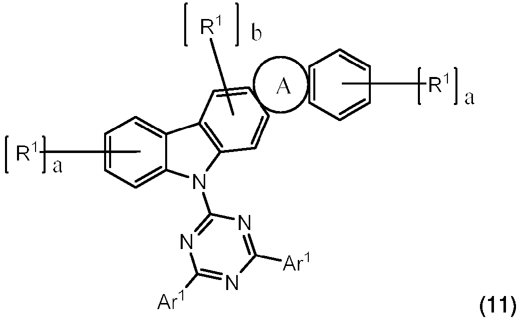

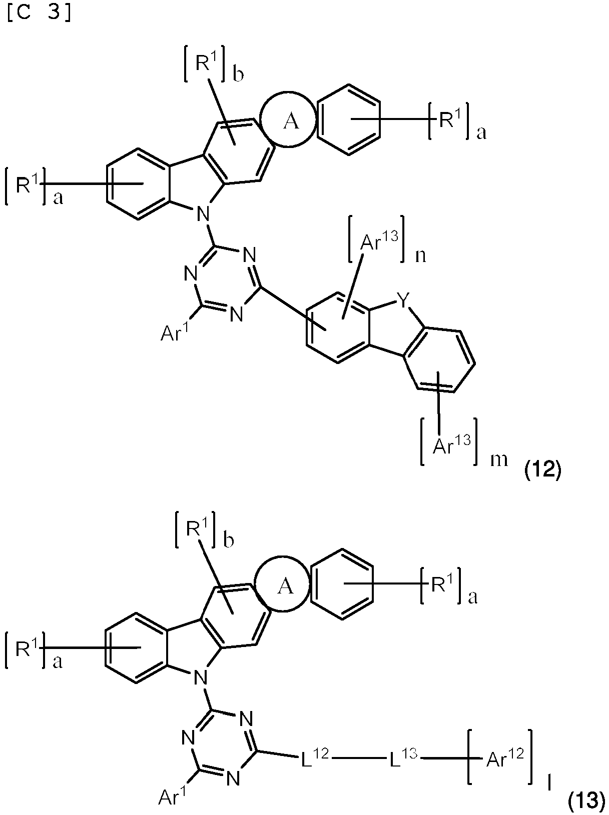

In the formulas, R¹, a ring A, Ar¹, a and b are as defined for the general formula (1),
Y represents O, S, NAr¹⁴, or CAr¹⁵Ar¹⁶,
Ar¹³, Ar¹⁴, Ar¹⁵, and Ar¹⁶ each independently represent an aliphatic hydrocarbon group having 1 to 10 carbon atoms, a substituted or unsubstituted aromatic hydrocarbon group having 6 to 30 carbon atoms, a substituted or unsubstituted aromatic heterocyclic group having 3 to 17 carbon atoms, or a substituted or unsubstituted linked aromatic group in which two to five of these aromatic rings are linked to each other, and
m represents an integer of 0 to 4, n represents an integer of 0 to 3, and 1 represents an integer of 0 to 4. L¹² represents a substituted or unsubstituted aromatic hydrocarbon group having 6 to 30 carbon atoms,
L¹³ represents a nitrogen-containing 6-membered ring group, and
Ar¹² independently represents a substituted or unsubstituted aromatic hydrocarbon group having 6 to 30 carbon atoms, a substituted or unsubstituted aromatic heterocyclic group having 3 to 17 carbon atoms, or a substituted or unsubstituted linked aromatic group in which two to five of these aromatic rings are linked to each other.

The second host material can be a compound represented by the following general formula (2), general formula (3), or general formula (4).

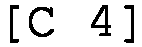

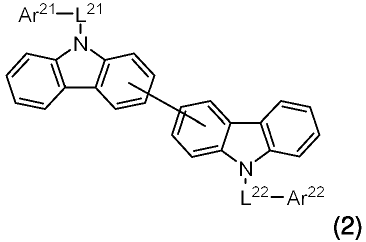

In the formula, Ar²¹ and Ar²² independently represent hydrogen, deuterium, a substituted or unsubstituted aromatic hydrocarbon group having 6 to 14 carbon atoms, an aromatic heterocyclic group having 3 to 17 carbon atoms, or a substituted or unsubstituted linked aromatic group in which two of these aromatic rings are linked to each other. L²¹ and L²² independently represent a direct bond or a phenylene group.

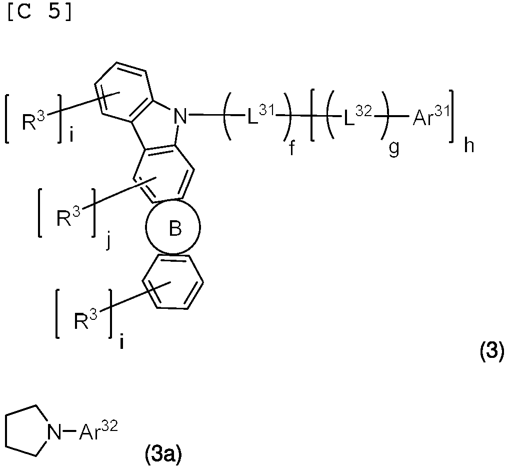

In the formula, a ring B is a heterocycle represented by formula (3a) and the ring B is fused to an adjacent ring at any position,
R³ independently represents deuterium, an aliphatic hydrocarbon group having 1 to 10 carbon atoms, a substituted or unsubstituted aromatic hydrocarbon group having 6 to 30 carbon atoms, a substituted or unsubstituted aromatic heterocyclic group having 3 to 17 carbon atoms, or a substituted or unsubstituted linked aromatic group in which two to five of these aromatic rings are linked to each other,
L³¹ independently represents a substituted or unsubstituted aromatic hydrocarbon group having 6 to 30 carbon atoms, or a substituted or unsubstituted aromatic heterocyclic group having 3 to 17 carbon atoms,
L³² independently represents a substituted or unsubstituted aromatic hydrocarbon group having 6 to 10 carbon atoms,
Ar³¹ represents a substituted or unsubstituted aromatic hydrocarbon group having 6 to 10 carbon atoms, a substituted or unsubstituted carbazolyl group, or a substituted or unsubstituted linked aromatic group in which two to five of these aromatic rings are linked to each other,
Ar³² represents a substituted or unsubstituted aromatic hydrocarbon group having 6 to 30 carbon atoms, a substituted or unsubstituted aromatic heterocyclic group having 3 to 17 carbon atoms, or a substituted or unsubstituted linked aromatic group in which two to five of these aromatic rings are linked to each other,
i represents an integer of 0 to 4, and j represents an integer of 0 to 2,
f is the number of repetitions, and represents an integer of 1 to 3, g is the number of repetitions and independently represents an integer of 0 to 3, and h represents the number of substitutions and represents an integer of 0 to 7.

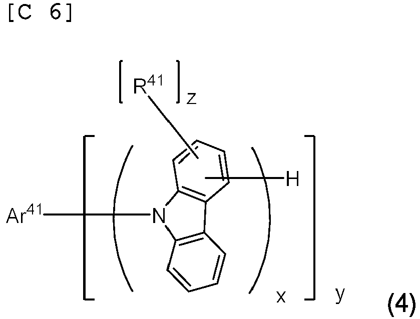

In the formula, Ar⁴¹ represents a substituted or unsubstituted aromatic hydrocarbon group having 6 to 30 carbon atoms, a substituted or unsubstituted aromatic heterocyclic group having 3 to 17 carbon atoms, or a linked aromatic group in which two to five of these aromatic rings are linked. When Ar⁴¹ has a hydrogen atom, the hydrogen atom may be replaced by deuterium.

R⁴¹ each independently represents, deuterium, an aliphatic hydrocarbon group having 1 to 10 carbon atoms, a substituted or unsubstituted aromatic hydrocarbon group having 6 to 30 carbon atoms, or a substituted or unsubstituted aromatic heterocyclic group having 3 to 17 carbon atoms, provided that R⁴¹ is not a carbazolyl group. x is the number of repetitions and independently represents an integer of 1 to 4, provided that at least one x is an integer of 2 to 4. y is the number of substitutions and represents an integer of 1 to 4. When x and y are each 2 or more, a plurality of carbazolyl groups in the formula may be the same or different. z is an integer of 0 to 3.

The third host material can be a compound represented by the following general formula (5).

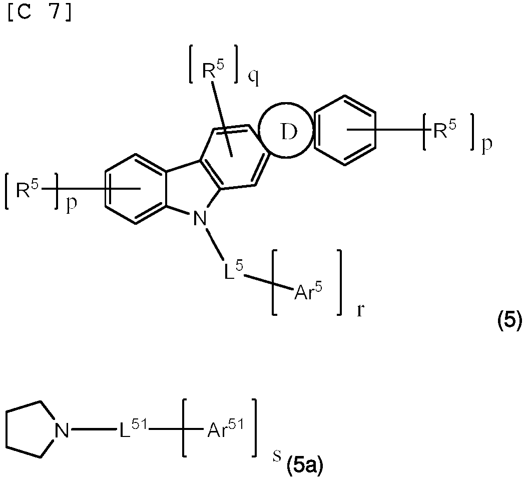

In the formula, a ring D is a heterocycle represented by formula (5a) and the ring D is fused to an adjacent ring at any position,
R⁵ independently represents deuterium, an aliphatic hydrocarbon group having 1 to 10 carbon atoms, a substituted or unsubstituted aromatic hydrocarbon group having 6 to 30 carbon atoms, a substituted or unsubstituted aromatic heterocyclic group having 3 to 17 carbon atoms, or a substituted or unsubstituted linked aromatic group in which two to five of these aromatic rings are linked to each other,
p, r and s each independently represent an integer of 0 to 4, and q represents an integer of 0 to 2,
L⁵ and L⁵¹ represent a substituted or unsubstituted aromatic hydrocarbon group having 6 to 30 carbon atoms, or a substituted or unsubstituted aromatic heterocyclic group having 3 to 17 carbon atoms, and at least one thereof represents the aromatic heterocyclic group.
Ar⁵ and Ar⁵¹ each independently represent a substituted or unsubstituted aromatic hydrocarbon group having 6 to 30 carbon atoms, a substituted or unsubstituted aromatic heterocyclic group having 3 to 17 carbon atoms, or a substituted or unsubstituted linked aromatic group in which two to seven of these aromatic rings are linked to each other.

In a preferred aspect, at least one of L⁵ and L⁵¹ represents a substituted or unsubstituted nitrogen-containing 6-membered ring or ring-fused aromatic heterocyclic group containing a nitrogen-containing 6-membered ring.

The third host material can be a compound represented by the following formula (51).

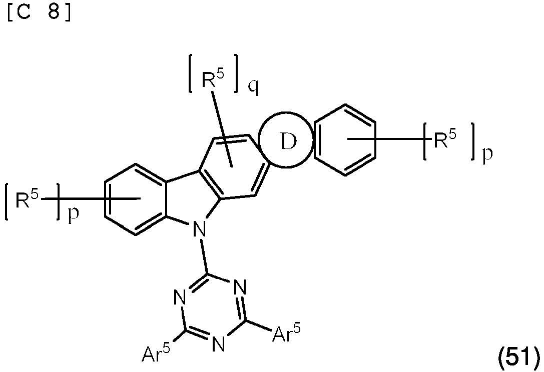

In the formula, R⁵, D, Ar⁵, p, and q are as defined for the general formula (5).

The proportion of the first host material can be larger than 1.0 wt% and less than 30 wt% and the proportion of the third host material can be larger than 5.0 wt% and less than 80 wt% based on the first host material, the second host material and the third host material in total.

The present invention is also a method for producing an organic electroluminescent device including one or more light-emitting layers between an anode and a cathode opposed to each other, at least one of the light-emitting layers including a host material and a light-emitting dopant material, the method including forming each of the light-emitting layers by vapor depositing the light-emitting dopant material and a host material composition in which the host material includes a first host material, a second host material and a third host material and the LUMO energy of the first host material is -1.95 eV or less, the LUMO energy of the second host material is -1.54 eV or more, the LUMO energy of the third host material is -1.94 to -1.77 eV.

Furthermore, the present invention is a host material composition for use in the method for producing an organic electroluminescent device.

Use of a mixture of two host materials, which is currently in practical use mainly for phosphorescence-emitting devices, results in enhancements in lifetime characteristics, but has the problem of causing deterioration in charge injection/transport ability and thus an increase in driving voltage. In other words, lifetime characteristics and driving voltage are often in a trade-off relationship.

On the contrary, the present invention can allow for an improvement in driving voltage with favorable efficiency and lifetime characteristics being kept, by use of a mixed host material of three host materials including a host material having a low LUMO energy.

A conventional method in which two hosts of an electron transporting host and a hole transporting host are mixed for optimization of the charge balance effective for enhancements in lifetime characteristics has caused particularly reductions in transport rates of electrons and holes, resulting in an increase in driving voltage. It is considered that a proper amount of a host material low in LUMO energy can be further mixed to thereby result in an improvement in electron injection ability and a reduction in driving voltage with the charge balance being still optimized and favorable efficiency characteristics and lifetime characteristics being kept.

### Brief Description of Drawing

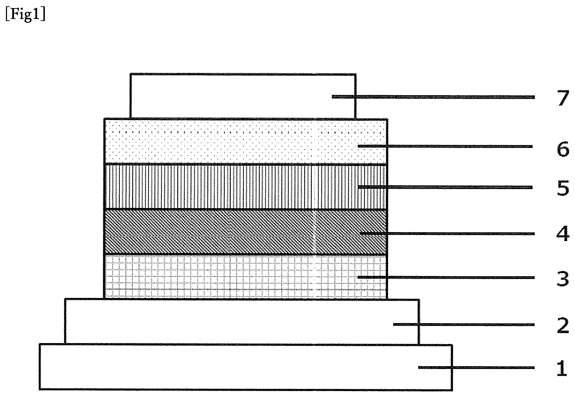

[Figure 1] Figure 1 is a schematic cross-sectional view showing one example of an organic electroluminescent device.

### Description of Embodiments

An organic electroluminescent device of the present invention has an organic layer comprising a plurality of layers between an anode and a cathode opposed to each other; and at least one layer of the plurality of layers is a light-emitting layer, and there may be a plurality of light-emitting layers. Then, at least one of the light-emitting layers contains a first host material, a second host material and a third host material, and a light-emitting dopant material. The light-emitting layer is preferably comprising a vapor deposition layer.

In the present specification, materials including the first host material, the second host material and the third host material herein are collectively referred to as "host material". The material containing the first host material, the second host material and the third host material, used for formation of the light-emitting layer by a vapor deposition method, is referred to as "host material composition".

Under the assumption that the LUMO energies of the first host material, the second host material and the third host material are LM1, LM2 and LM3, respectively, LM2 ≥ LM3 ≥ LM1 is satisfied. LM2 > LM3 > LM1 is preferably satisfied.

The LUMO energy LM1 of the first host material is - 1.95 eV or less. The energy is preferably -1.97 eV or less and further preferably -2.00 eV or less.

The LUMO energy LM2 of the second host material is -1.54 eV or more, and is preferably -1.30 eV or more and further preferably -1.07 eV or more.

The LUMO energy LM3 of the third host material is -1.94 to -1.77 eV, and is preferably -1.94 to -1.83 eV and further preferably -1.94 to -1.87 eV.

The triplet excitation (T1) energies of the first host material, the second host material and the third host material are each preferably 2.55 eV or more, more preferably 2.60 eV or more, further preferably 2.65 eV or more.

The first host material, the second host material and the third host material may satisfy the characteristics, and the following compounds are preferred.

The compound suitable as the first host material is the compound represented by the general formula (1).

In the general formula (1),
Ar¹ and Ar¹¹ each independently represent a substituted or unsubstituted aromatic hydrocarbon group having 6 to 30 carbon atoms, a substituted or unsubstituted aromatic heterocyclic group having 3 to 17 carbon atoms, or a substituted or unsubstituted linked aromatic group in which two to seven of these aromatic rings are linked to each other. Preferred is a substituted or unsubstituted aromatic hydrocarbon group having 6 to 10 carbon atoms, a substituted or unsubstituted aromatic heterocyclic group having 3 to 12 carbon atoms, or a substituted or unsubstituted linked aromatic group in which two to seven of these aromatic rings are linked to each other.

In the case of Ar¹ and Ar¹¹ being each an unsubstituted aromatic hydrocarbon group, an unsubstituted aromatic heterocyclic group or an unsubstituted linked aromatic group, specific examples thereof include a group generated by removing one hydrogen from benzene, pentalene, indene, naphthalene, azulene, heptalene, octalene, indacene, acenaphthylene, phenalene, phenanthrene, anthracene, trindene, fluoranthene, acephenanthrylene, aceanthrylene, triphenylene, pyrene, chrysene, tetraphene, tetracene, pleiadene, picene, perylene, pentaphene, pentacene, tetraphenylene, cholanthrylene, helicene, hexaphene, rubicene, coronene, trinaphthylene, heptaphene, pyranthrene, furan, benzofuran, isobenzofuran, xanthene, oxathrene, dibenzofuran, peri-xanthenoxanthene, thiophene, thioxanthene, thianthrene, phenoxathiin, thionaphthene, isothianaphthene, thiophthene, thiophanthrene, dibenzothiophene, pyrrole, pyrazole, tellurazole, selenazole, thiazole, isothiazole, oxazole, furazan, indolizine, indole, isoindole, indazole, purine, quinolizine, isoquinoline, imidazole, naphthyridine, phthalazine, carbazole, benzodiazepine, quinoxaline, cinnoline, quinoline, pteridine, phenanthridine, acridine, perimidine, phenanthroline, phenazine, carboline, phenotellurazine, phenoselenazine, phenothiazine, phenoxazine, anthyridine, benzothiazole, benzimidazole, benzoxazole, benzisooxazole, benzisothiazole, or a compound formed by linking two to seven of these to each other. Preferred is a group generated from pyridine, pyrimidine, triazine, dibenzofuran, dibenzothiophene, carbazole, benzene, naphthalene, or a compound formed by linking two to seven of these to each other.

R¹ independently represents deuterium, an aliphatic hydrocarbon group having 1 to 10 carbon atoms, a substituted or unsubstituted aromatic hydrocarbon group having 6 to 30 carbon atoms, a substituted or unsubstituted aromatic heterocyclic group having 3 to 17 carbon atoms, or a substituted or unsubstituted linked aromatic group in which two to five of these aromatic rings are linked to each other. Preferred is an aliphatic hydrocarbon group having 1 to 6 carbon atoms, a substituted or unsubstituted aromatic hydrocarbon group having 6 to 10 carbon atoms, or a substituted or unsubstituted aromatic heterocyclic group having 3 to 12 carbon atoms.

When R¹ represents an aliphatic hydrocarbon group, the group may be linear, branched or cyclic, and is preferably an aliphatic hydrocarbon group having 1 to 8 carbon atoms and further preferably an alkyl group having 1 to 6 carbon atoms.

Specific examples of the aliphatic hydrocarbon group include methyl, ethyl, propyl, butyl, t-butyl, pentyl, hexyl, cyclohexyl, heptyl, octyl, nonyl, or decyl. The group is preferably methyl, ethyl, propyl, butyl, t-butyl, pentyl, hexyl, cyclohexyl, heptyl, or octyl, and further preferably methyl, ethyl, propyl, butyl, t-butyl, pentyl, hexyl, and cyclohexyl.

In the case of R¹ being an unsubstituted aromatic hydrocarbon group having 6 to 30 carbon atoms, an unsubstituted aromatic heterocyclic group having 3 to 17 carbon atoms, or an unsubstituted linked aromatic group, specific examples thereof are the same as in the case of Ar¹ and Ar¹¹ except that two to five of the aromatic rings in the linked aromatic group are linked to each other.

a, b, c and d each represent the number of substitutions, a, c and d each independently represent an integer of 0 to 4, preferably an integer of 0 to 3 and further preferably 0 to 1. b represents an integer of 0 to 2, preferably represents 0 to 1.

In the present specification, the linked aromatic group refers to an aromatic group in which respective
aromatic rings of the aromatic group are bound and linked to each other by a single bond. Two or more aromatic groups linked to each other are here intended, and may be each linear or branched. These aromatic groups may be each an aromatic hydrocarbon group or an aromatic heterocyclic group, and may be the same or different.

In the present specification, the above unsubstituted aromatic hydrocarbon group, aromatic heterocyclic group, nitrogen-containing 6-membered ring group, fused aromatic heterocyclic group, carbazolyl group, or linked aromatic group each has a substituent. When the group has a substituent, the substituent is an aliphatic hydrocarbon group having 1 to 10 carbon atoms, an alkoxy group having 1 to 5 carbon atoms, an alkenyl group having 2 to 5 carbon atoms, a diarylamino group having 12 to 44 carbon atoms, deuterium, halogen, or a cyano group.

The number of substituents is 0 to 5 and preferably 0 to 3. When the aromatic hydrocarbon group and the aromatic heterocyclic group each have a substituent, the number of carbon atoms is calculated with not including the number of carbon atoms of the substituent. However, it is preferred to satisfy the above range by the number of carbon atoms including the number of carbon atoms of the substituent.

Specific examples of the substituent include cyano, methyl, ethyl, propyl, butyl, pentyl, hexyl, heptyl, octyl, nonyl, decyl, diphenylamino, naphthylphenylamino, dinaphthylamino, dianthranylamino, diphenanthrenylamino, and dipyrenylamino. Preferred examples include cyano, methyl, ethyl, propyl, butyl, pentyl, hexyl, heptyl, octyl, diphenylamino, naphthylphenylamino, or dinaphthylamino.

It is understood that hydrogen may be deuterium in the present specification. Accordingly, when a skeleton such as an indolocarbazole ring or a carbazole ring, or a group such as R¹, L¹¹ or Ar¹¹ in the general formulas (1) to (5) has hydrogen, such hydrogen may be partially or fully deuterium.

L¹ and L¹¹ independently represent a substituted or unsubstituted aromatic hydrocarbon group having 6 to 30 carbon atoms, or a substituted or unsubstituted aromatic heterocyclic group having 3 to 17 carbon atoms, and at least one thereof represents the aromatic heterocyclic group. It is preferred that at least one of L¹ and L¹¹ represents a substituted or unsubstituted nitrogen-containing 6-membered ring, or a substituted or unsubstituted ring-fused aromatic heterocyclic group containing a nitrogen-containing 6-membered ring.

Specific examples of L¹ and L¹¹ being each an unsubstituted aromatic group or an unsubstituted aromatic heterocyclic group are the same as in the case of Ar¹ and Ar¹¹ except that the group is (c + 1)-valent or (d + 1)-valent. Preferred is a group generated from pyridine, pyrimidine, triazine, quinoline, isoquinoline, quinoxaline, naphthyridine, phenazine, dibenzofuran, dibenzothiophene, carbazole, benzene, or naphthalene.

Specific examples of the unsubstituted nitrogen-containing 6-membered ring group or the unsubstituted ring-fused aromatic heterocyclic group containing a nitrogen-containing 6-membered ring include pyridine, pyrimidine, triazine, quinoline, isoquinoline, quinoxaline, naphthyridine, or phenazine.

Preferred aspects of the general formula (1) include formula (11), and preferred aspects of the formula (11) include formula (12) or formula (13).

In the formula (11) to formula (13), the same symbols as in the general formula (1) have the same meaning.

In other words, R¹, a ring A, Ar¹, a, and b are as defined for the general formula (1).

In the formula (12), Y represents O, S, NAr¹⁴, CAr¹⁵, or Ar¹⁶, preferably O, S, or NAr¹⁴, and further preferably O or S.

Ar¹³, Ar¹⁴, Ar¹⁵, and Ar¹⁶ each independently represent an aliphatic hydrocarbon group having 1 to 10 carbon atoms, a substituted or unsubstituted aromatic hydrocarbon group having 6 to 30 carbon atoms, a substituted or unsubstituted aromatic heterocyclic group having 3 to 17 carbon atoms, or a substituted or unsubstituted linked aromatic group in which two to five of these aromatic rings are linked to each other. Preferred is a substituted or unsubstituted aromatic hydrocarbon group having 6 to 24 carbon atoms, a substituted or unsubstituted aromatic heterocyclic group having 3 to 17 carbon atoms, or a substituted or unsubstituted linked aromatic group in which two to five of these aromatic rings are linked to each other, and more preferred is a substituted or unsubstituted aromatic hydrocarbon group having 6 to 18 carbon atoms, a substituted or unsubstituted aromatic heterocyclic group having 3 to 12 carbon atoms, or a substituted or unsubstituted linked aromatic group in which two to five of these aromatic rings are linked to each other.

In the case of Ar¹³, Ar¹⁴, Ar¹⁵, and Ar¹⁶ being each an unsubstituted aromatic hydrocarbon group, an unsubstituted aromatic heterocyclic group, or an unsubstituted linked aromatic group, specific examples thereof are the same as in the case of Ar¹ and Ar¹¹, provided that two to five aromatic rings are linked to each other in the case of the linked aromatic group. In the case of an aliphatic hydrocarbon group, specific examples thereof are the same as in the case where R¹ is an aliphatic hydrocarbon group.

m represents an integer of 0 to 4, n represents an integer of 0 to 3, m and n are each preferably 0 to 2 and further preferably 0 to 1.

In the formula (13), L¹² represents a substituted or unsubstituted aromatic hydrocarbon group having 6 to 30 carbon atoms, preferably represents a substituted or unsubstituted aromatic hydrocarbon group having 6 to 10 carbon atoms, and further preferably represents a phenylene group represented by any of formula (1b) or formula (1c).

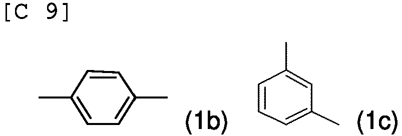

L¹³ represents a nitrogen-containing 6-membered ring group, preferably represents a pyridine group or a triazine group, and further preferably represents a triazine group.

Ar¹² independently represents a substituted or unsubstituted aromatic hydrocarbon group having 6 to 30 carbon atoms, a substituted or unsubstituted aromatic heterocyclic group having 3 to 17 carbon atoms, or a substituted or unsubstituted linked aromatic group in which two to five of these aromatic rings are linked to each other. Preferred is a substituted or unsubstituted aromatic hydrocarbon group having 6 to 10 carbon atoms, a substituted or unsubstituted aromatic heterocyclic group having 3 to 12 carbon atoms, or a substituted or unsubstituted linked aromatic group in which two to five of these aromatic rings are linked to each other.

In the case of Ar¹² being an unsubstituted aromatic hydrocarbon group, an unsubstituted aromatic heterocyclic group, or an unsubstituted linked aromatic group, specific examples thereof are the same as in the case of Ar¹ and Ar¹¹ except that two to five of the aromatic rings in the linked aromatic group are linked to each other.

1 represents an integer of 0 to 4, preferably 0 to 2.

Specific examples of the compounds represented by the general formula (1) are described below, but the compounds are not limited to these exemplified compounds.

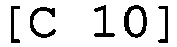

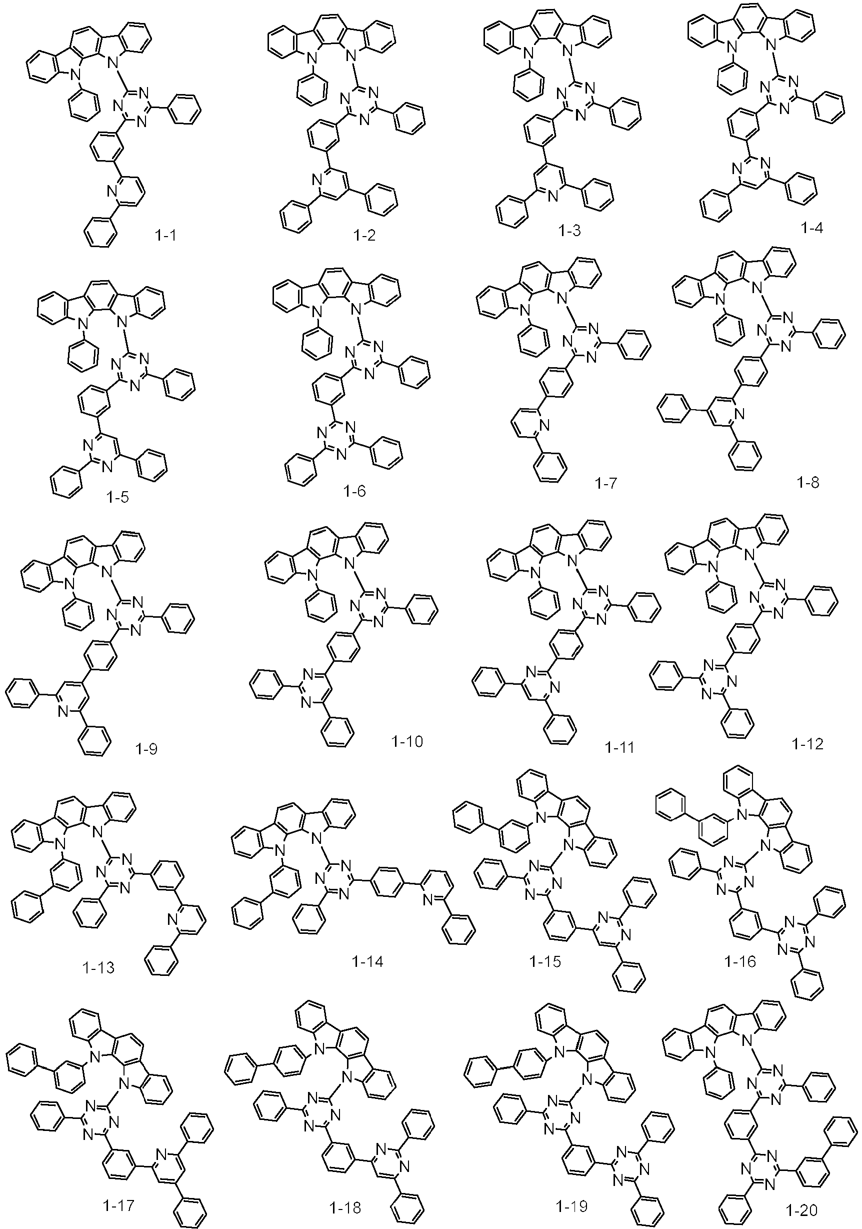

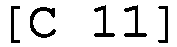

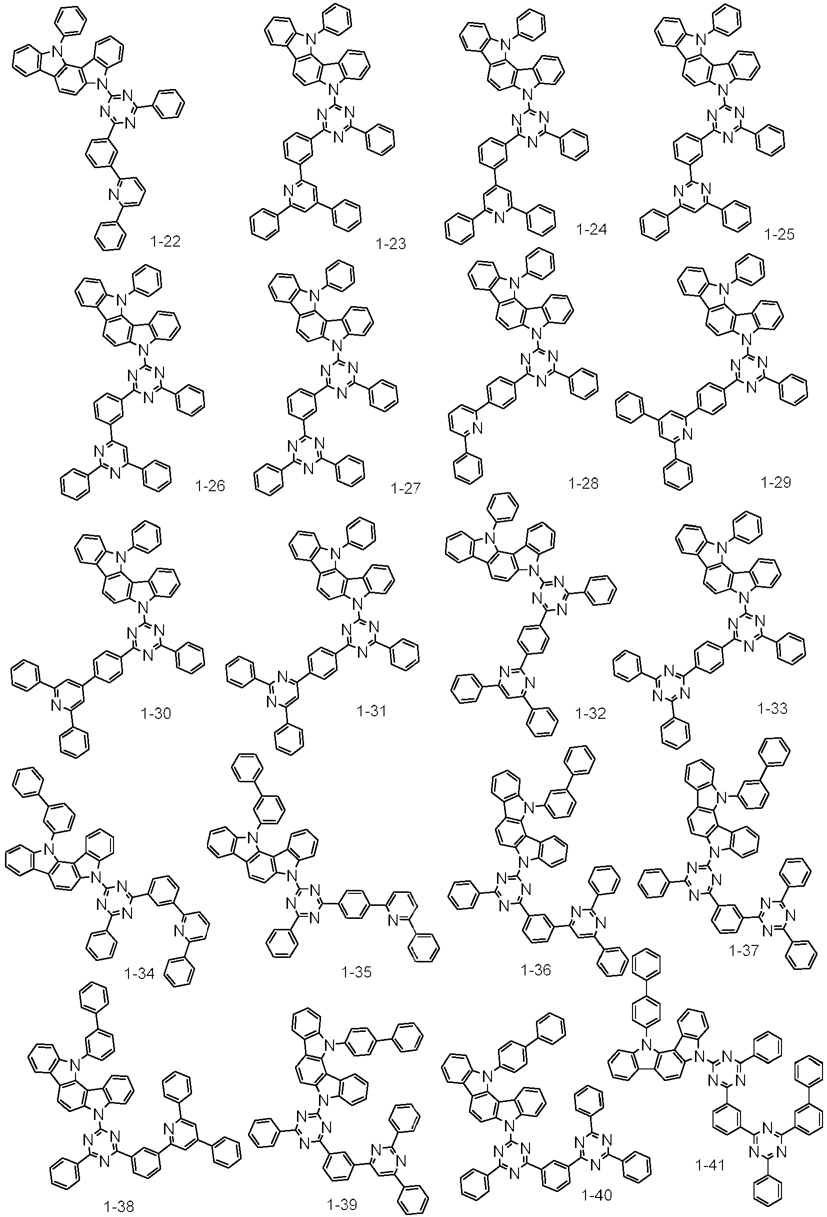

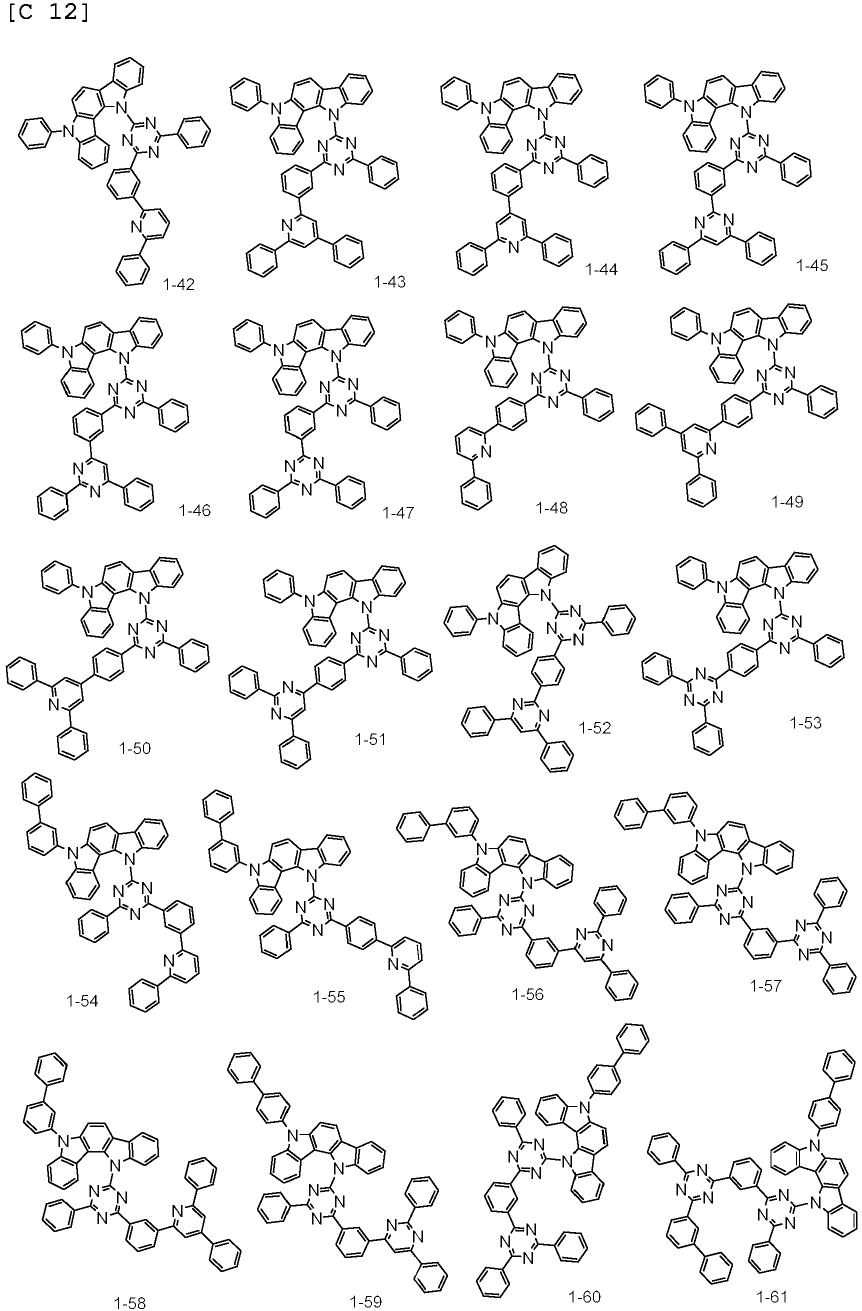

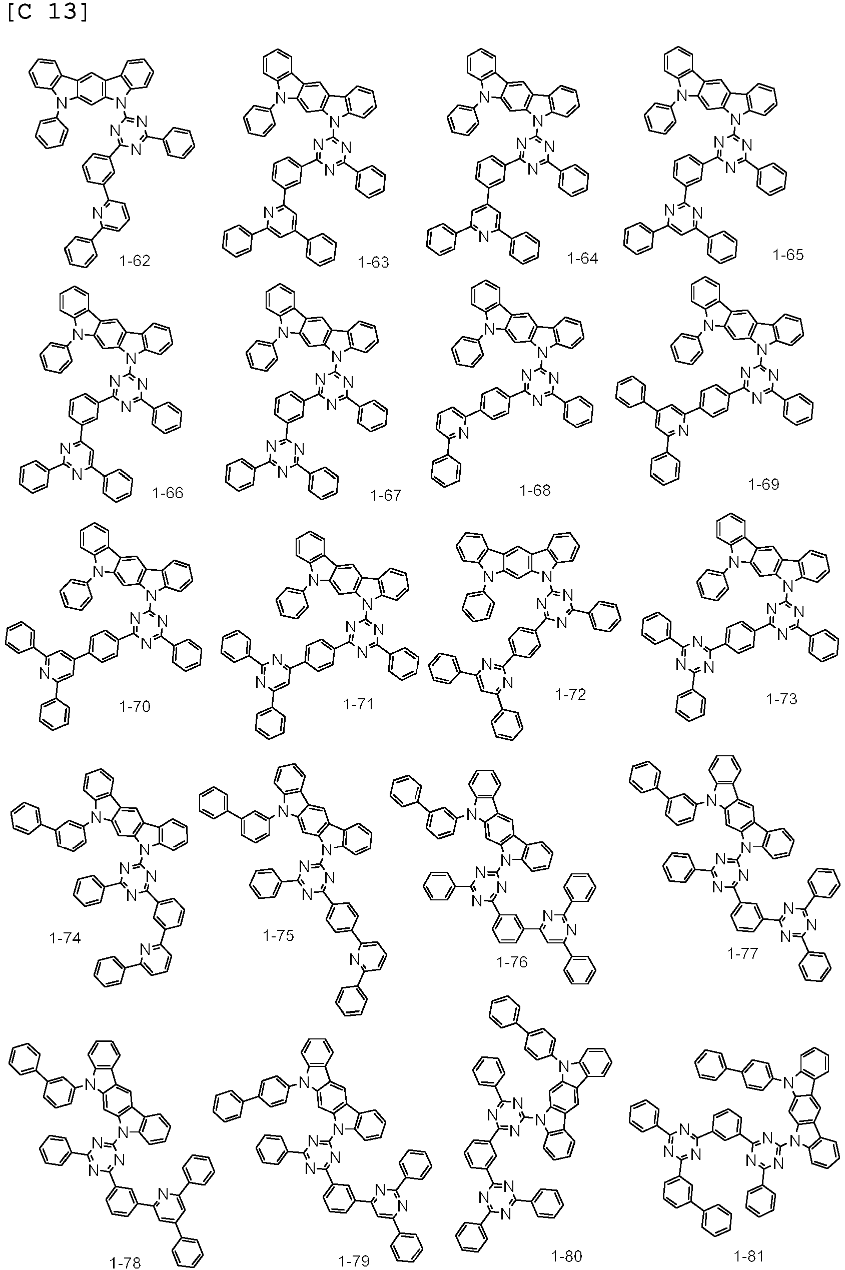

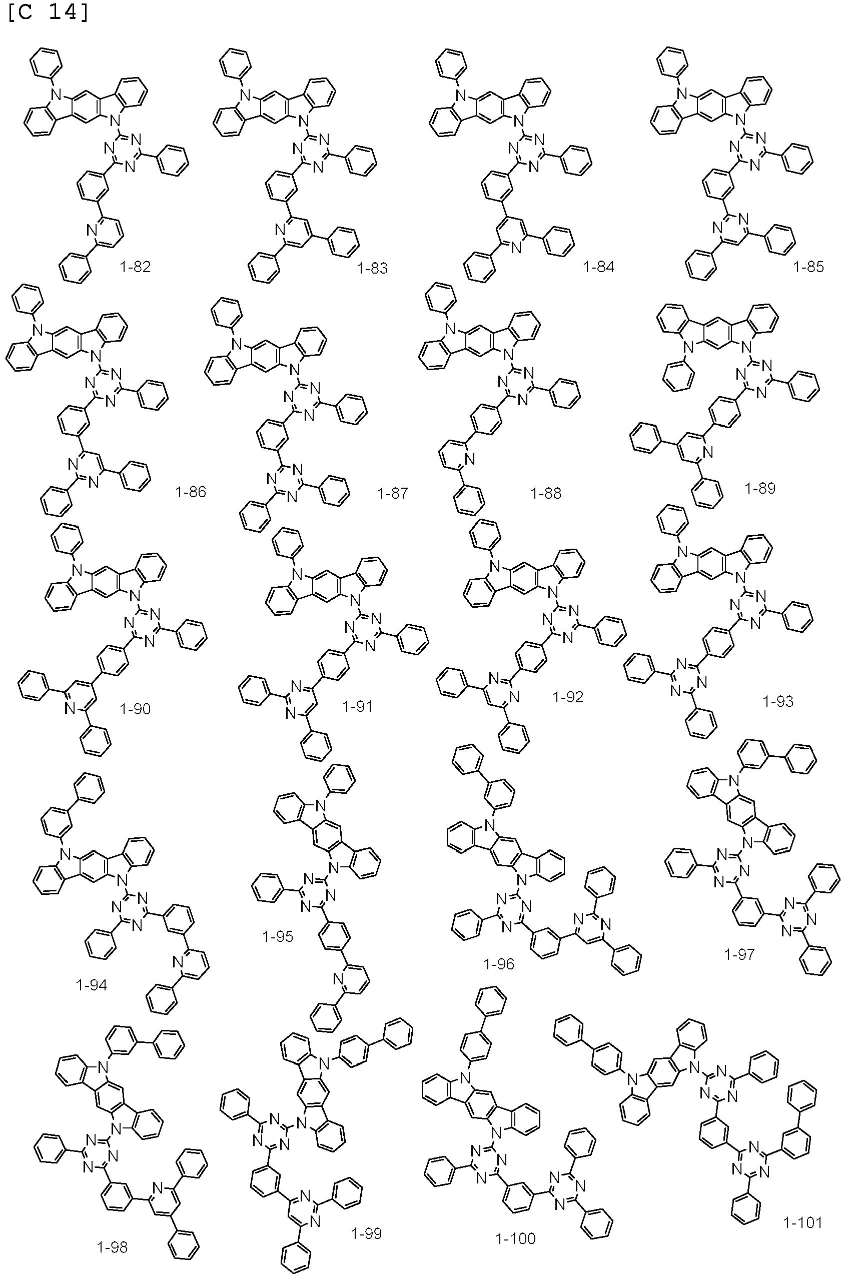

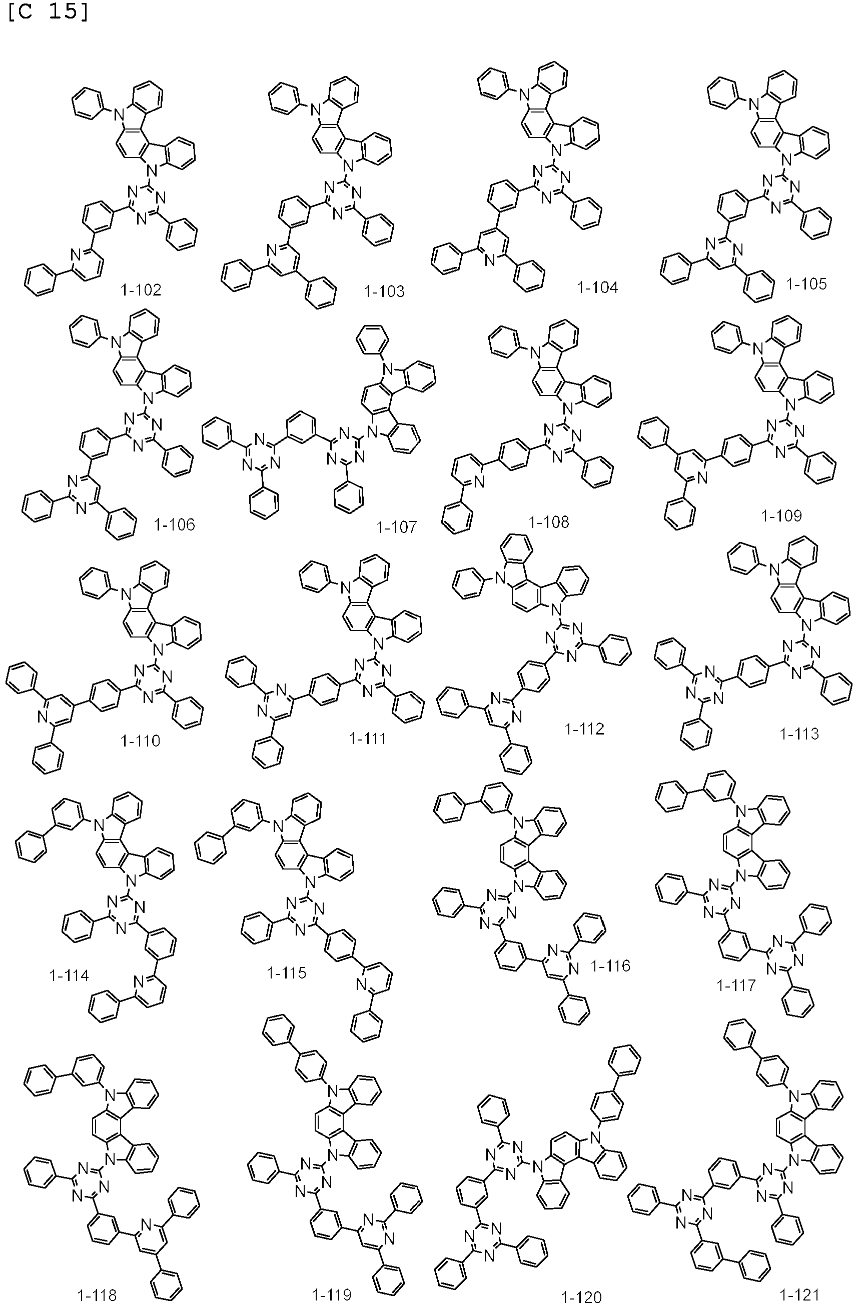

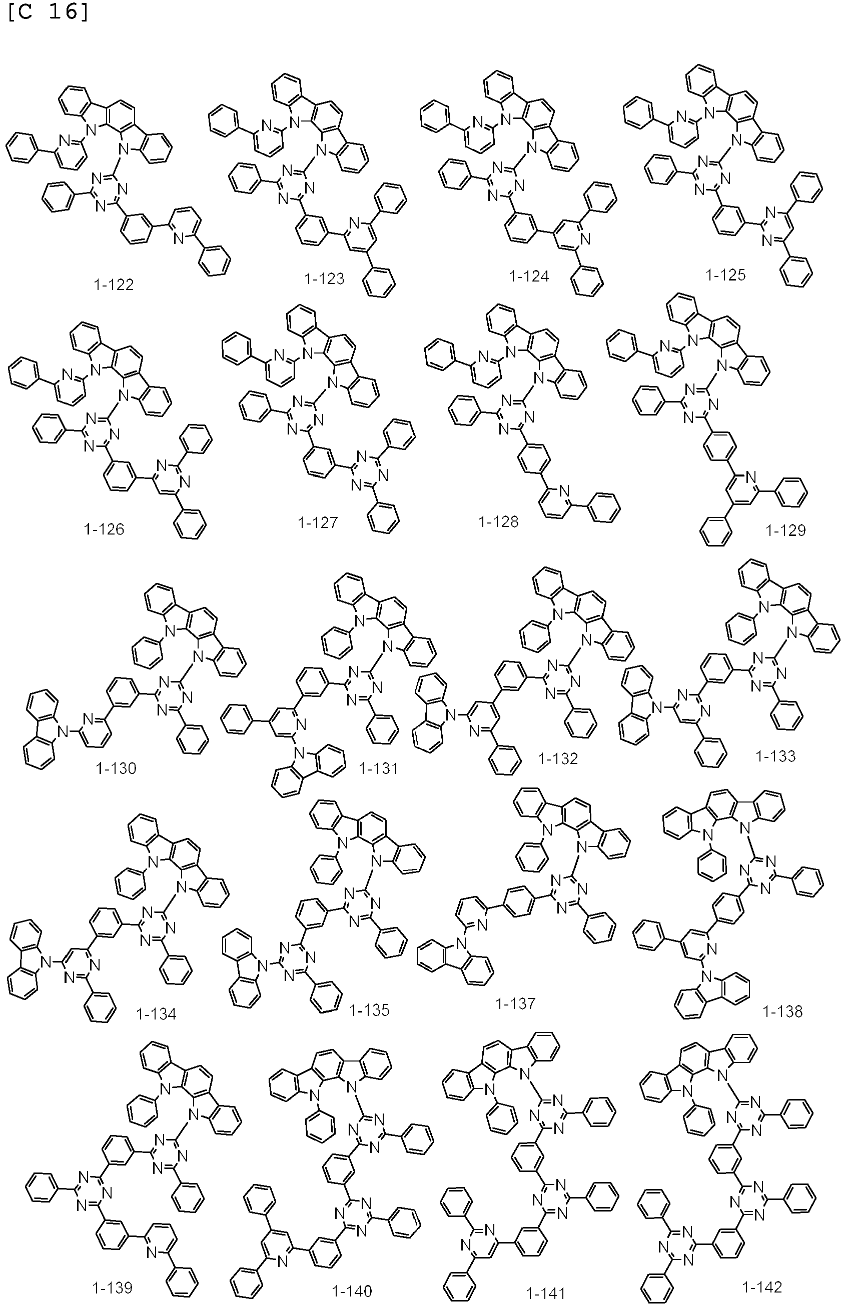

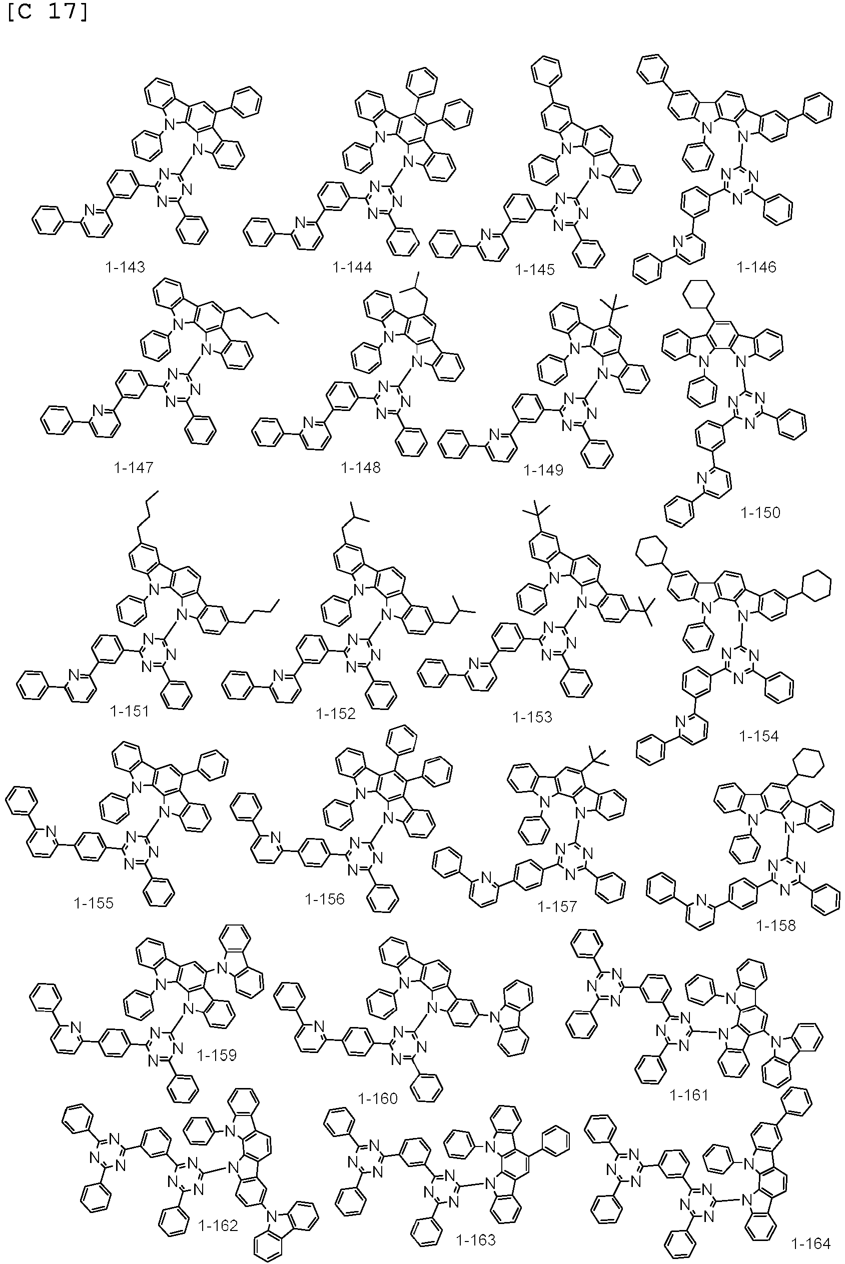

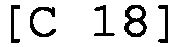

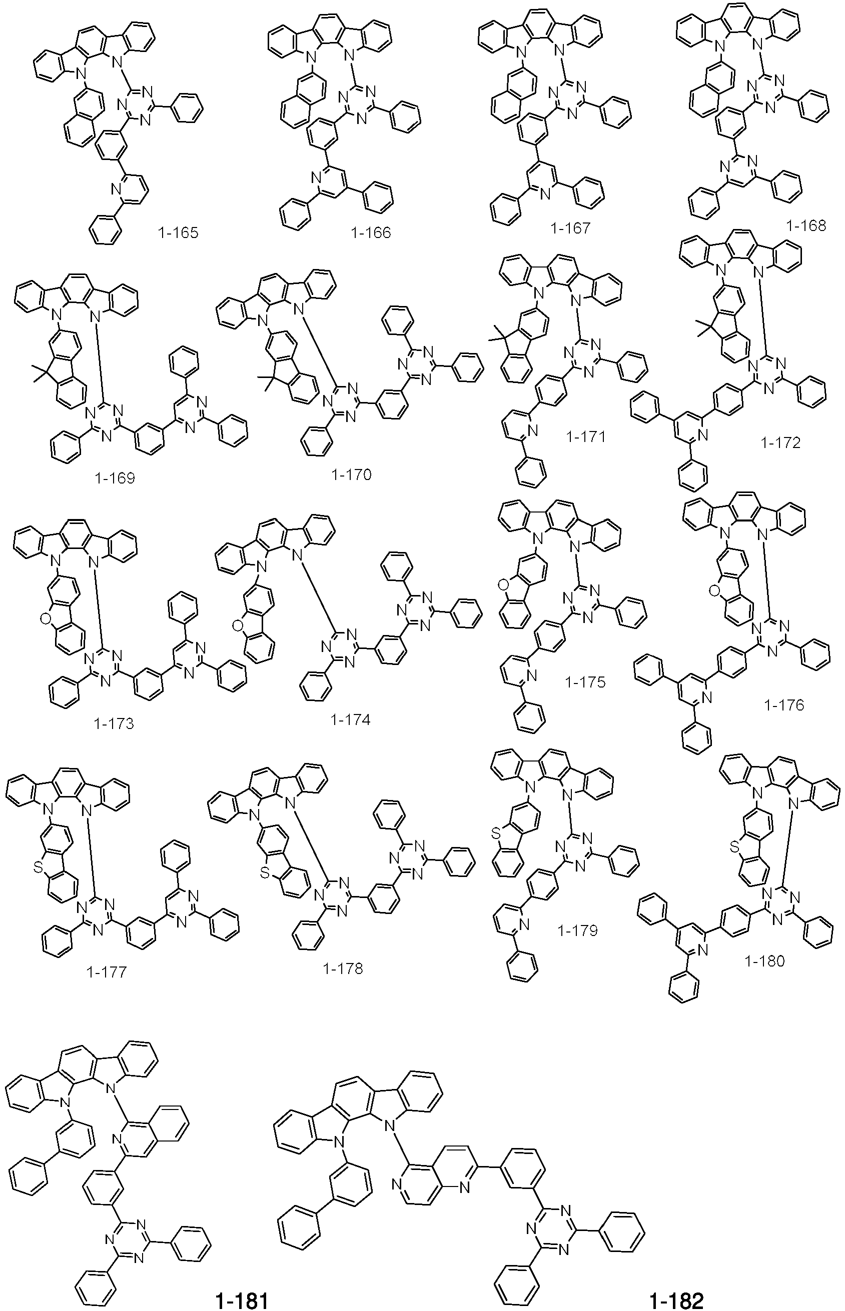

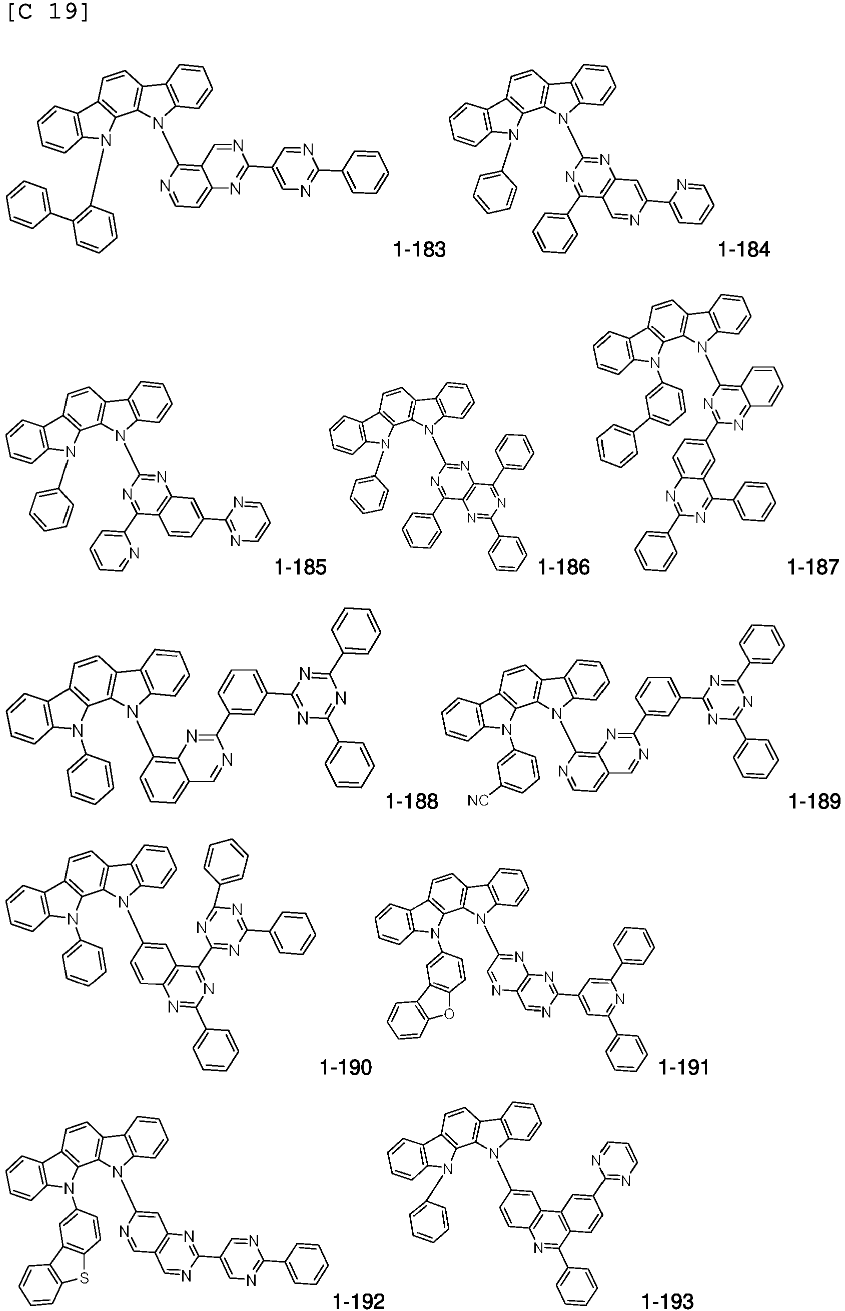

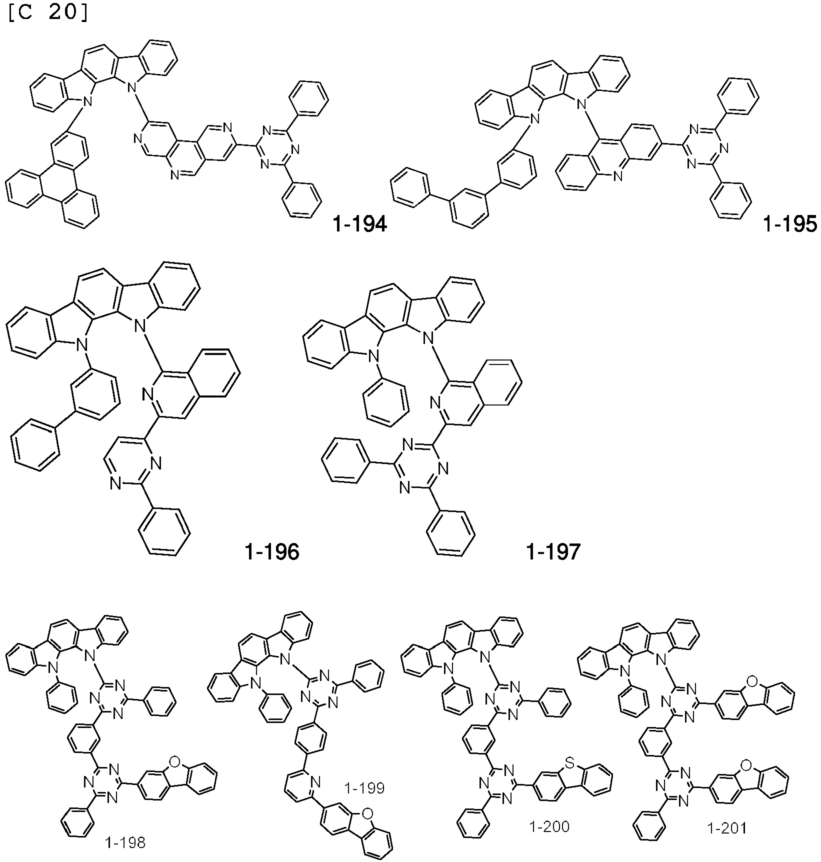

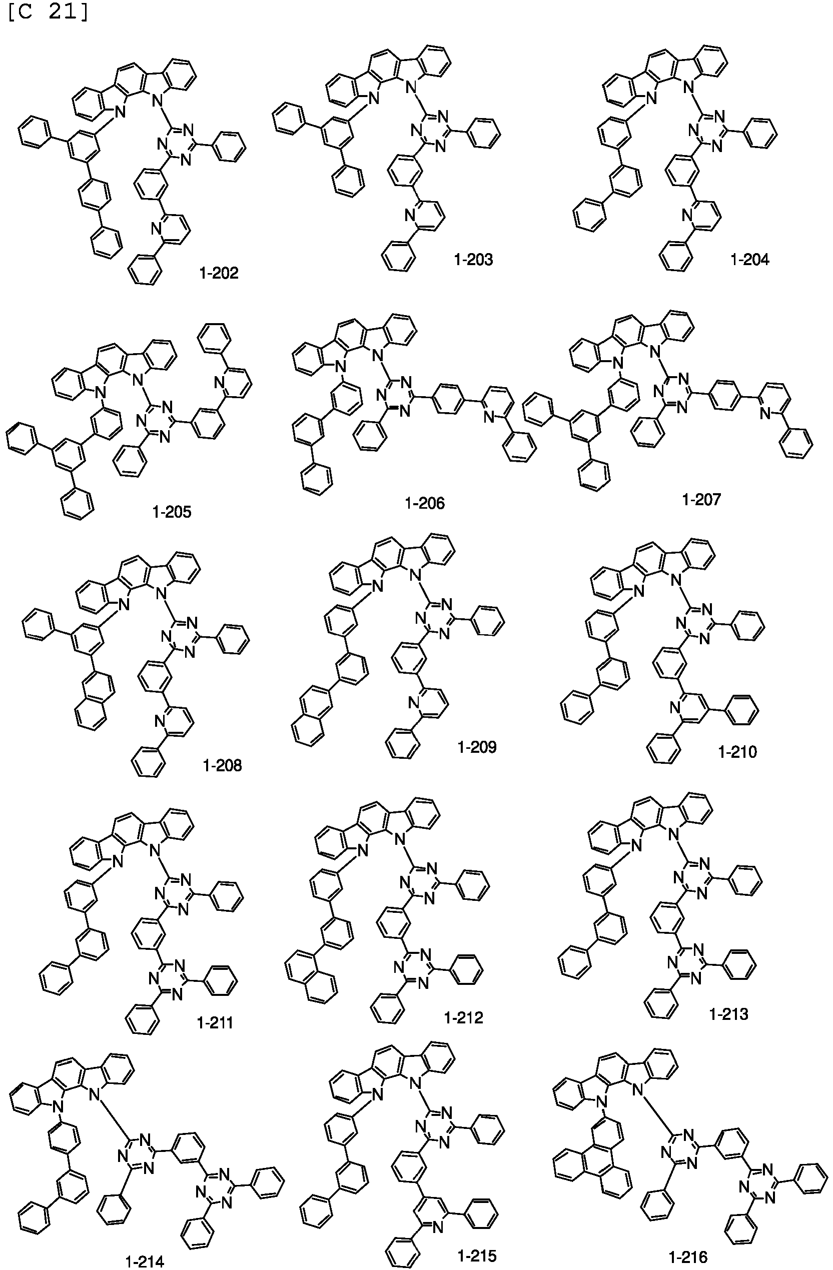

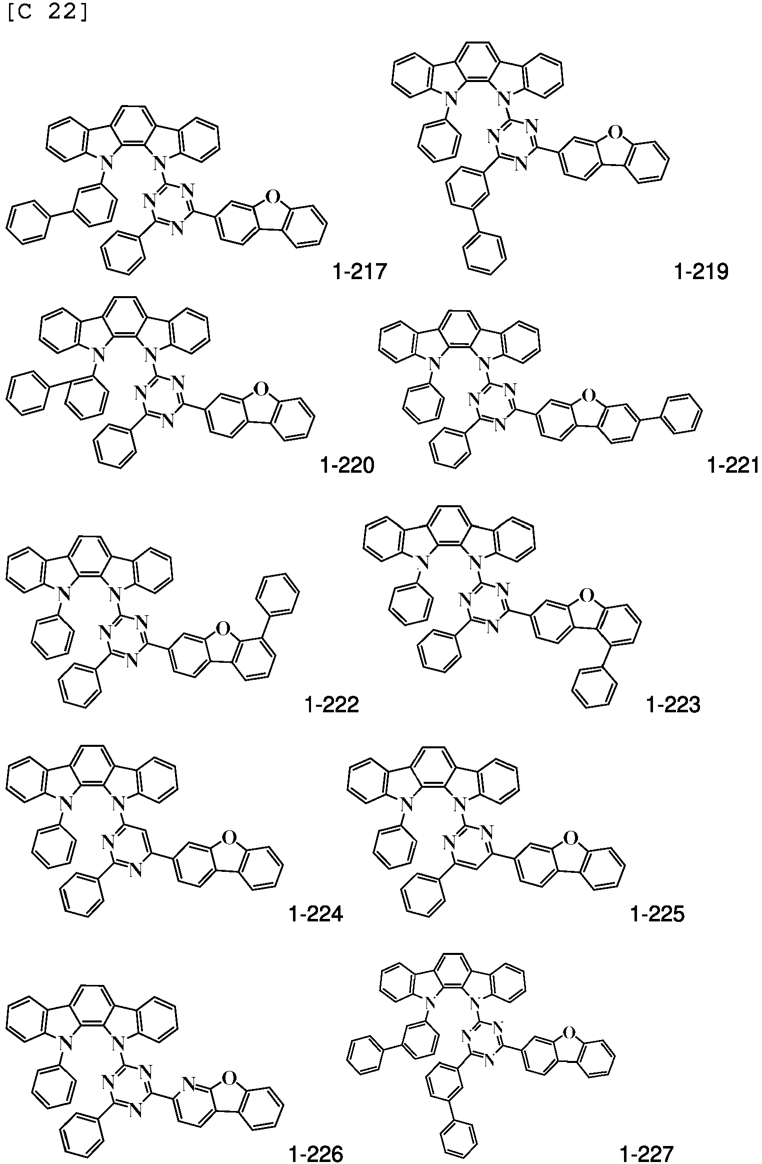

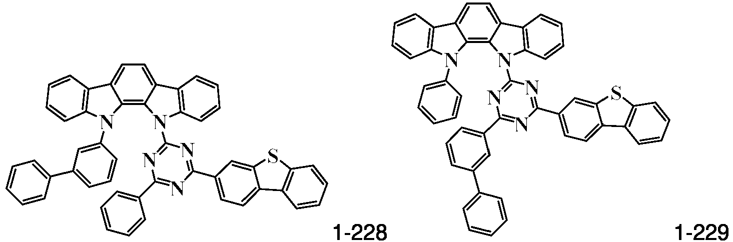

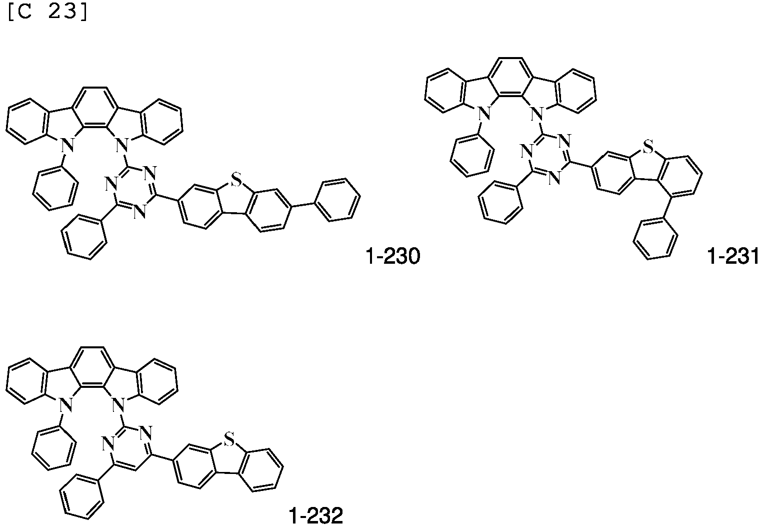

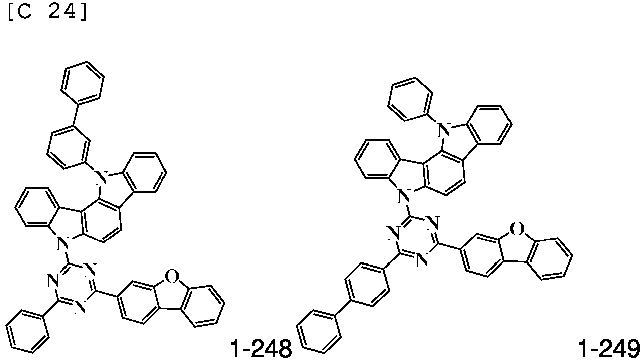

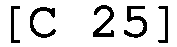

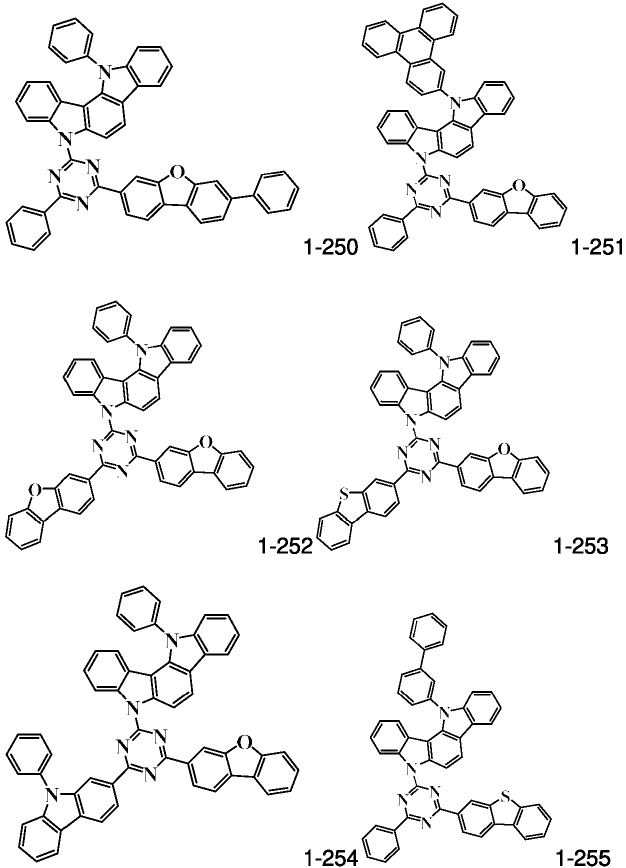

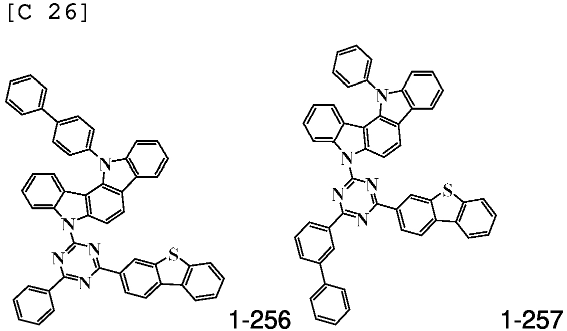

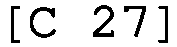

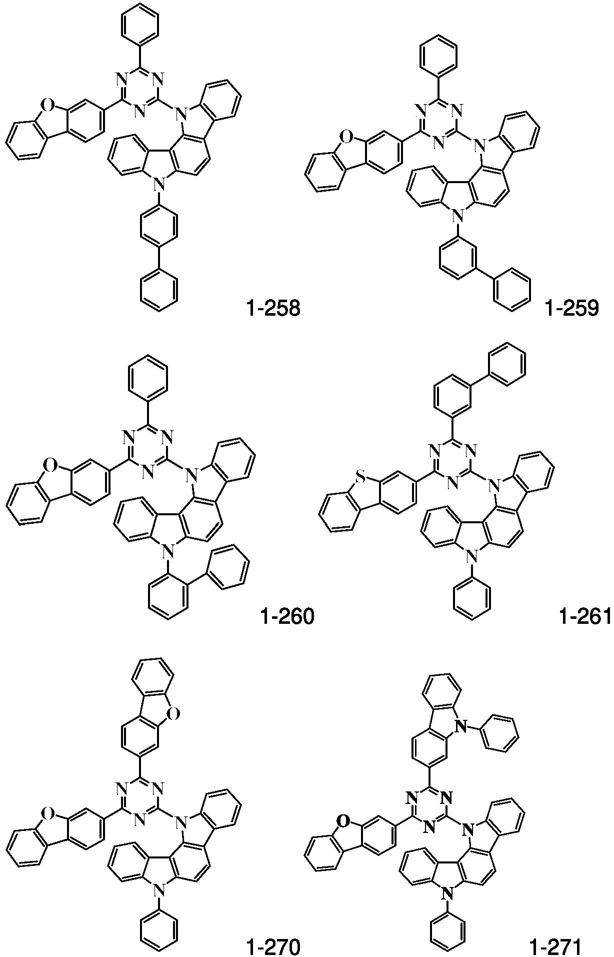

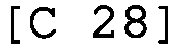

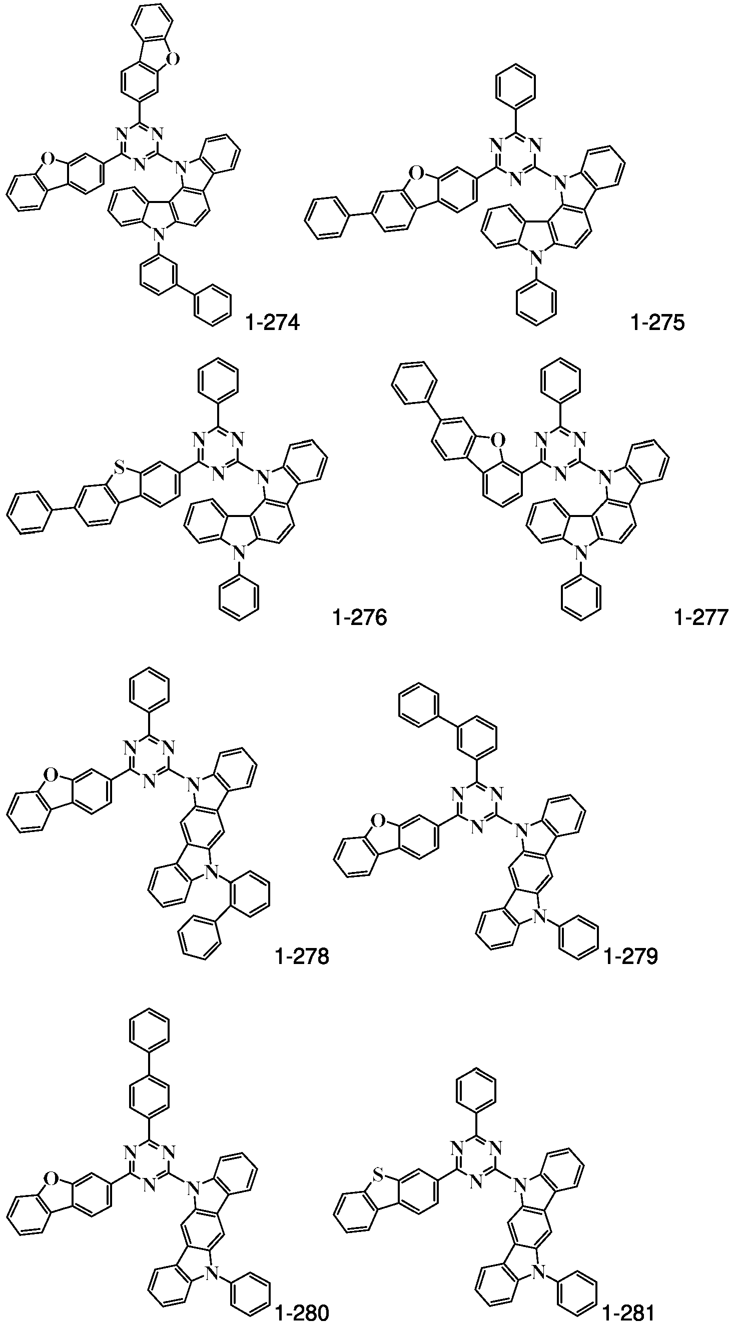

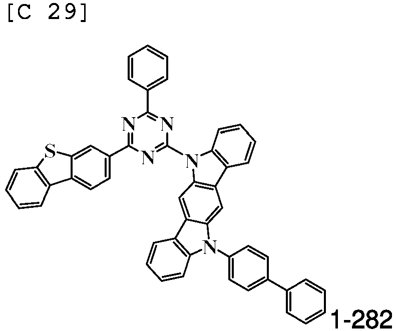

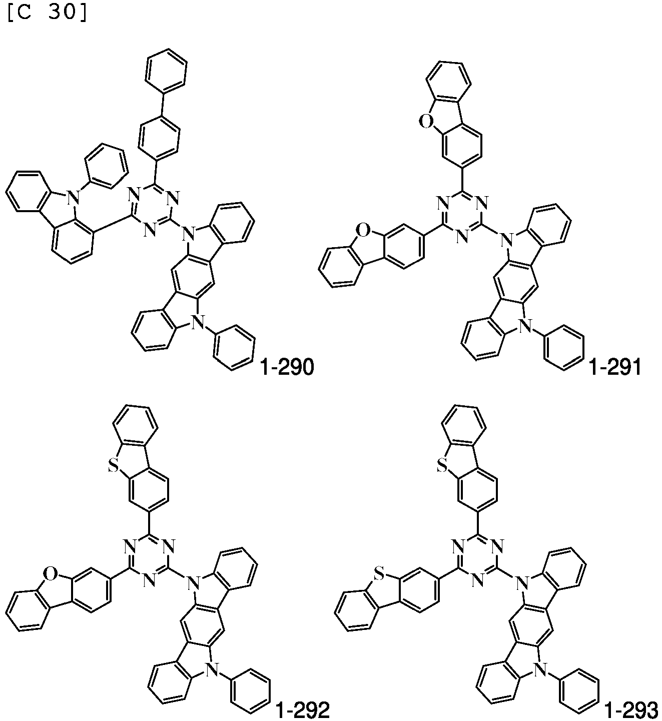

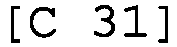

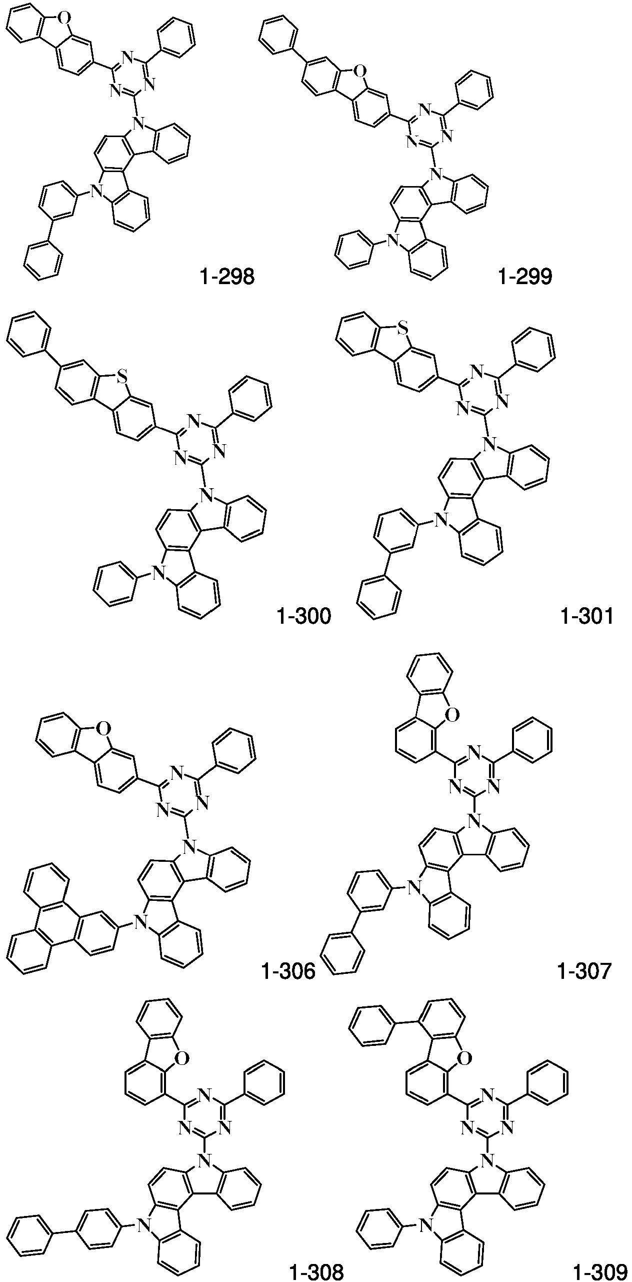

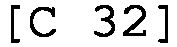

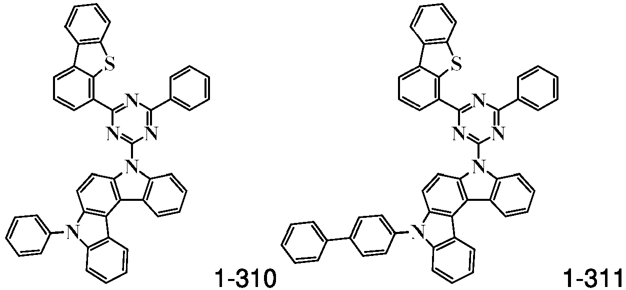

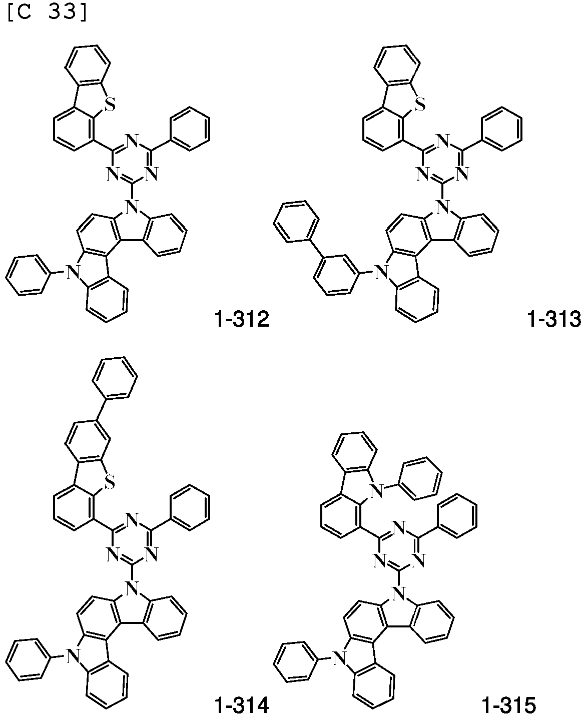

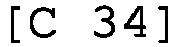

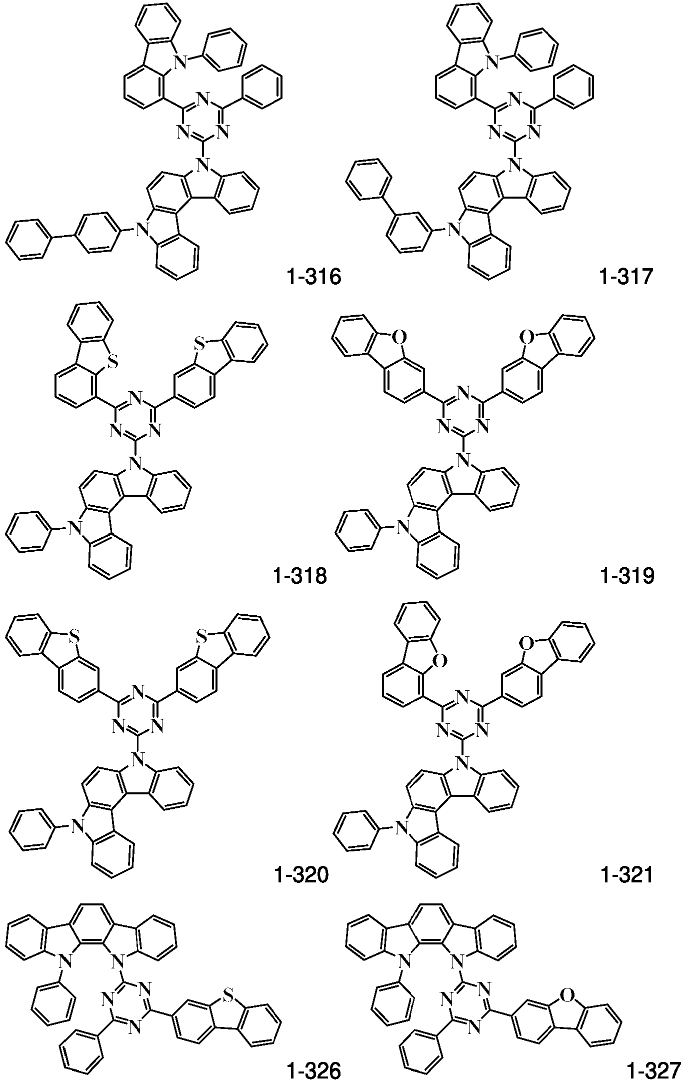

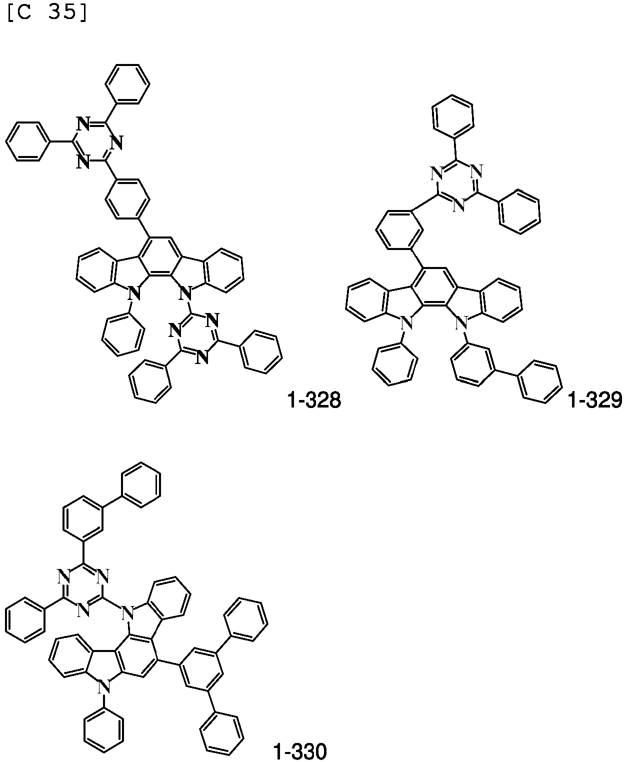

The second host material is preferably a compound represented by general formula (2), general formula (3) or general formula (4).

In the general formula (2), Ar²¹, and Ar²² independently represent hydrogen, deuterium, a substituted or unsubstituted aromatic hydrocarbon group having 6 to 14 carbon atoms, a substituted or unsubstituted aromatic heterocyclic group having 3 to 17 carbon atoms, or a substituted or unsubstituted linked aromatic group in which two of the aromatic rings are linked to each other. Preferred is hydrogen, an aromatic hydrocarbon group having 6 to 12 carbon atoms, an aromatic heterocyclic group having 3 to 17 carbon atoms, or a substituted or unsubstituted linked aromatic group in which two of the aromatic rings are linked to each other, and more preferred is hydrogen, an aromatic hydrocarbon group having 6 to 10 carbon atoms, or a substituted or unsubstituted linked aromatic group in which two of these are linked to each other.

In the case of Ar²¹ and Ar²² being each an unsubstituted aromatic hydrocarbon group, an unsubstituted aromatic heterocyclic group, or an unsubstituted linked aromatic group, specific examples thereof include a linked aromatic group generated by removing one H atom from benzene, naphthalene, anthracene, phenanthrene, fluorene, dibenzofuran, dibenzothiophene, carbazole, pyridine, or a compound in which two of these aromatic rings are linked. Preferred examples include an aromatic group generated from benzene, naphthalene, pyridine, dibenzofuran, dibenzothiophene or carbazole, or a linked aromatic group in which two of these aromatic groups are linked to each other, and more preferred is an aromatic group generated from benzene or naphthalene. Ar²¹ or Ar²² may be hydrogen, and in this case, other thereof is preferably the aromatic group (which means the aromatic hydrocarbon group or the aromatic heterocyclic group.) or the linked aromatic group.

L²¹ and L²² are each a direct bond or a phenylene group, and the phenylene group may be any of an o-phenylene group, a m-phenylene group and a p-phenylene group. Preferred is a p-phenylene group or a m-phenylene group. When L²¹ and L²² are direct bonds, it is preferred that Ar²¹ and Ar²² are not hydrogen or deuterium.

Specific examples of the compounds represented by the general formula (2) are described below, but the compounds are not limited to these exemplified compounds.

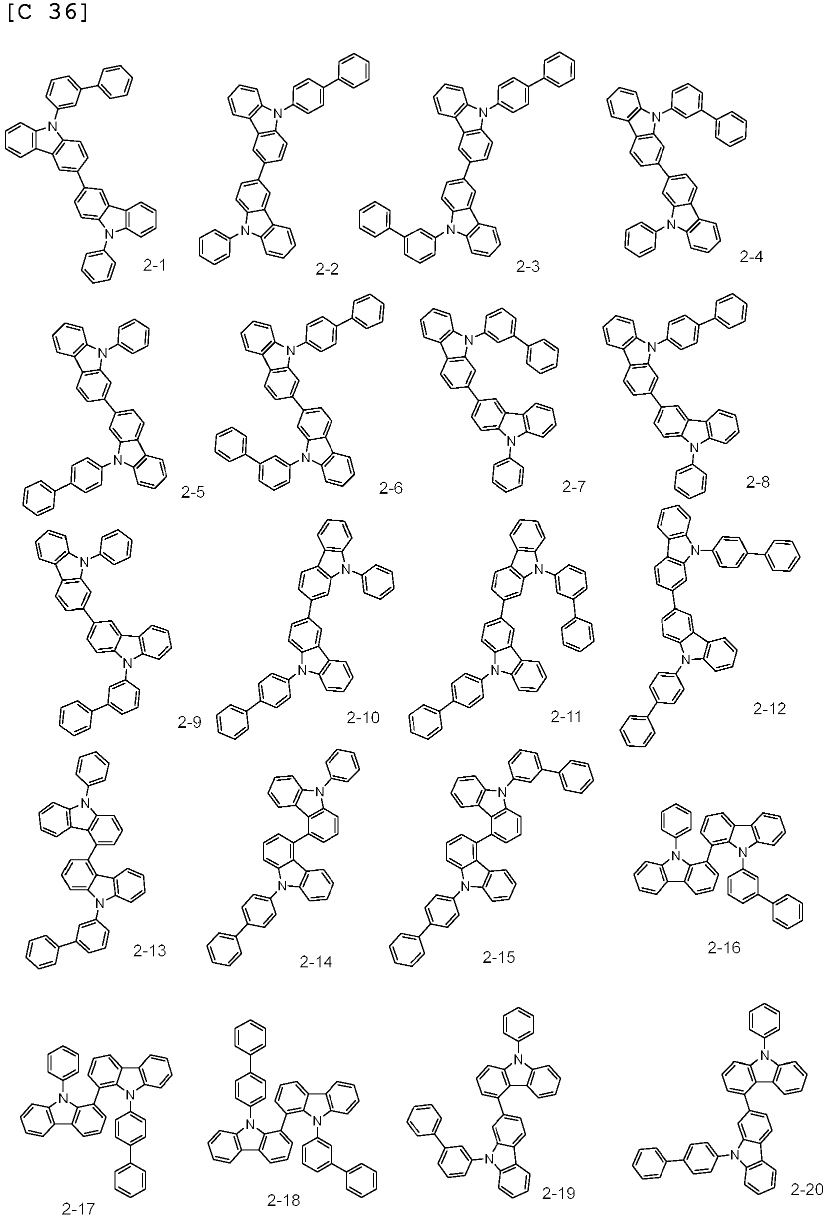

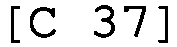

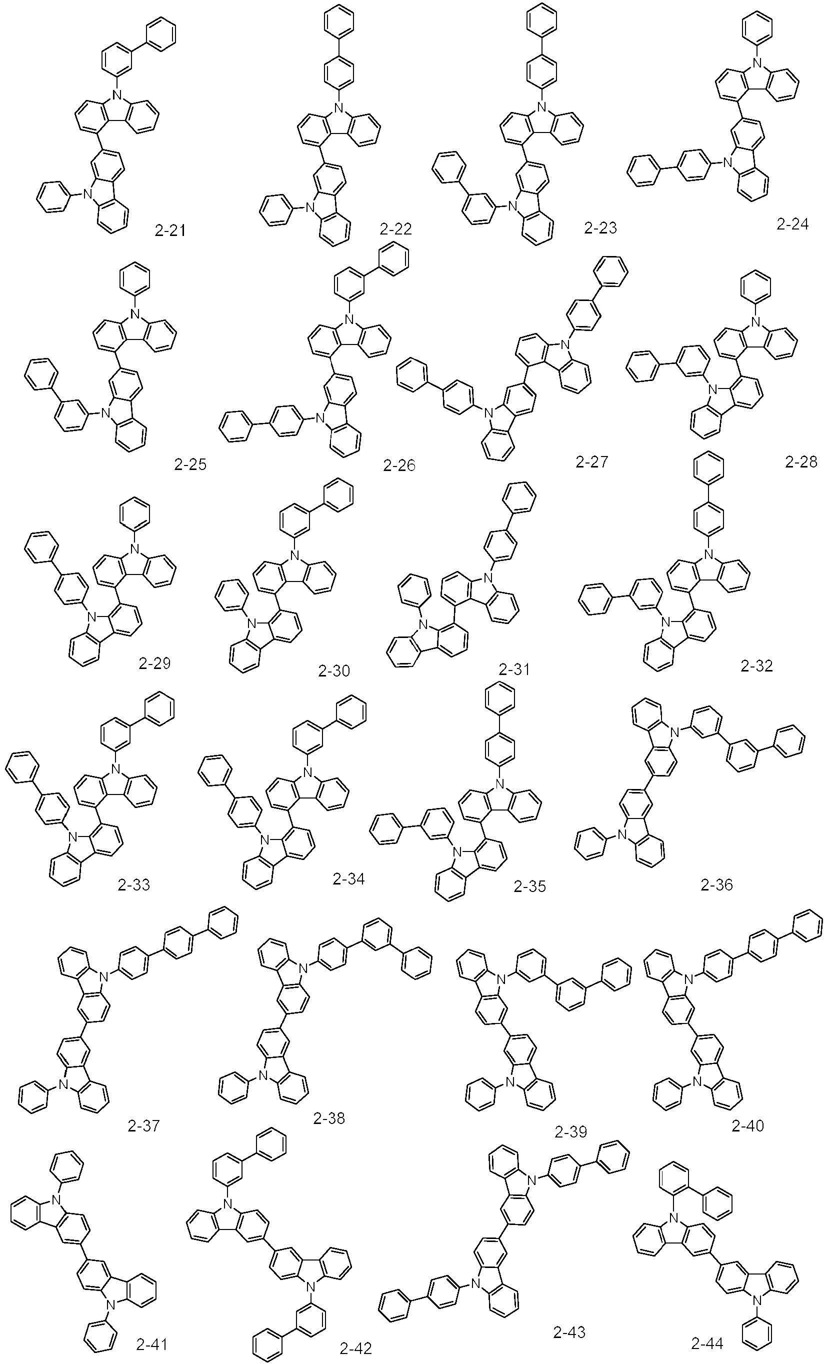

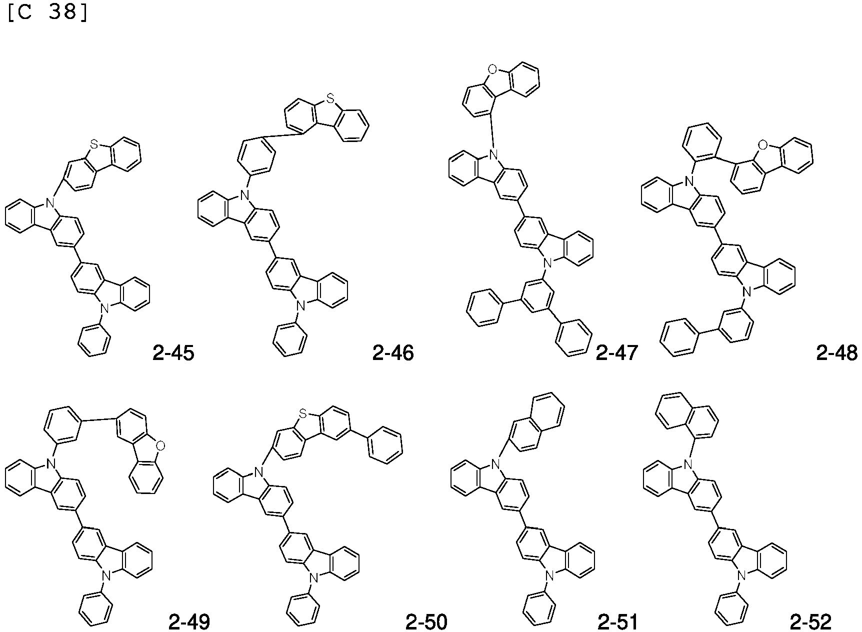

In the general formula (3), a ring B is a heterocycle represented by formula (3a) and the ring B is fused to an adjacent ring at any position.

R³ independently represents deuterium, an aliphatic hydrocarbon group having 1 to 10 carbon atoms, a substituted or unsubstituted aromatic hydrocarbon group having 6 to 30 carbon atoms, a substituted or unsubstituted aromatic heterocyclic group having 3 to 17 carbon atoms, or a substituted or unsubstituted linked aromatic group in which two to five of these aromatic rings are linked to each other. Preferred is an aliphatic hydrocarbon group having 1 to 8 carbon atoms, a substituted or unsubstituted aromatic hydrocarbon group having 6 to 10 carbon atoms, or a substituted or unsubstituted aromatic heterocyclic group having 3 to 12 carbon atoms. More preferred is an aliphatic hydrocarbon group having 1 to 6 carbon atoms, a substituted or unsubstituted phenyl group, or a substituted or unsubstituted aromatic heterocyclic group having 3 to 6 carbon atoms.

In the case of R³ being an aliphatic hydrocarbon group, an aromatic hydrocarbon group, an aromatic heterocyclic group, or a linked aromatic group, specific examples thereof are the same as in the case of R¹ described above.

L³¹ independently represents a substituted or unsubstituted aromatic hydrocarbon group having 6 to 30 carbon atoms, or a substituted or unsubstituted aromatic heterocyclic group having 3 to 17 carbon atoms. Preferred is a substituted or unsubstituted aromatic hydrocarbon group having 6 to 10 carbon atoms, or a substituted or unsubstituted aromatic heterocyclic group having 3 to 12 carbon atoms.

L³² independently represents a substituted or unsubstituted aromatic hydrocarbon group having 6 to 10 carbon atoms, preferably represents a group generated from benzene.

Ar³¹ represents a substituted or unsubstituted aromatic hydrocarbon group having 6 to 10 carbon atoms, a substituted or unsubstituted carbazolyl group, or a substituted or unsubstituted linked aromatic group in which two to five of these aromatic rings are linked to each other. The aromatic ring is here selected from an aromatic hydrocarbon ring and a carbazole ring.

Preferred is a substituted or unsubstituted aromatic hydrocarbon group having 6 to 10 carbon atoms, a carbazolyl group, or a substituted or unsubstituted linked aromatic group in which two to three of these aromatic rings are linked to each other.

Specific examples of the unsubstituted aromatic hydrocarbon group include a phenyl group and a naphthyl group.

Ar³² represents a substituted or unsubstituted aromatic hydrocarbon group having 6 to 30 carbon atoms, a substituted or unsubstituted aromatic heterocyclic group having 3 to 17 carbon atoms, or a substituted or unsubstituted linked aromatic group in which two to five of these aromatic rings are linked to each other. Preferred is a substituted or unsubstituted aromatic hydrocarbon group having 6 to 10 carbon atoms, a substituted or unsubstituted aromatic heterocyclic group having 3 to 12 carbon atoms, or a substituted or unsubstituted linked aromatic group in which two to five of these aromatic rings are linked to each other.

In the case of R³, L³¹, and Ar³² being each an unsubstituted aromatic hydrocarbon group or an aromatic heterocyclic group, specific examples thereof are the same as in the case of Ar¹ and Ar¹¹ except that L³¹ is a divalent or (h + 1) -valent group. In the case of Ar³² and R³ being an unsubstituted linked aromatic group, specific examples thereof are the same as in the case of Ar¹ and Ar¹¹ except that two to five aromatic rings are linked to each other.

f is the number of repetitions, and represents an integer of 1 to 3, g is the number of repetitions and represents an integer of 0 to 3, and h represents the number of substitutions and each independently represents an integer of 0 to 7. Preferably, f is 1, g is 0 to 1, and h is 0 to 2. i represents an integer of 0 to 4, and j represents an integer of 0 to 2, and preferably, i is 0 or 1 and j is 0 or 1.

Preferred examples of the compound represented by the general formula (3) include a compound represented by the following formula (31) or formula (32).

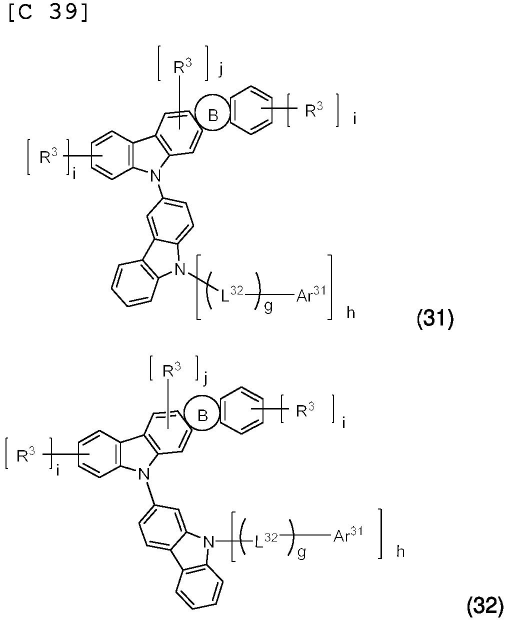

In the formula (31) or formula (32), a ring B, R³, L³², Ar³¹, g, h, i and j are as defined for the general formula (3).

Specific examples of the compounds represented by the general formula (3) are described below, but the compounds are not limited to these exemplified compounds.

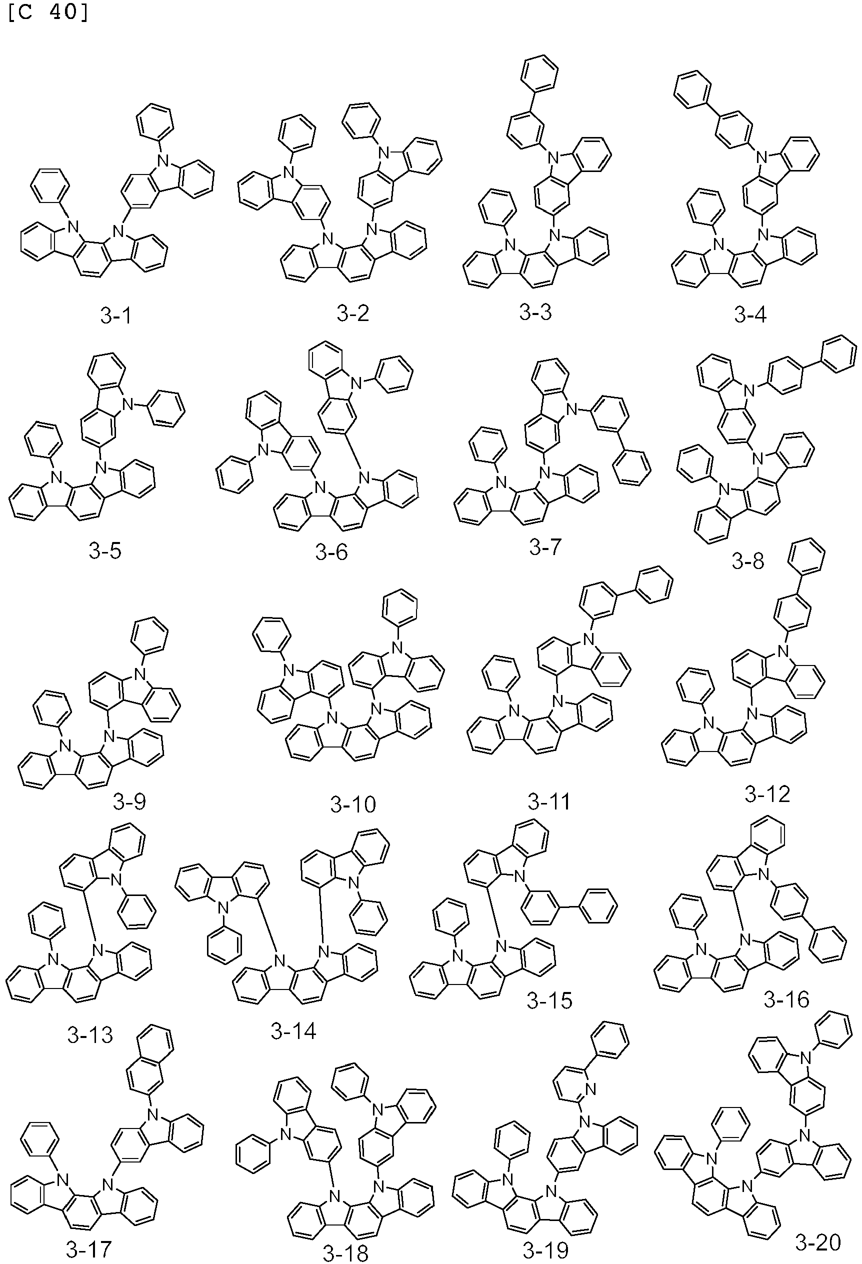

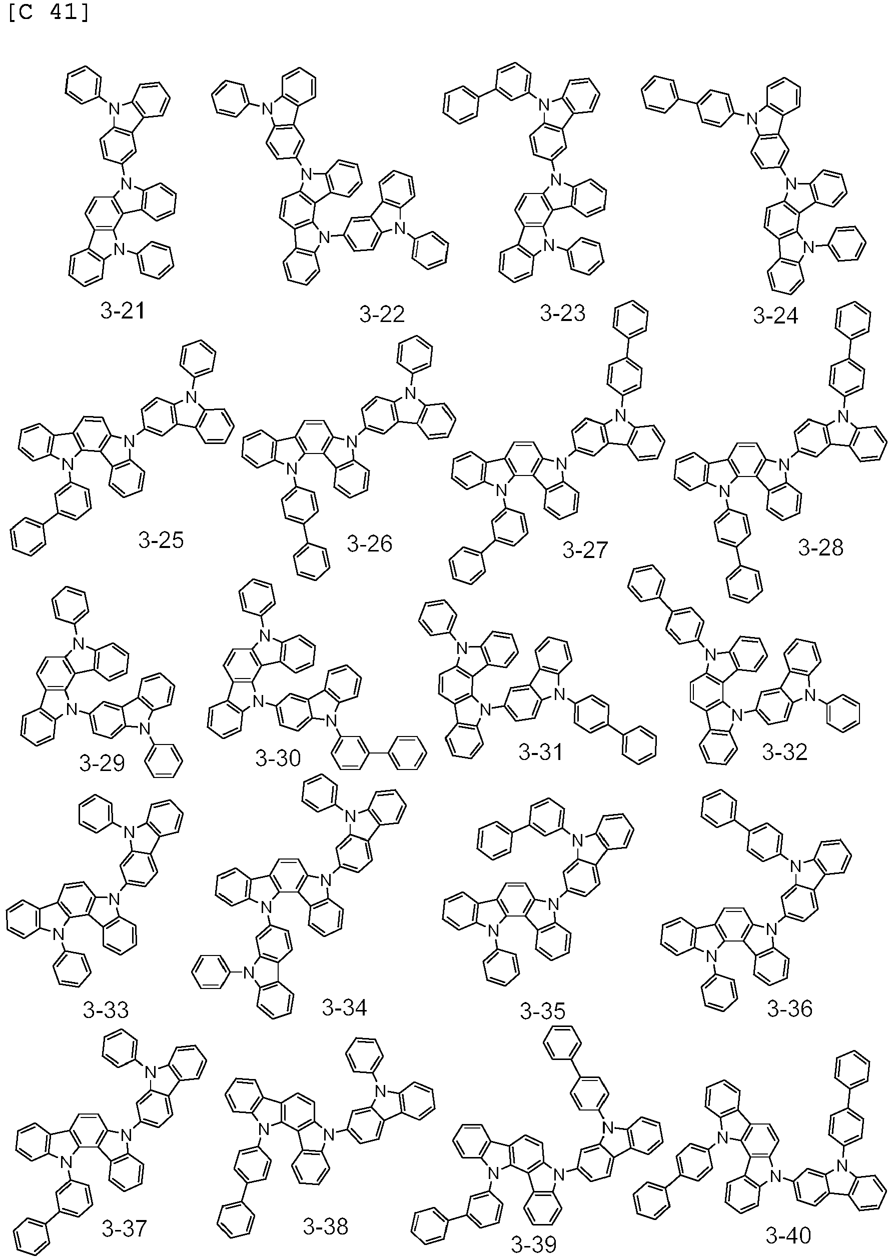

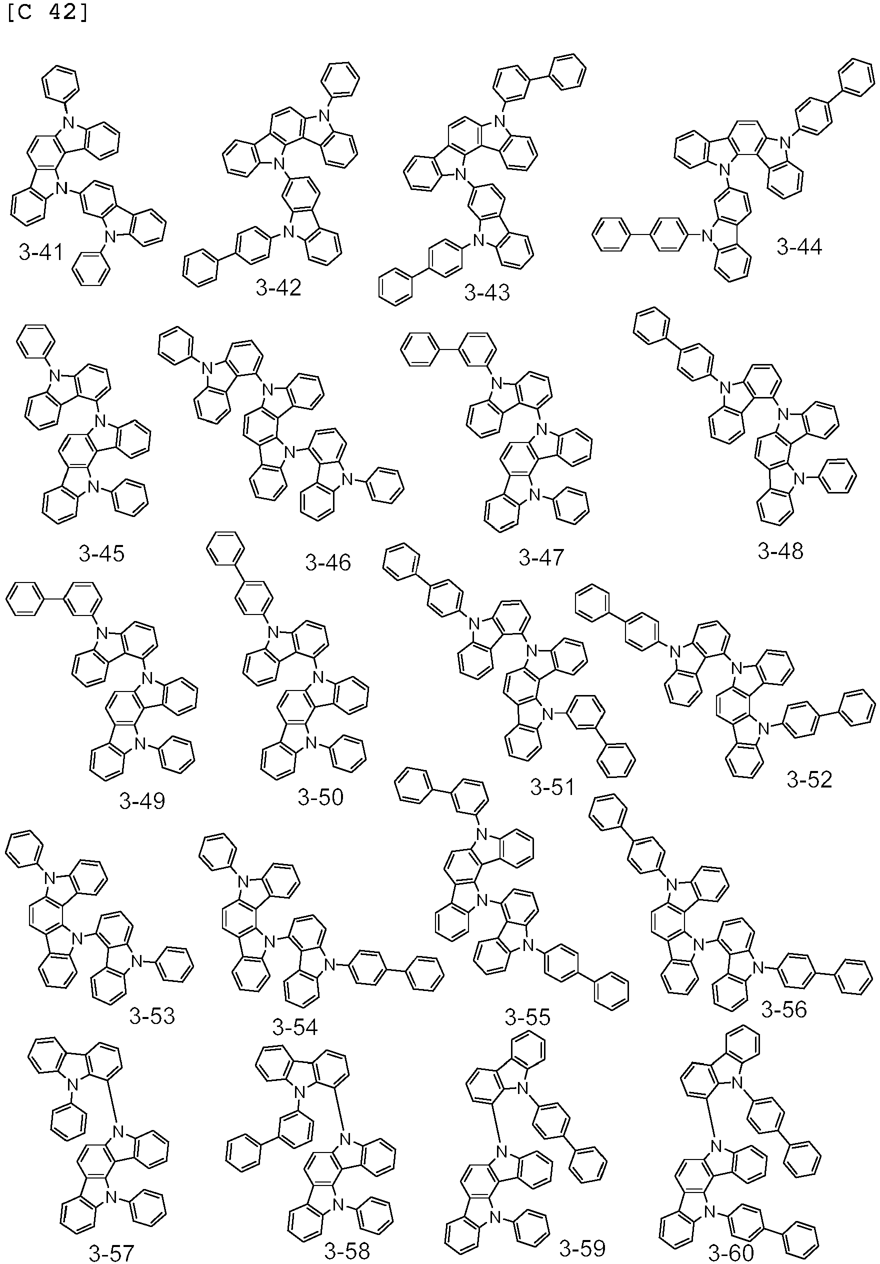

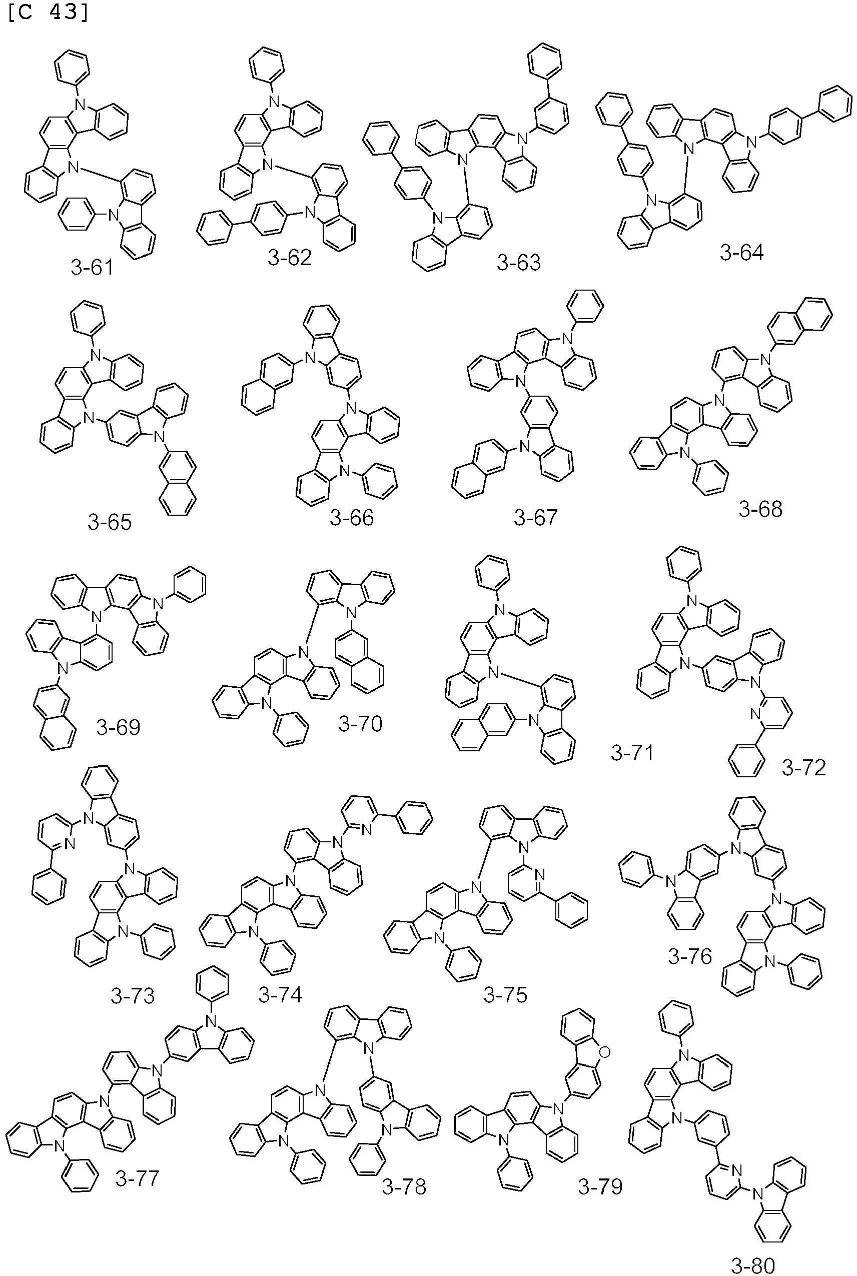

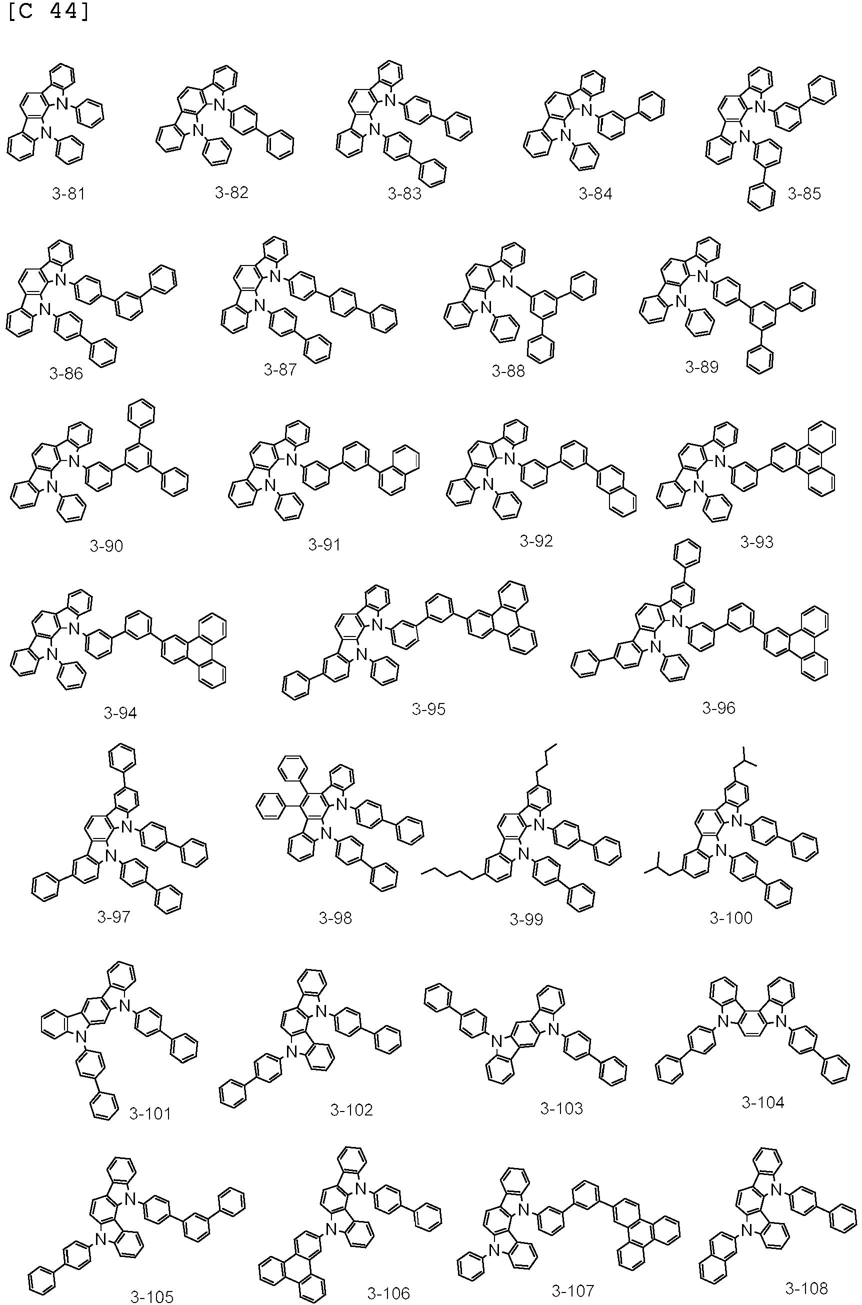

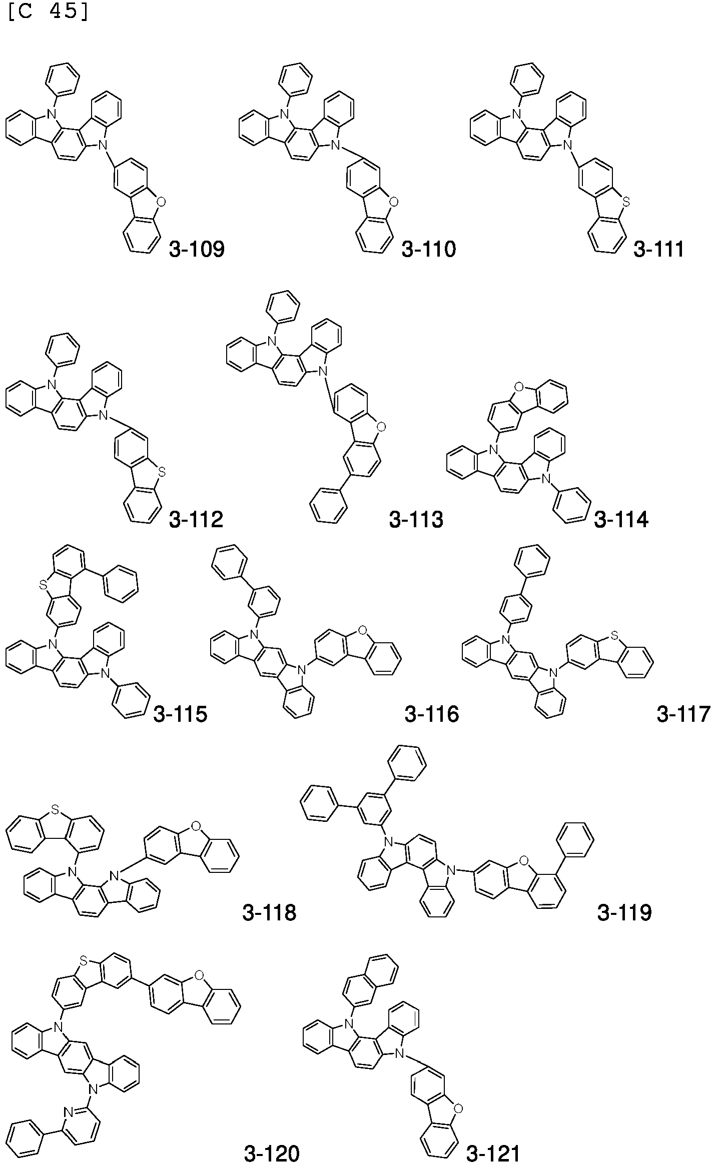

Next, the general formula (4) will be described.

In the general formula (4), Ar⁴¹ represents a substituted or unsubstituted aromatic hydrocarbon group having 6 to 30 carbon atoms, a substituted or unsubstituted aromatic heterocyclic group having 3 to 17 carbon atoms, or a linked aromatic group in which two to five of these aromatic rings are linked. Preferred is a substituted or unsubstituted aromatic hydrocarbon group having 6 to 10 carbon atoms, a substituted or unsubstituted aromatic heterocyclic group having 3 to 12 carbon atoms, or a substituted or unsubstituted linked aromatic group in which two to five of these aromatic rings are linked to each other.

In the case of Ar⁴¹ being an unsubstituted aromatic hydrocarbon group, an unsubstituted aromatic heterocyclic group, or an unsubstituted linked aromatic group, specific examples thereof are the same as in the case of Ar¹ and Ar¹¹ except that the group is y-valent and that, in the case of the linked aromatic group, two to five aromatic rings are linked to each other. Preferred is a group generated from pyridine, pyrimidine, triazine, dibenzofuran, dibenzothiophene, benzene, naphthalene, or a compound formed by linking two to five of these to each other.

R⁴¹ each independently represents deuterium, an aliphatic hydrocarbon group having 1 to 10 carbon atoms, a substituted or unsubstituted aromatic hydrocarbon group having 6 to 30 carbon atoms, or a substituted or unsubstituted aromatic heterocyclic group having 3 to 17 carbon atoms, provided that R⁴¹ is not a carbazole group and advantageously R⁴¹ is not a group containing a carbazole ring. Preferred is an aliphatic hydrocarbon group having 1 to 6 carbon atoms, a substituted or unsubstituted aromatic hydrocarbon group having 6 to 12 carbon atoms, or a substituted or unsubstituted aromatic heterocyclic group having 3 to 17 carbon atoms.

In the case of R⁴¹ being an aliphatic hydrocarbon group, specific examples thereof are the same as in the case of R¹.

In the case of R⁴¹ being an unsubstituted aromatic hydrocarbon group or aromatic heterocyclic group, specific examples thereof are the same as in the case of Ar¹ and Ar¹¹ except that no carbazole is included.

y represents the number of substitutions, and represents an integer of 1 to 4. Preferably, y is 1 or 2, and more preferably 1.
x is the number of repetitions, and each independently represents an integer of 1 to 4. Preferably, x is 1 to 3. However, at least one x is an integer of 2 to 4. z is an integer of 0 to 3, and preferably 0 or 1.

The sum of x (total number of carbazolyl groups) can be an integer of 2 to 12, and is preferably 2 to 9 and more preferably 2 to 6.

The general formula (4) preferably has at least one binding structure represented by the following formula (4a) or formula (4b) therein. It is more preferred that all of the binding structures among carbazolyl groups should be a binding structure represented by formula (4a) or formula (4b).

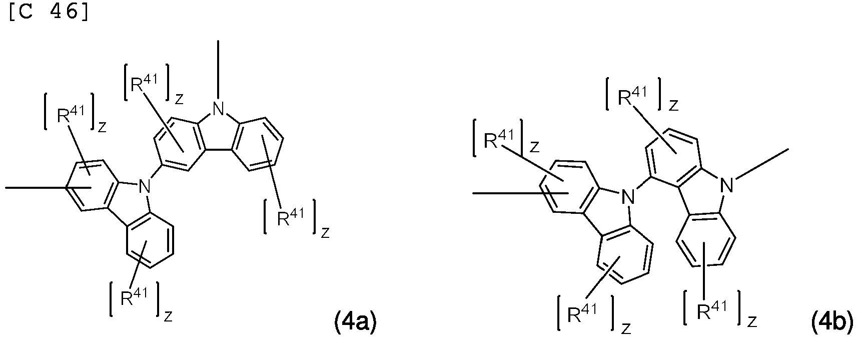

In the formulas, R⁴¹ and z are as defined for the general formula (4).

Specific examples of the compounds represented by the general formula (4) are described below, but the compounds are not limited to these exemplified compounds.

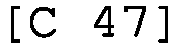

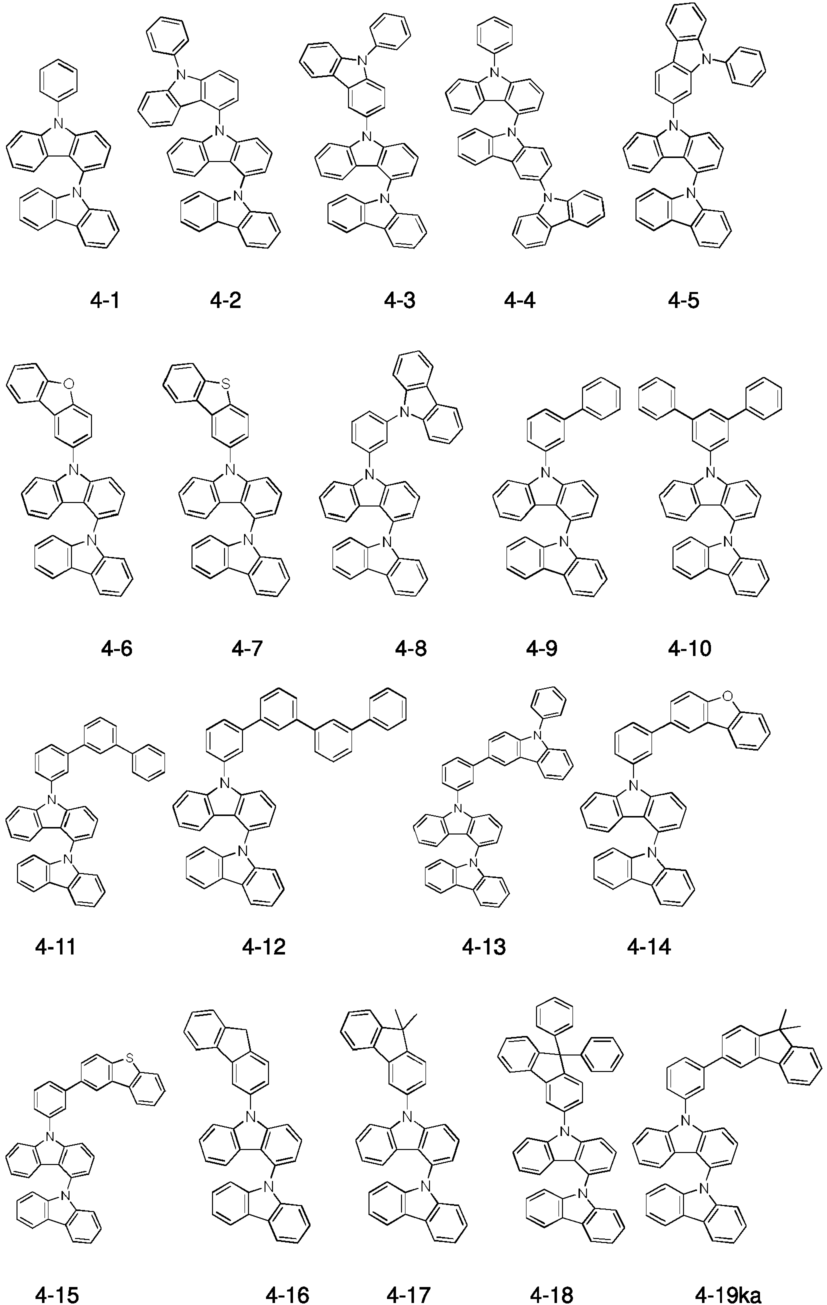

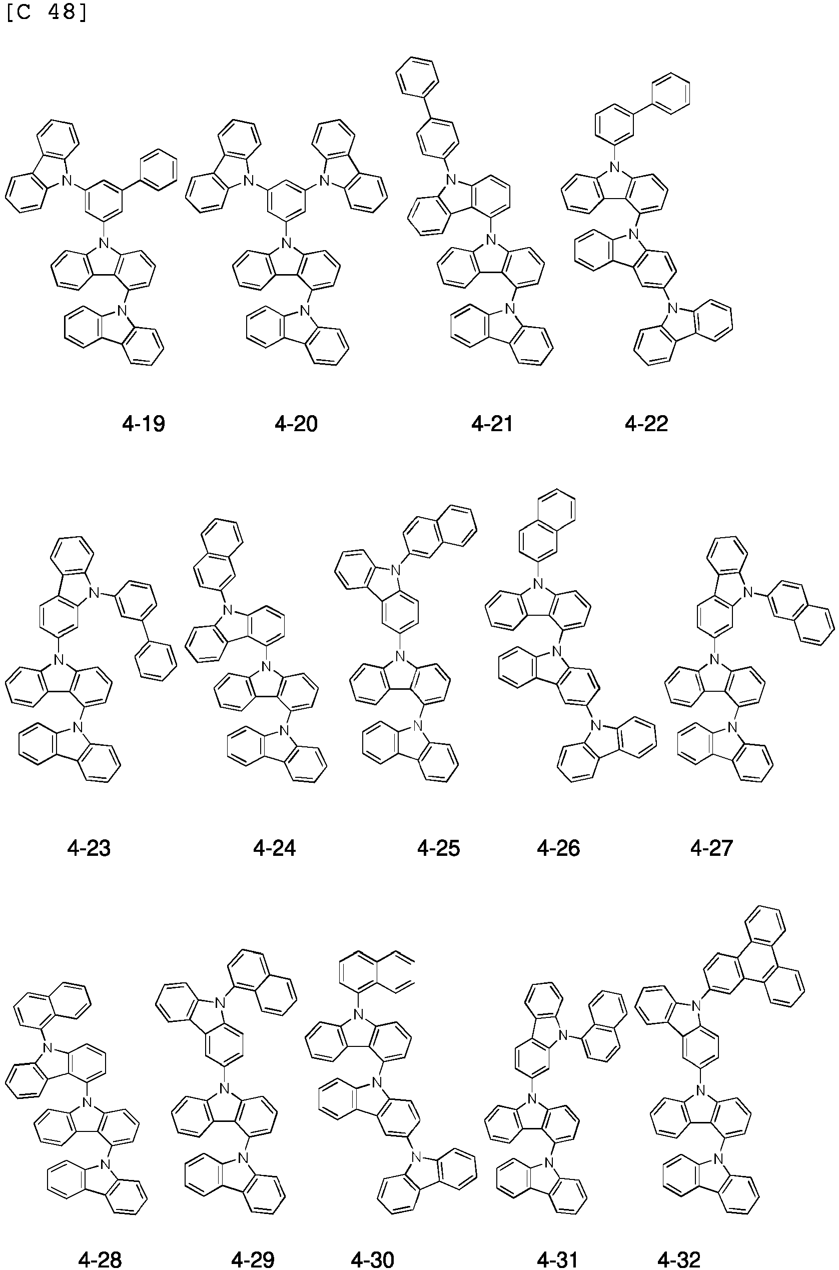

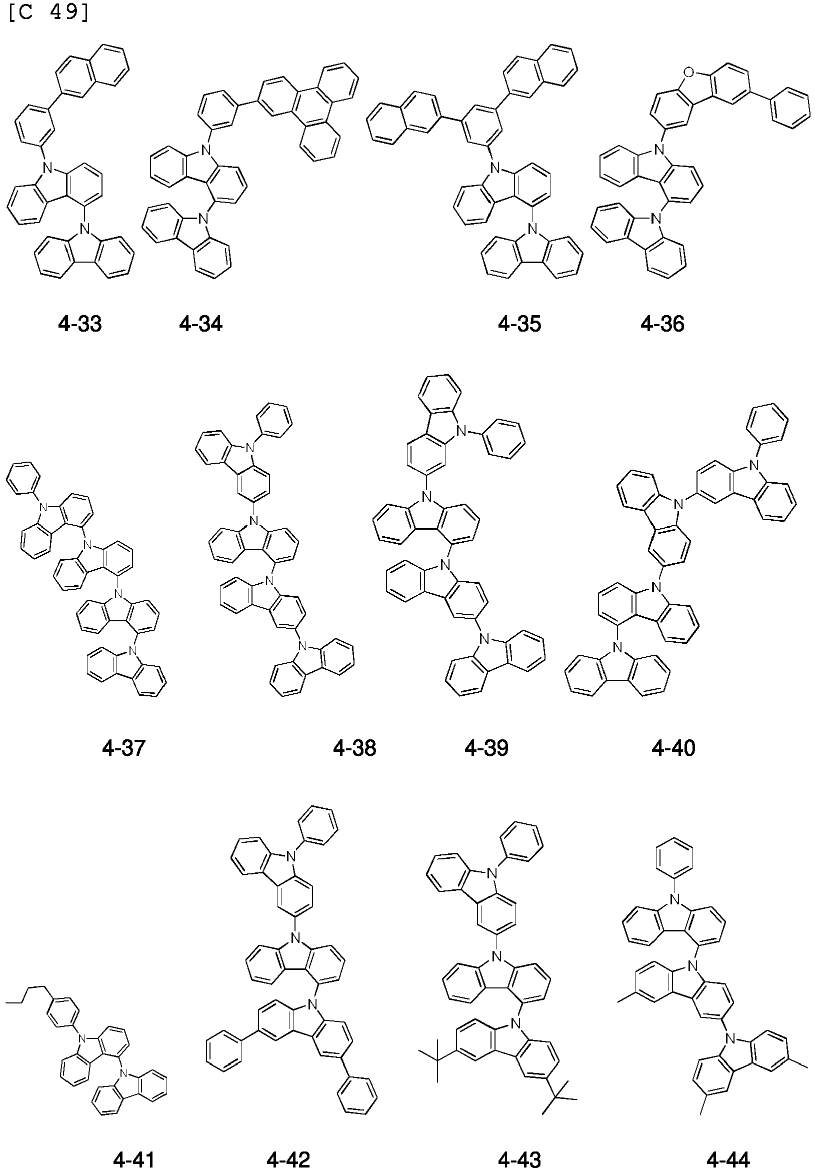

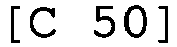

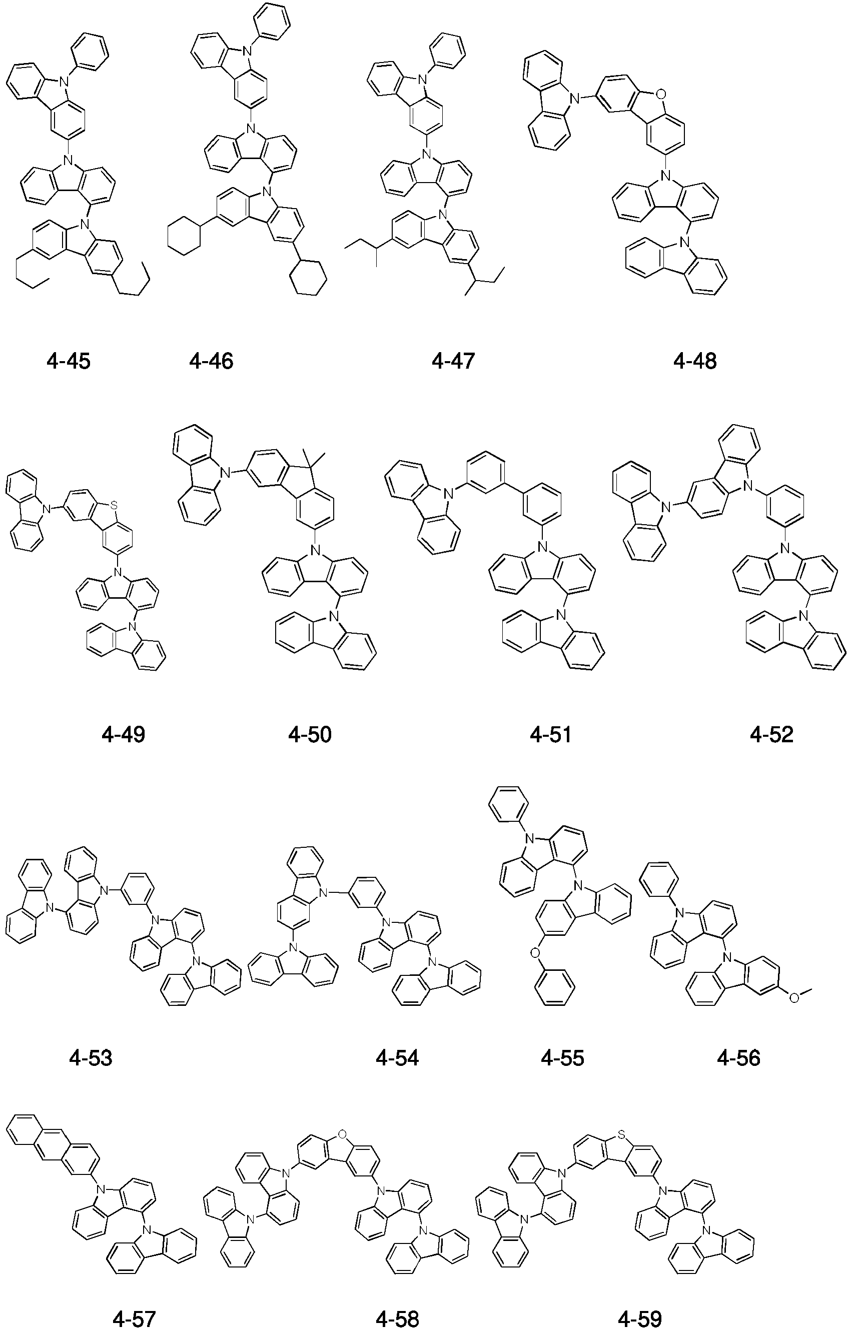

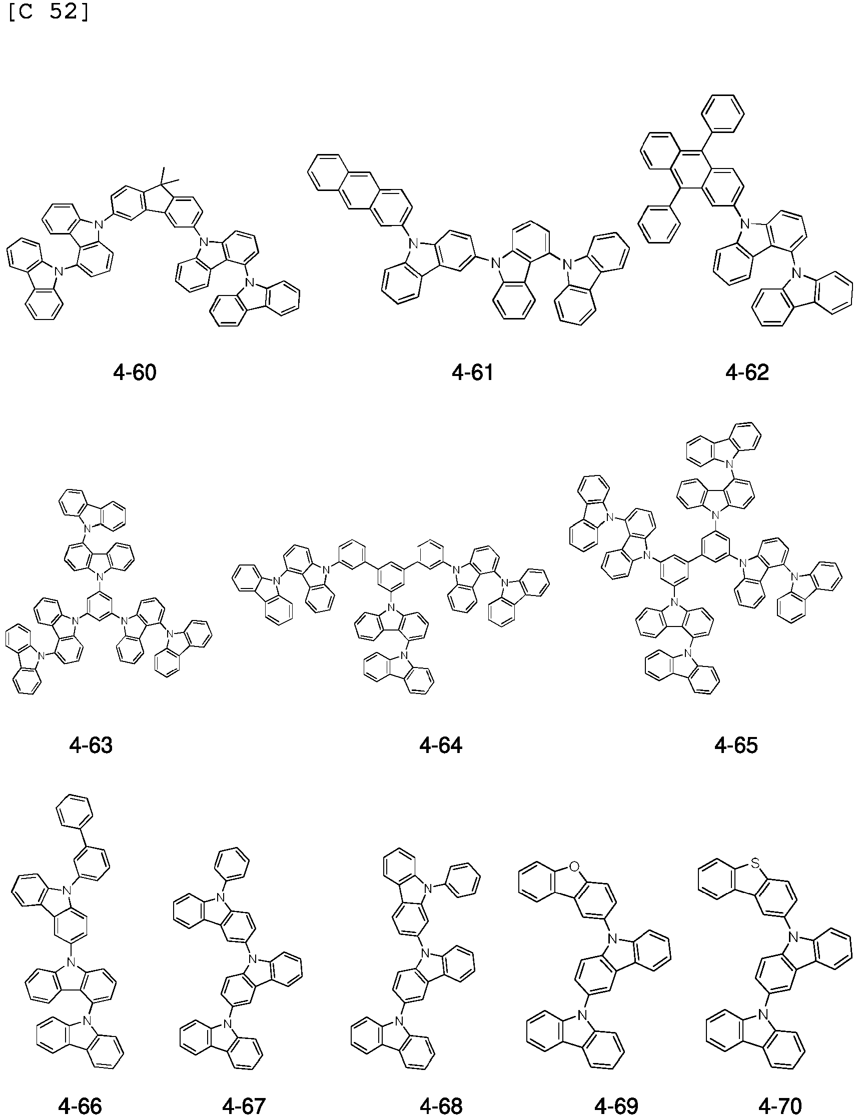

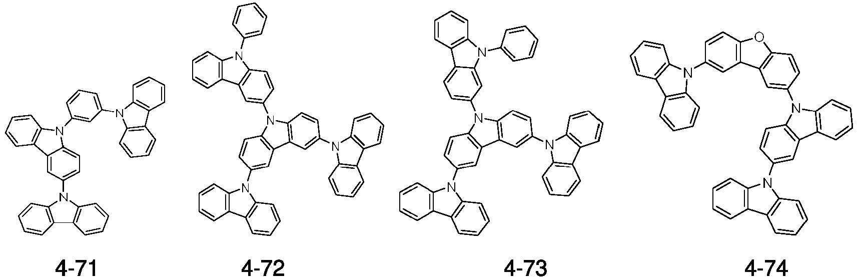

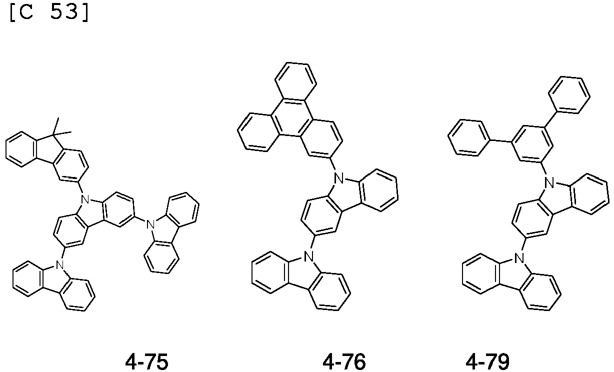

The second host material is preferably the compound represented by the general formula (2) or the general formula (3).

The third host material is suitably the compound represented by the general formula (5). Preferred is the compound represented by the formula (51).

In the general formula (5) and the formula (51), the same symbols have the same meaning.

A ring D is a heterocycle represented by formula (5a) and the ring D is fused to an adjacent ring at any position.

R⁵ independently represents deuterium, an aliphatic hydrocarbon group having 1 to 10 carbon atoms, a substituted or unsubstituted aromatic hydrocarbon group having 6 to 30 carbon atoms, or a substituted or unsubstituted aromatic heterocyclic group having 3 to 17 carbon atoms. Preferred is hydrogen, an aliphatic hydrocarbon group having 1 to 6 carbon atoms, a substituted or unsubstituted aromatic hydrocarbon group having 6 to 10 carbon atoms, or a substituted or unsubstituted aromatic heterocyclic group having 3 to 10 carbon atoms.

In the case of R⁵ being an aliphatic hydrocarbon group, specific examples thereof are the same as in the case of R¹.

In the case of R⁵ being an unsubstituted aromatic hydrocarbon group having 6 to 30 carbon atoms, an aromatic heterocyclic group having 3 to 17 carbon atoms, or a substituted or unsubstituted linked aromatic group in which two to five of these aromatic rings are linked to each other, specific examples thereof are the same as in the case of Ar¹ and Ar¹¹ except that two to five of these aromatic rings are linked to each other.

p, r, and s represent the number of substitutions, and each independently represent an integer of 0 to 4. These are each preferably an integer of 0 to 3, and further preferably 0 to 2. q represents an integer of 0 to 2, preferably represents an integer of 0 to 1.

Ar⁵ and Ar⁵¹ independently represent a substituted or unsubstituted aromatic hydrocarbon group having 6 to 30 carbon atoms, a substituted or unsubstituted aromatic heterocyclic group having 3 to 17 carbon atoms, or a substituted or unsubstituted linked aromatic group in which two to seven of these aromatic rings are linked to each other. Preferred is a substituted or unsubstituted aromatic hydrocarbon group having 6 to 10 carbon atoms, a substituted or unsubstituted aromatic heterocyclic group having 3 to 12 carbon atoms, or a substituted or unsubstituted linked aromatic group in which two to seven of these aromatic rings are linked to each other.

In the case of Ar⁵ and Ar⁵¹ being each an unsubstituted aromatic hydrocarbon group, an unsubstituted linked aromatic group, and an unsubstituted linked aromatic group, specific examples thereof are the same as in the case of Ar¹ and Ar¹¹. Preferred is a group generated from pyridine, pyrimidine, triazine, dibenzofuran, dibenzothiophene, carbazole, benzene, naphthalene, or a compound formed by linking two to seven of these aromatic groups to each other.

L⁵ and L⁵¹ independently represent a substituted or unsubstituted aromatic hydrocarbon group having 6 to 30 carbon atoms, or a substituted or unsubstituted aromatic heterocyclic group having 3 to 17 carbon atoms, and at least one thereof represents the aromatic heterocyclic group. It is preferred that at least one of L⁵ and L⁵¹ is a substituted or unsubstituted nitrogen-containing 6-membered ring, or a substituted or unsubstituted ring-fused aromatic heterocyclic group in which nitrogen-containing 6-membered rings are fused.

Specific examples of the unsubstituted nitrogen-containing 6-membered ring, or the unsubstituted ring-fused aromatic heterocyclic group containing a nitrogen-containing 6-membered ring include pyridine, pyrimidine, triazine, quinoline, isoquinoline, quinoxaline, naphthyridine, or phenazine.

Specific examples of the unsubstituted aromatic hydrocarbon group or the aromatic heterocyclic group are the same as in the case where Ar¹ and Ar¹¹ are each the group except that the group is (r + 1)-valent or (s + 1)-valent. Preferred is a group generated from pyridine, pyrimidine, triazine, quinoline, isoquinoline, quinoxaline, naphthyridine, phenazine, dibenzofuran, dibenzothiophene, carbazole, benzene, or naphthalene.

Specific examples of the compounds represented by the general formula (5) are described below, but the compounds are not limited to these exemplified compounds.

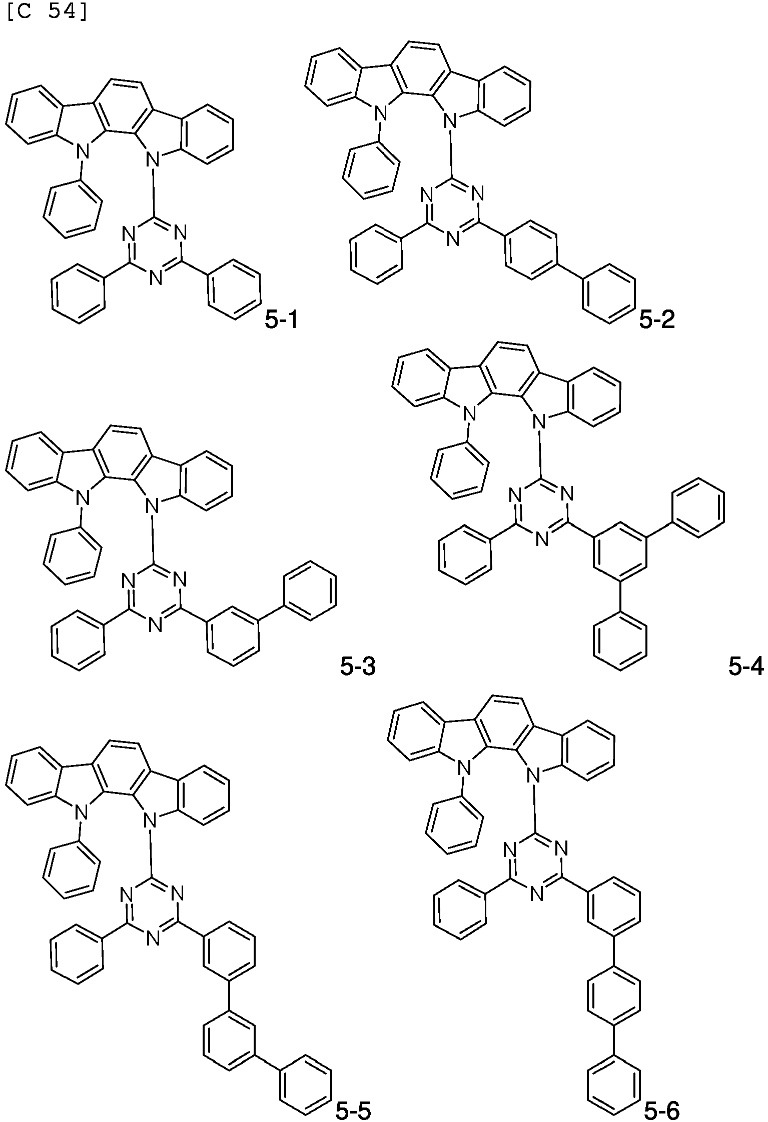

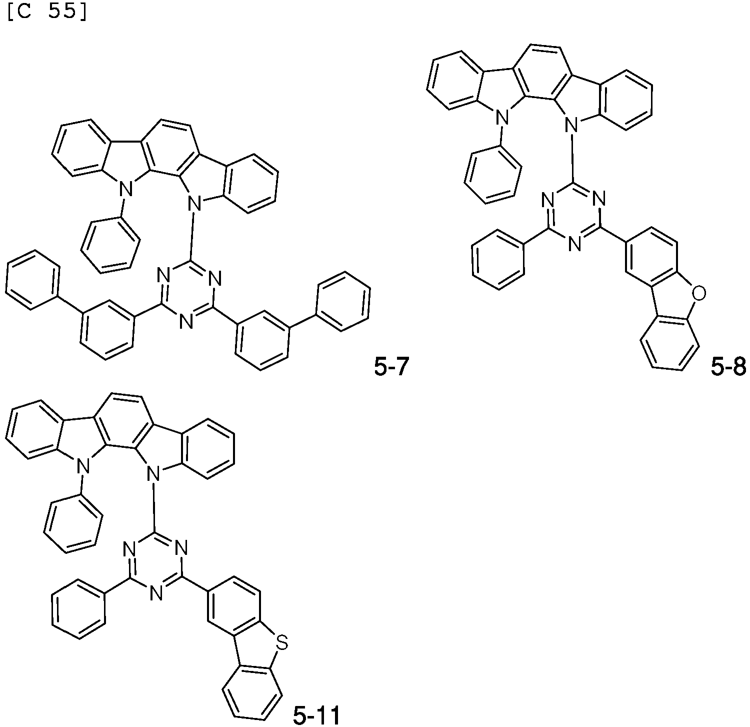

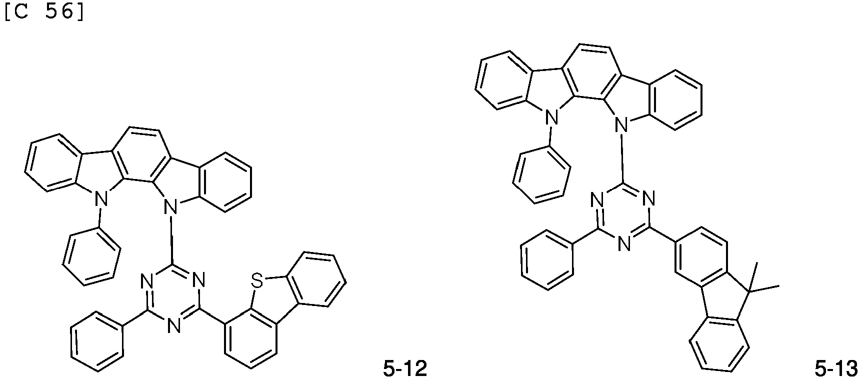

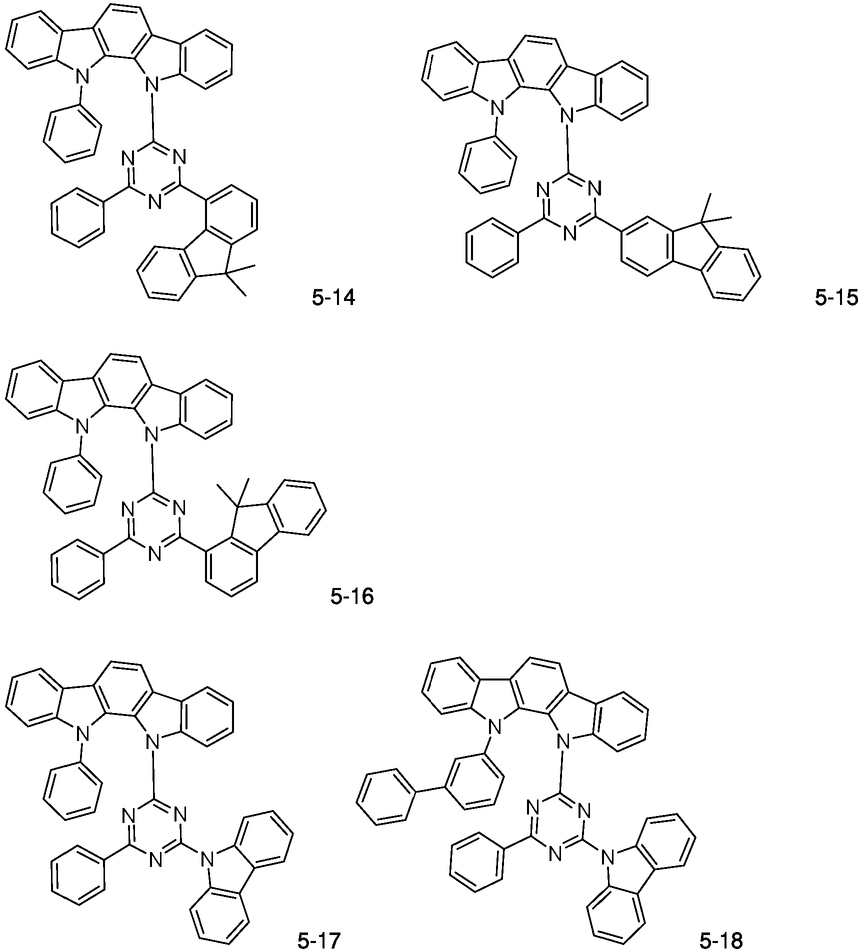

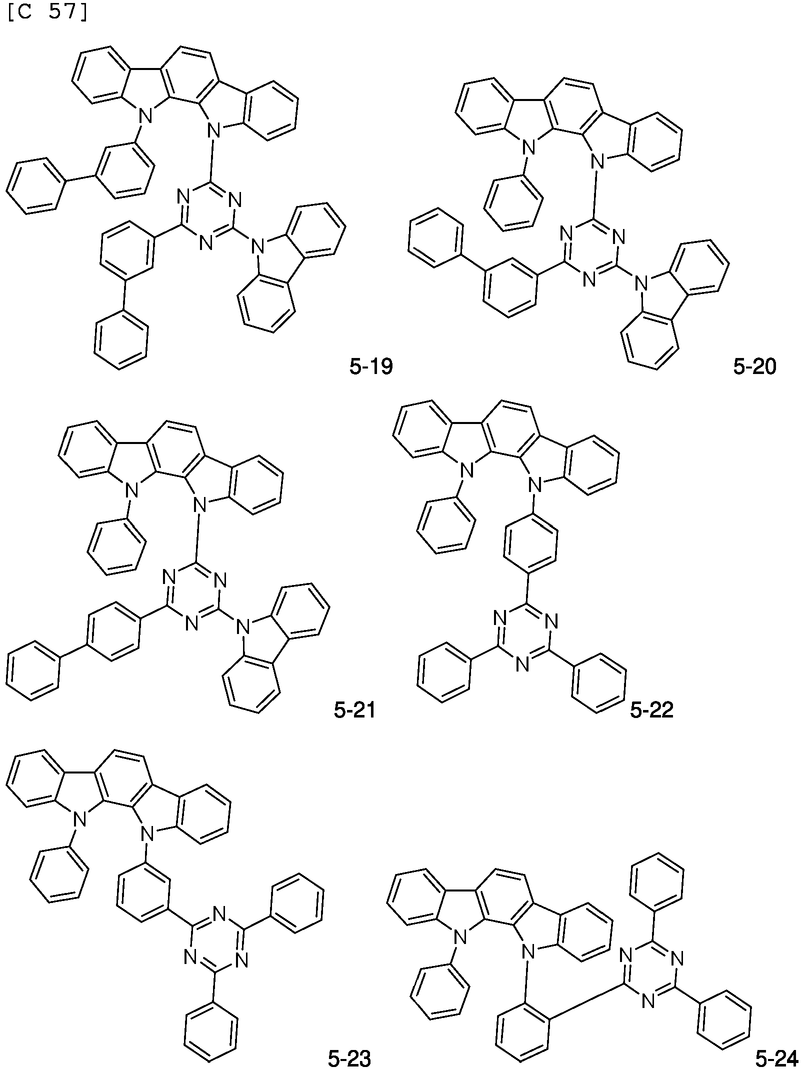

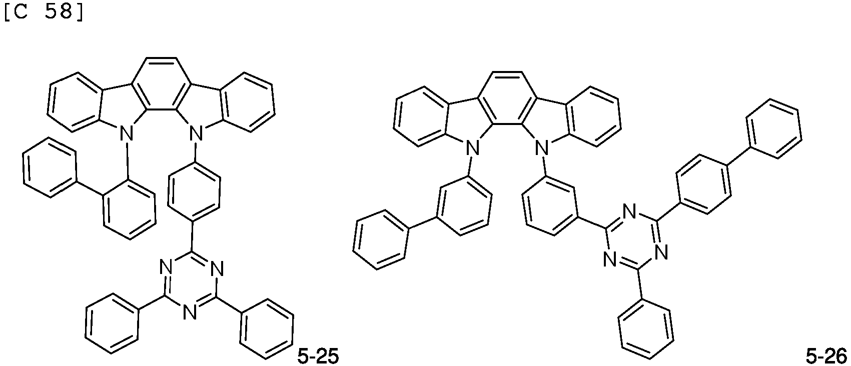

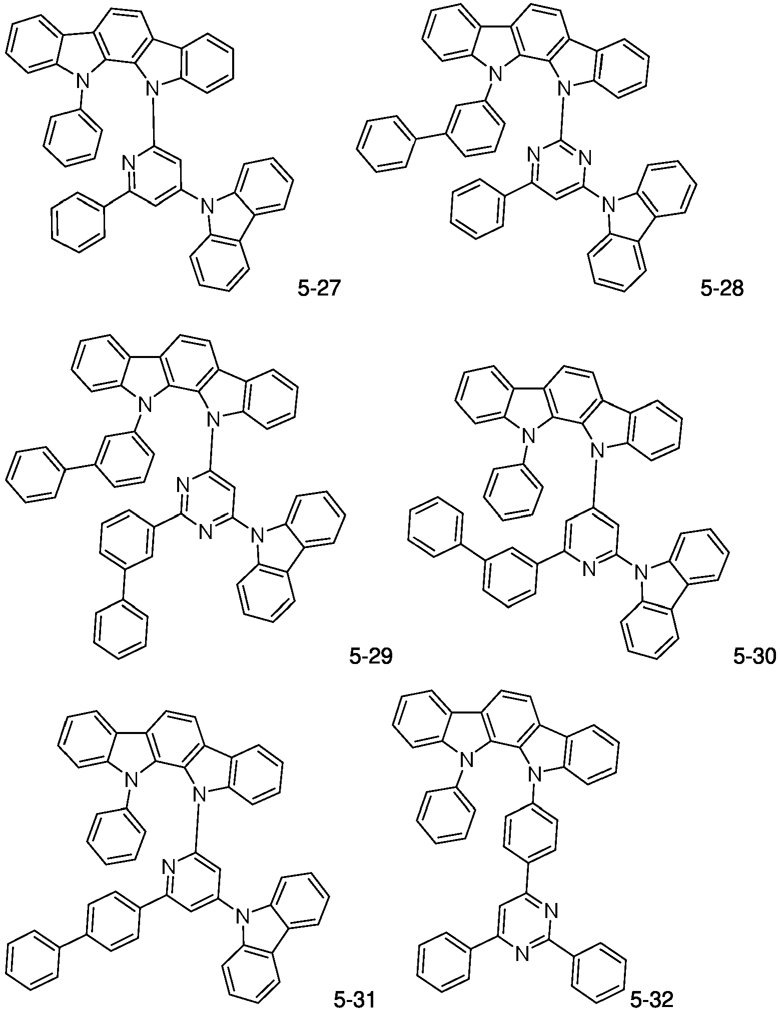

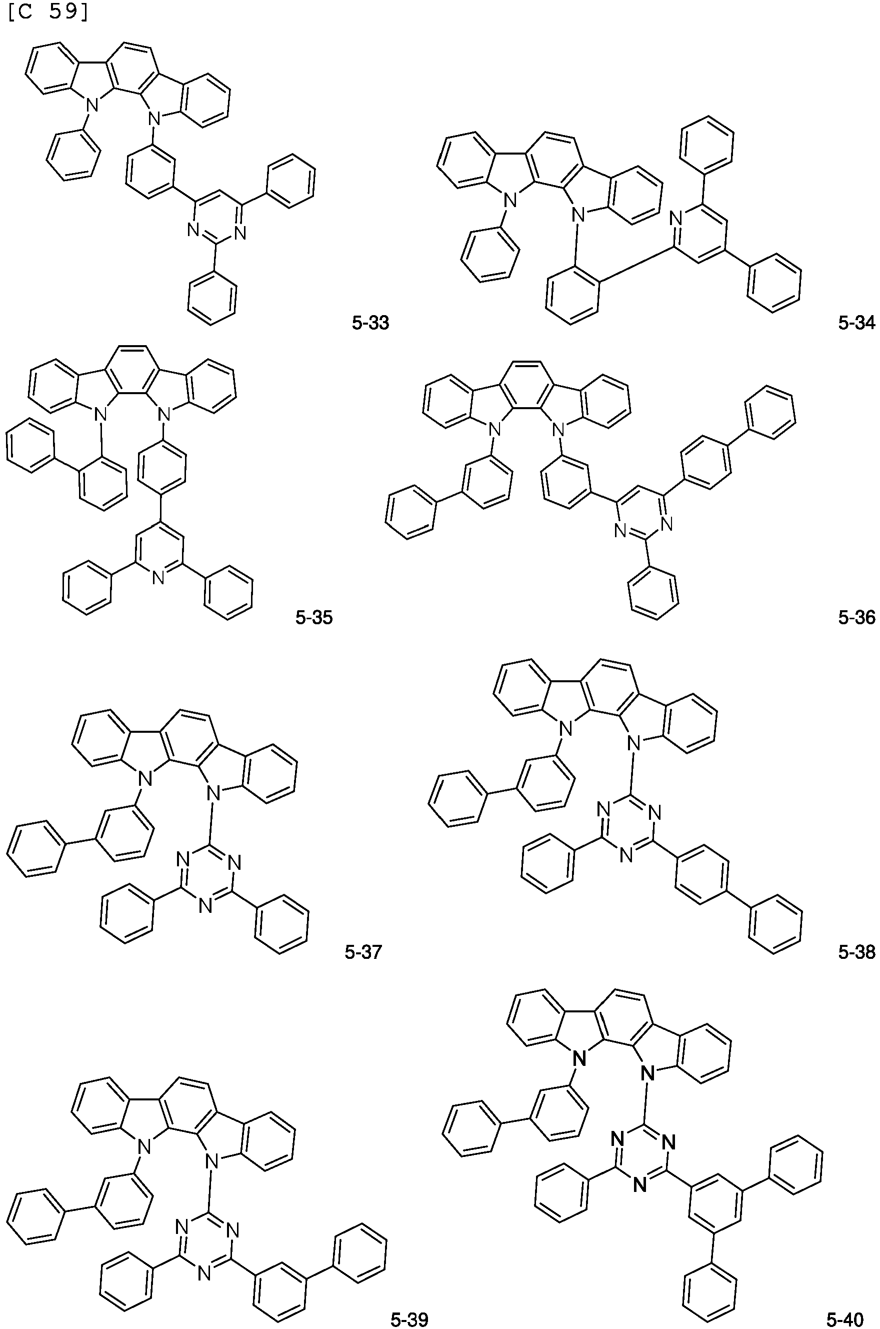

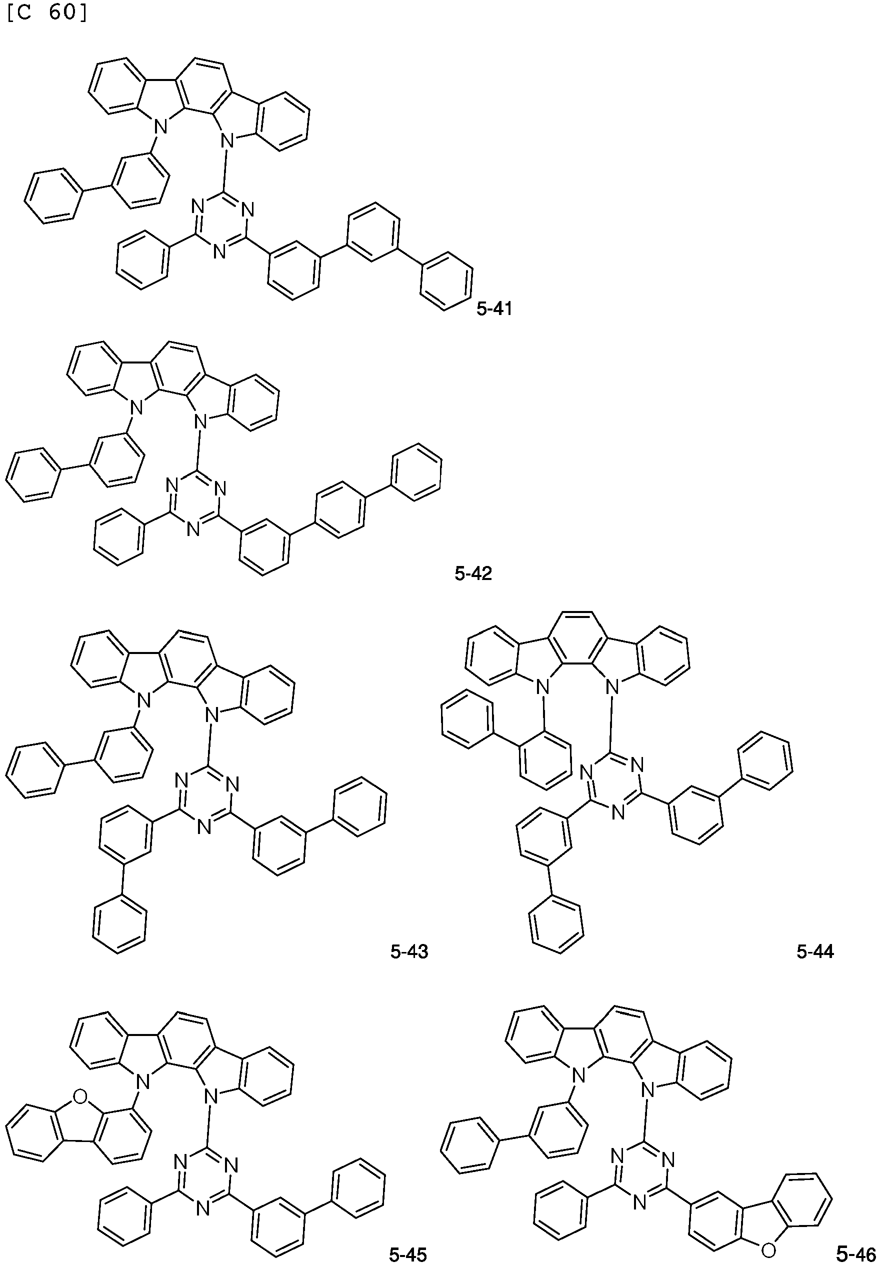

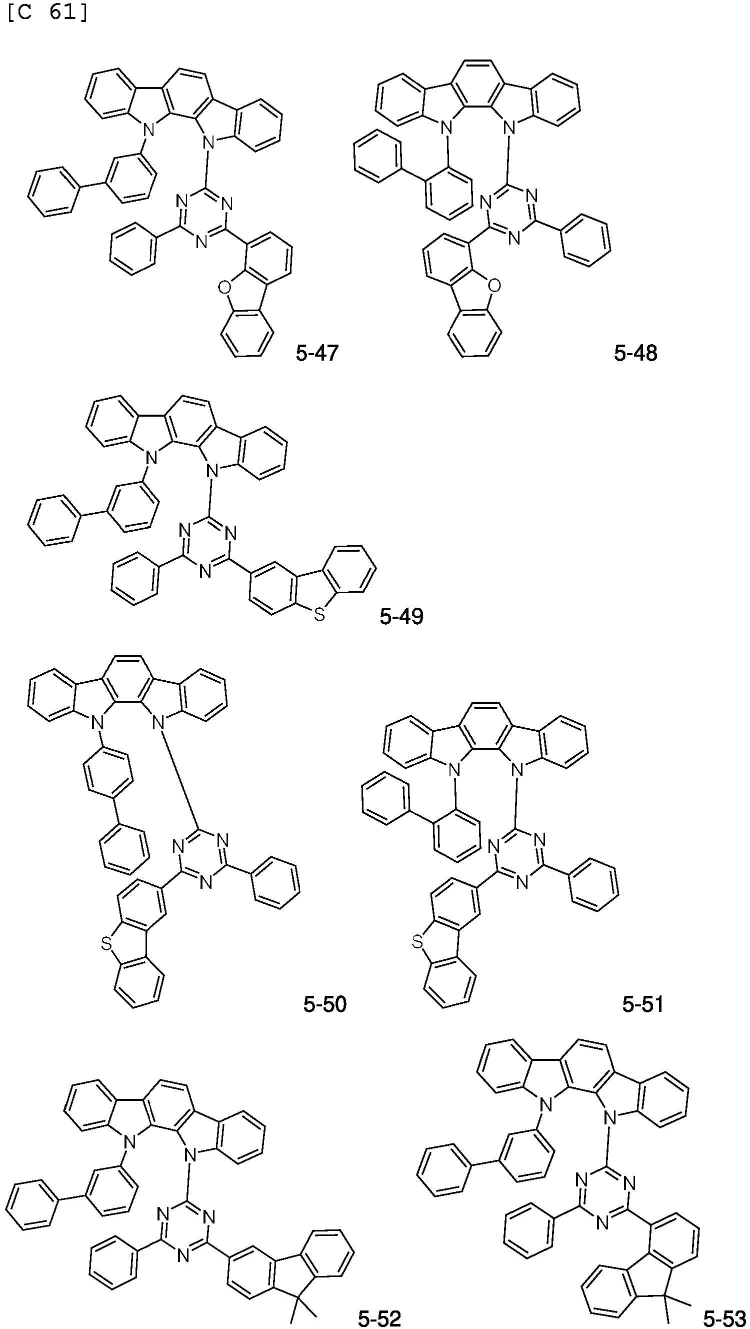

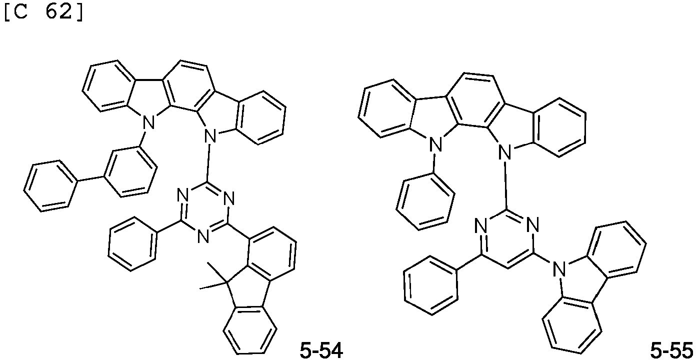

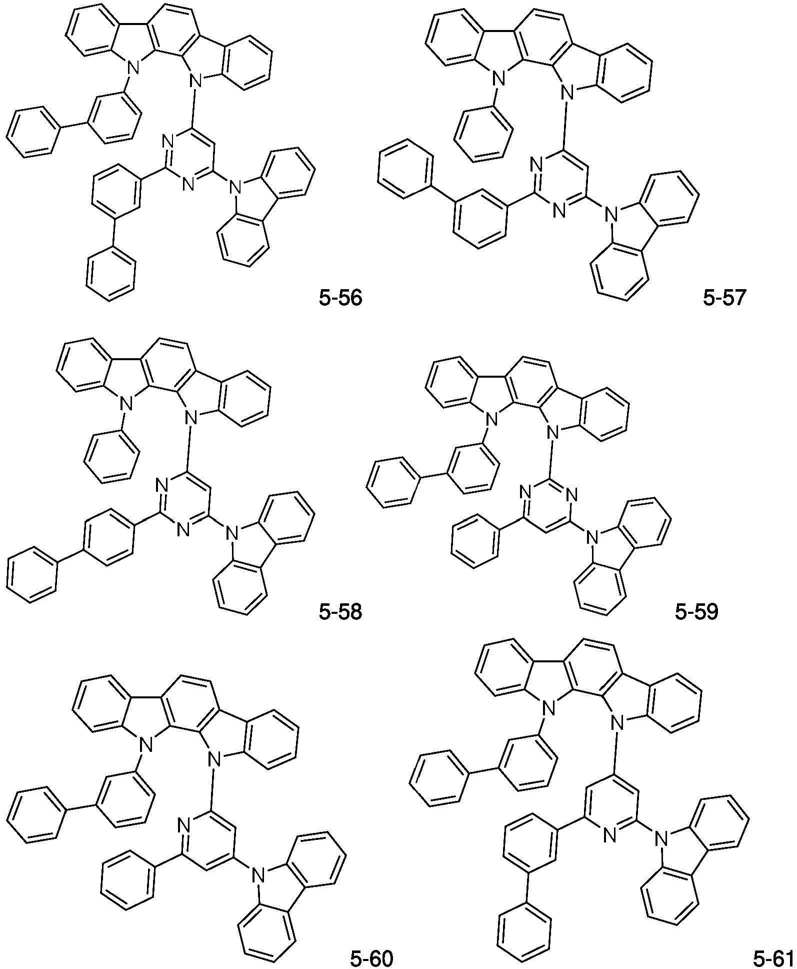

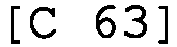

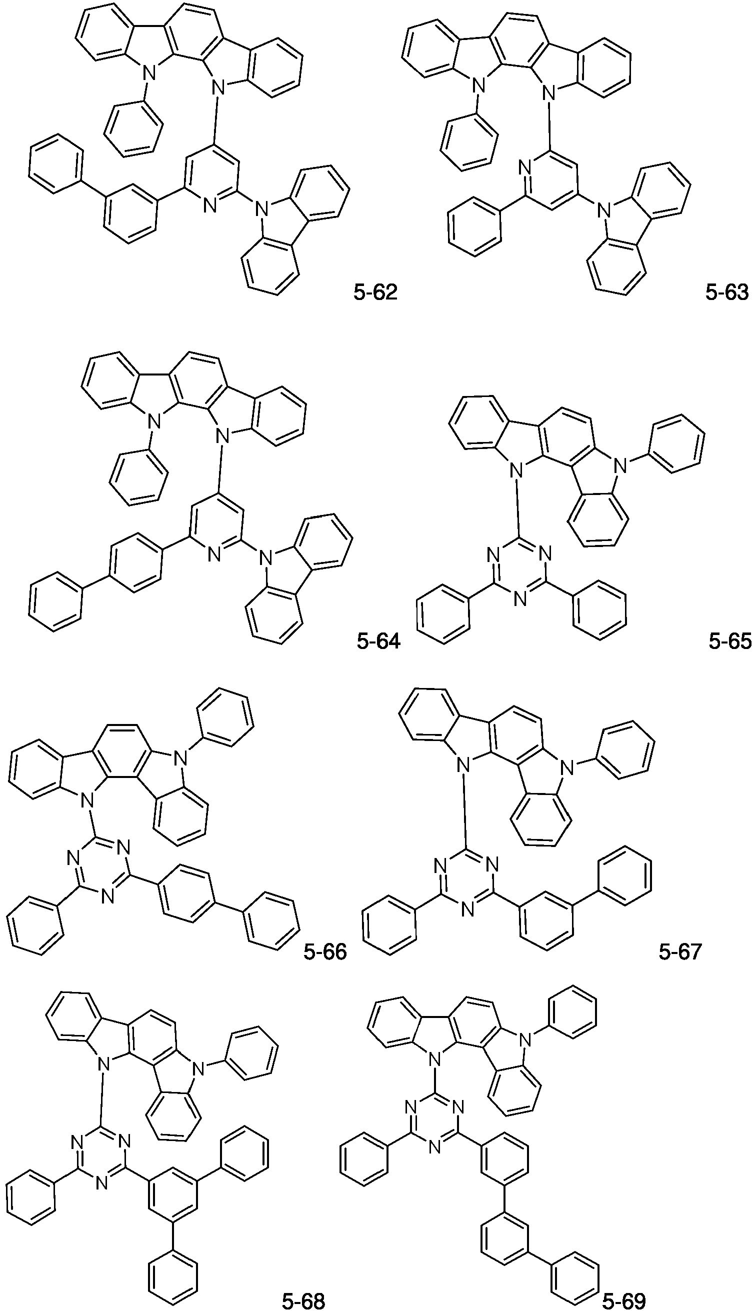

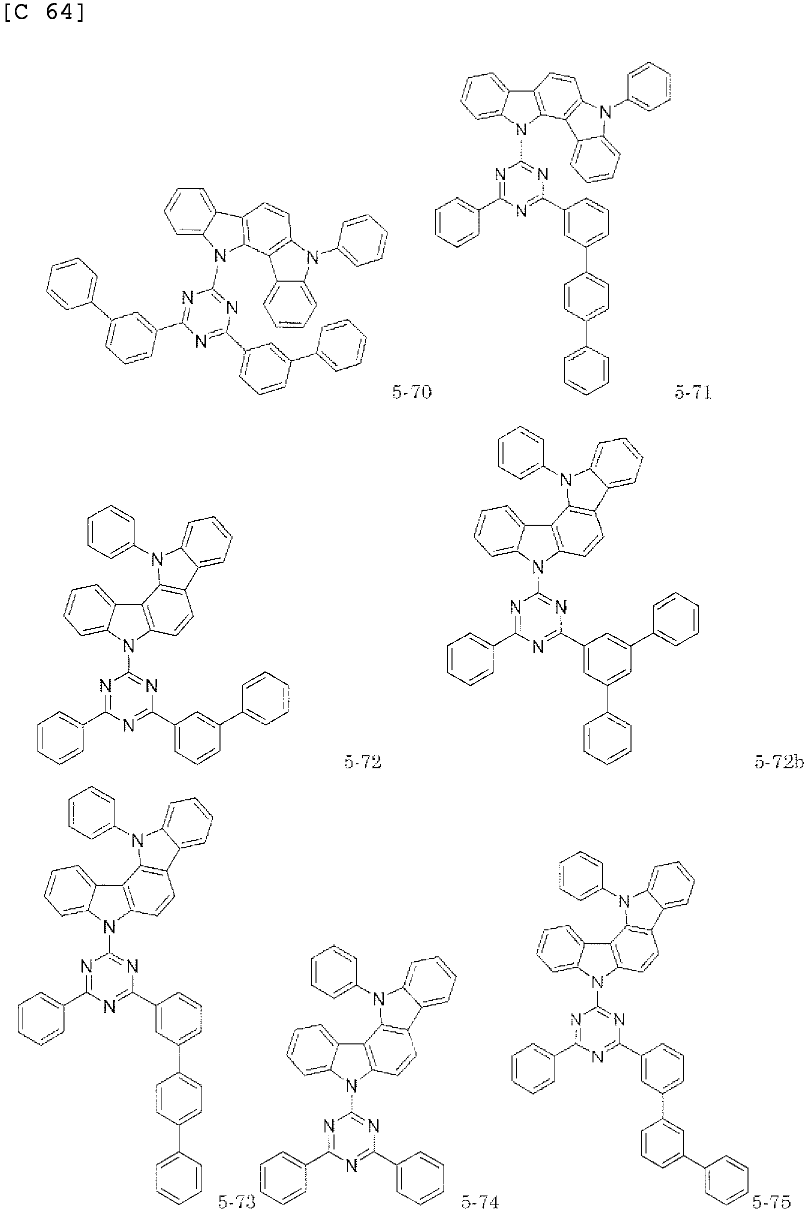

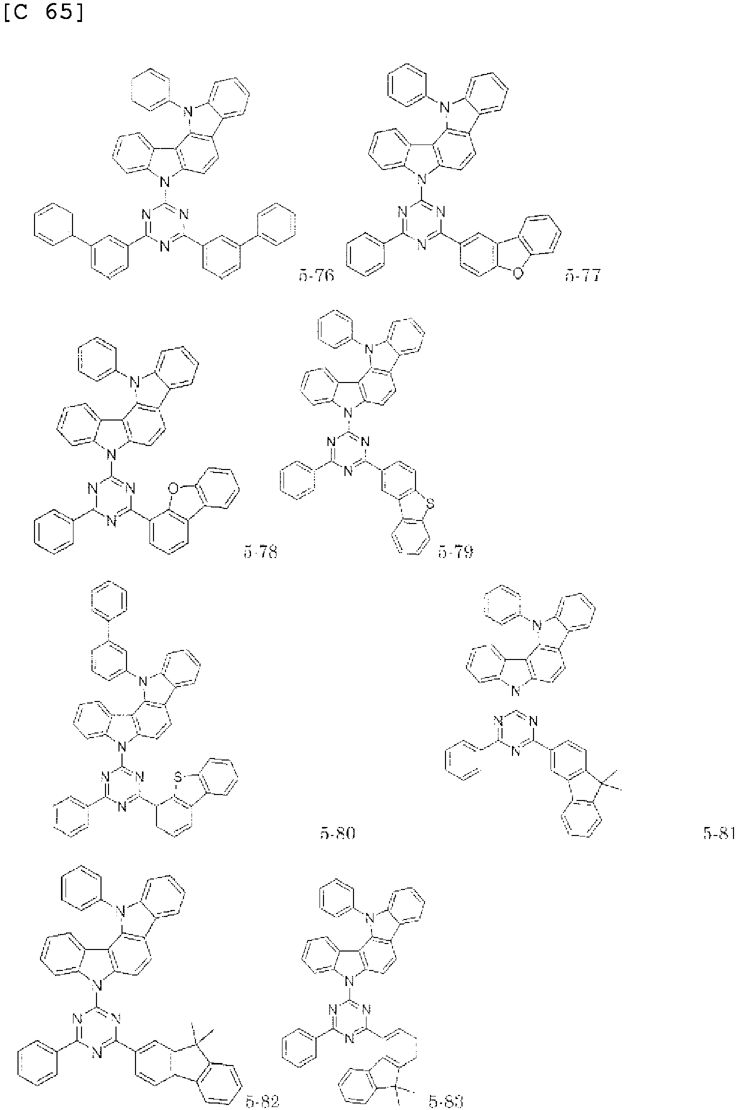

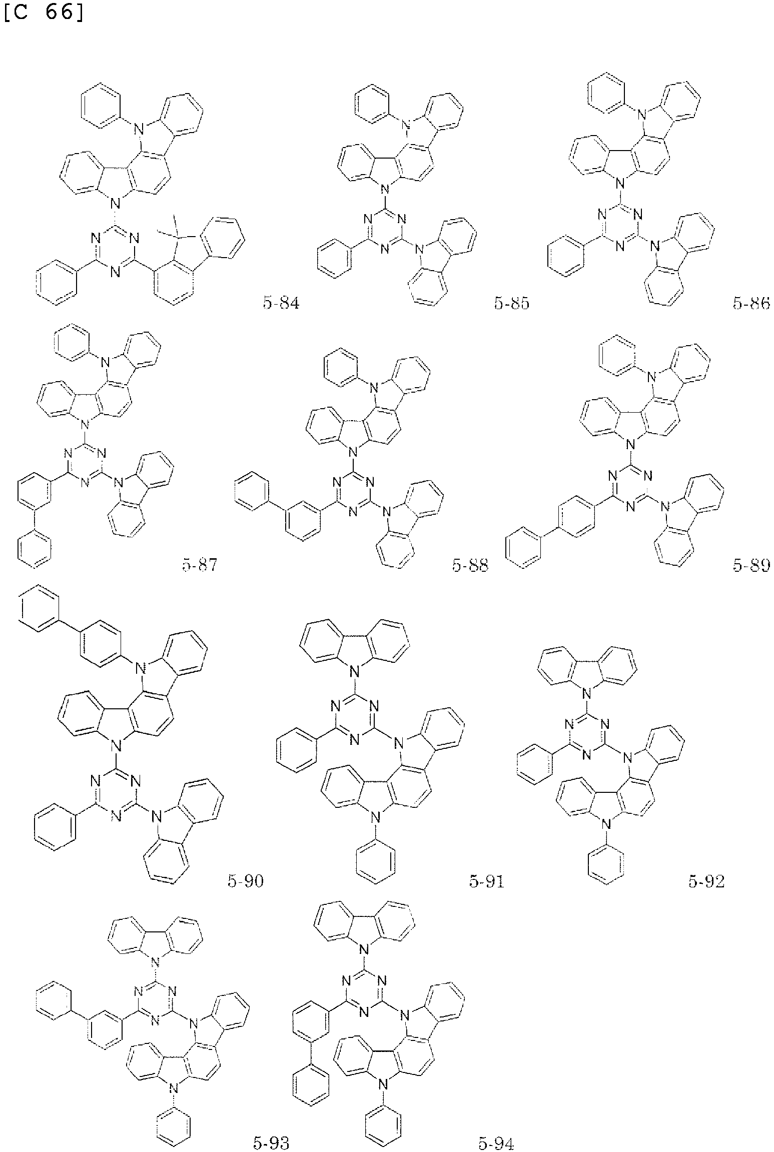

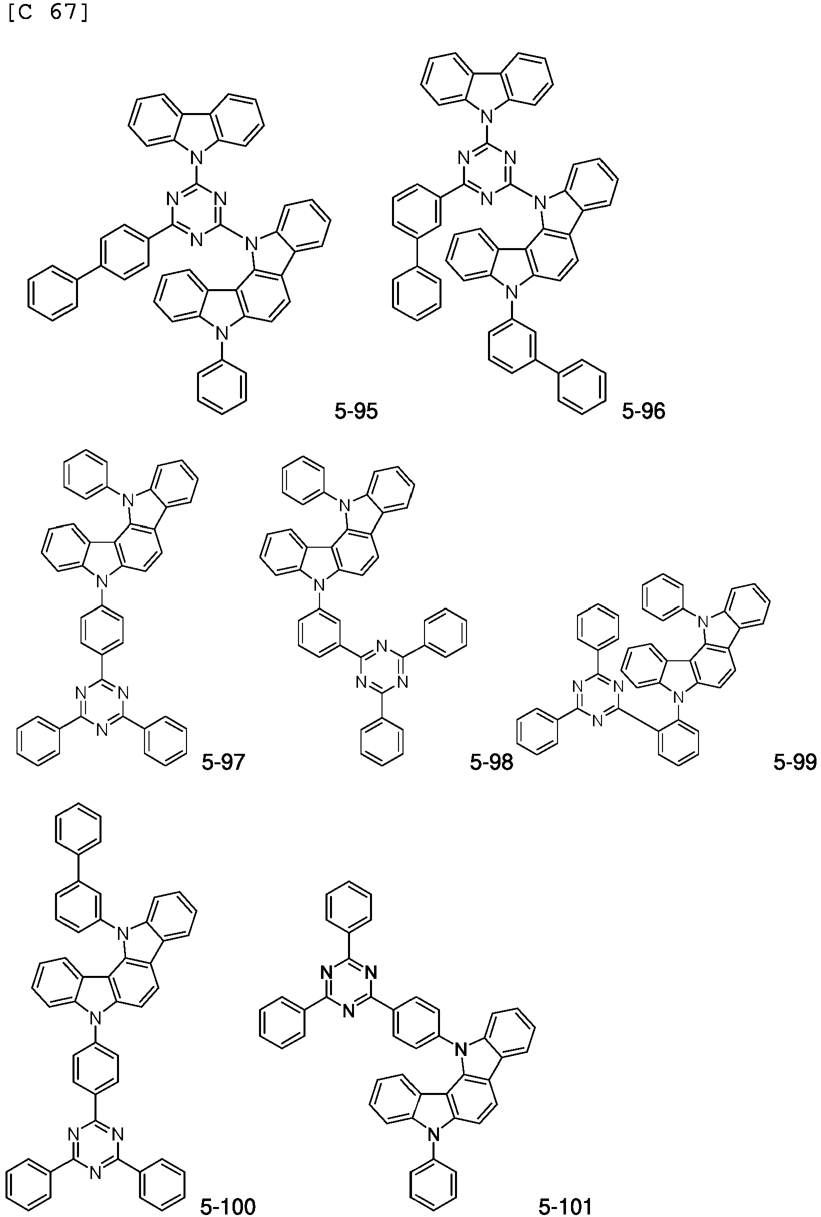

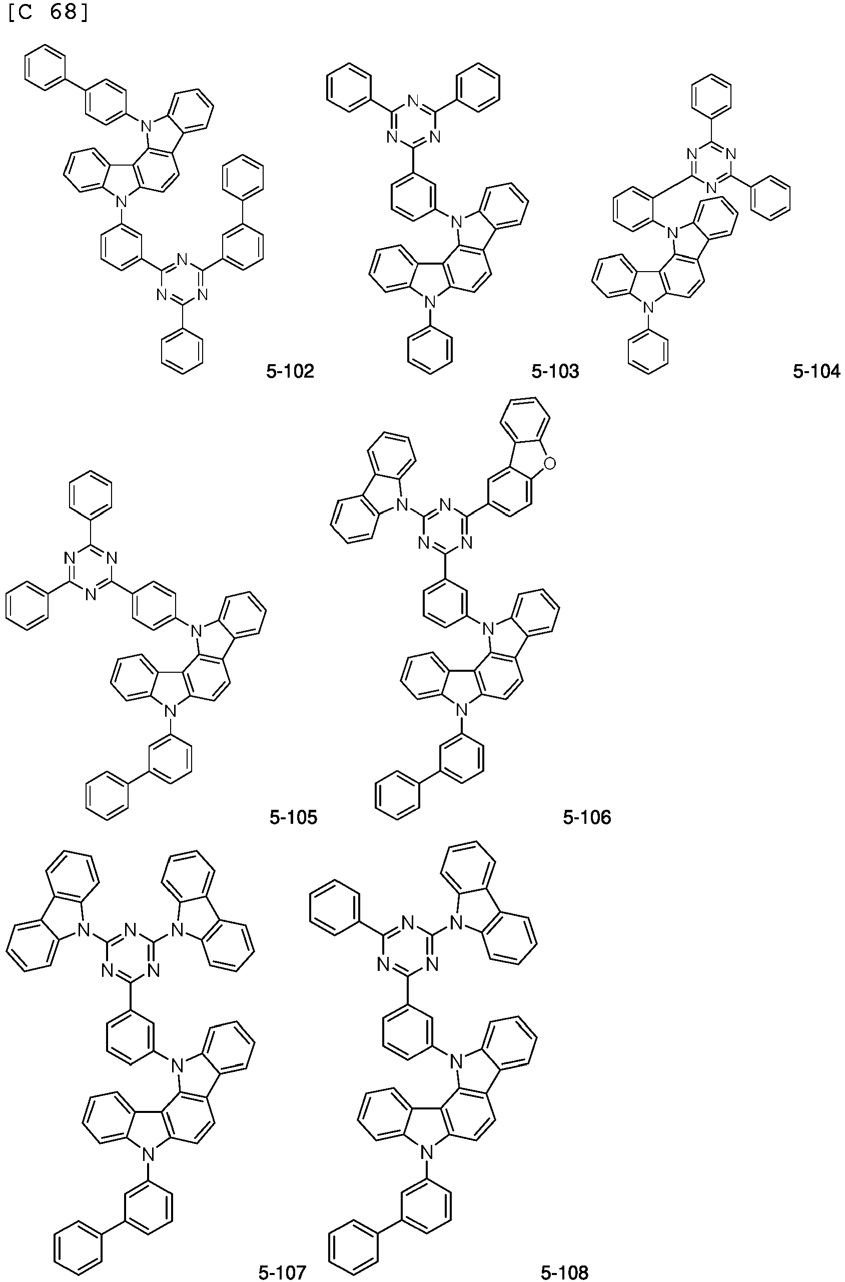

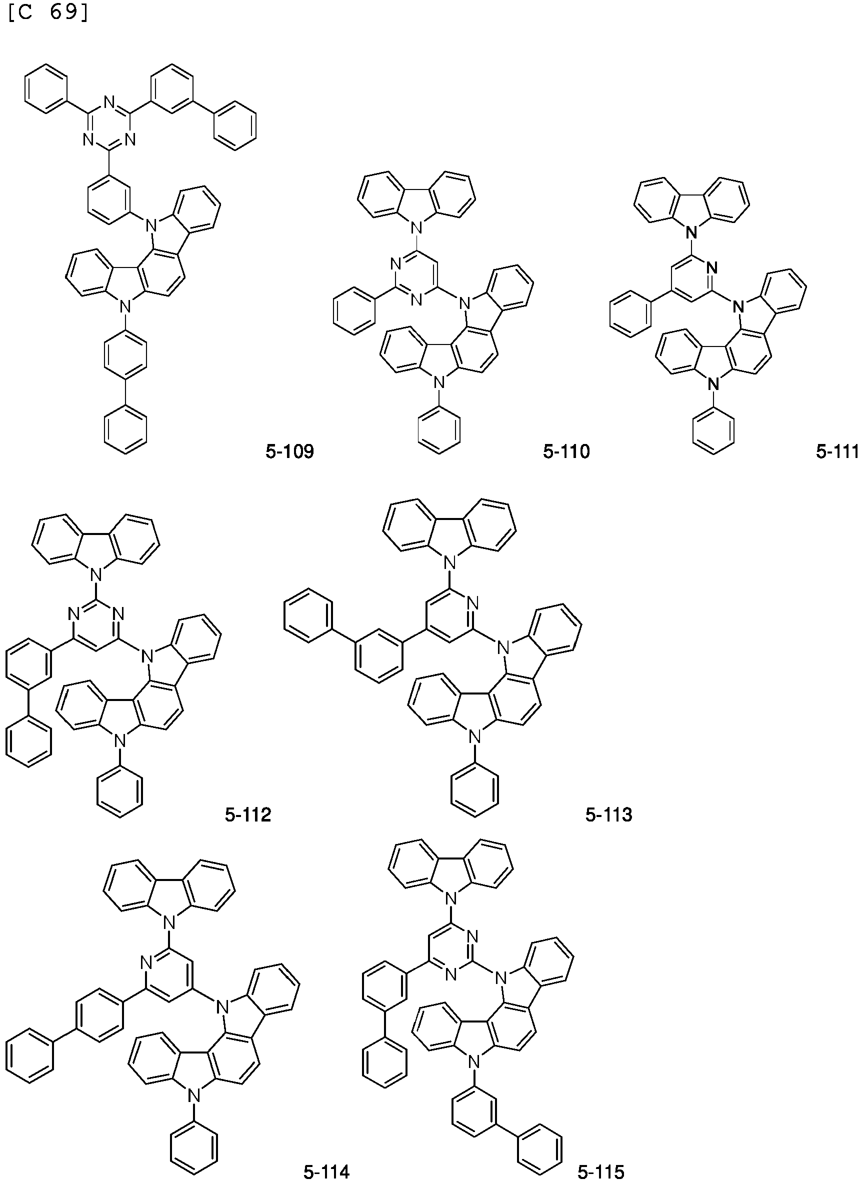

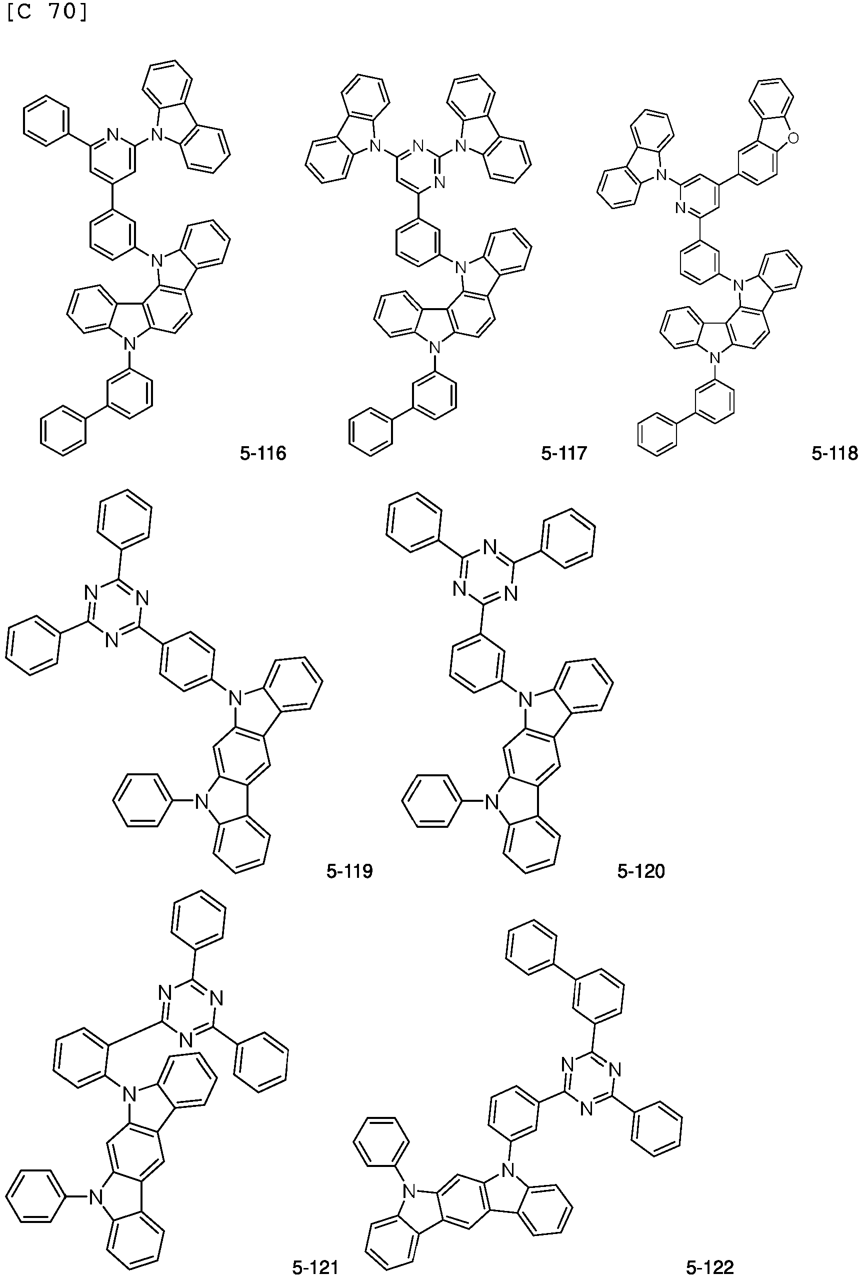

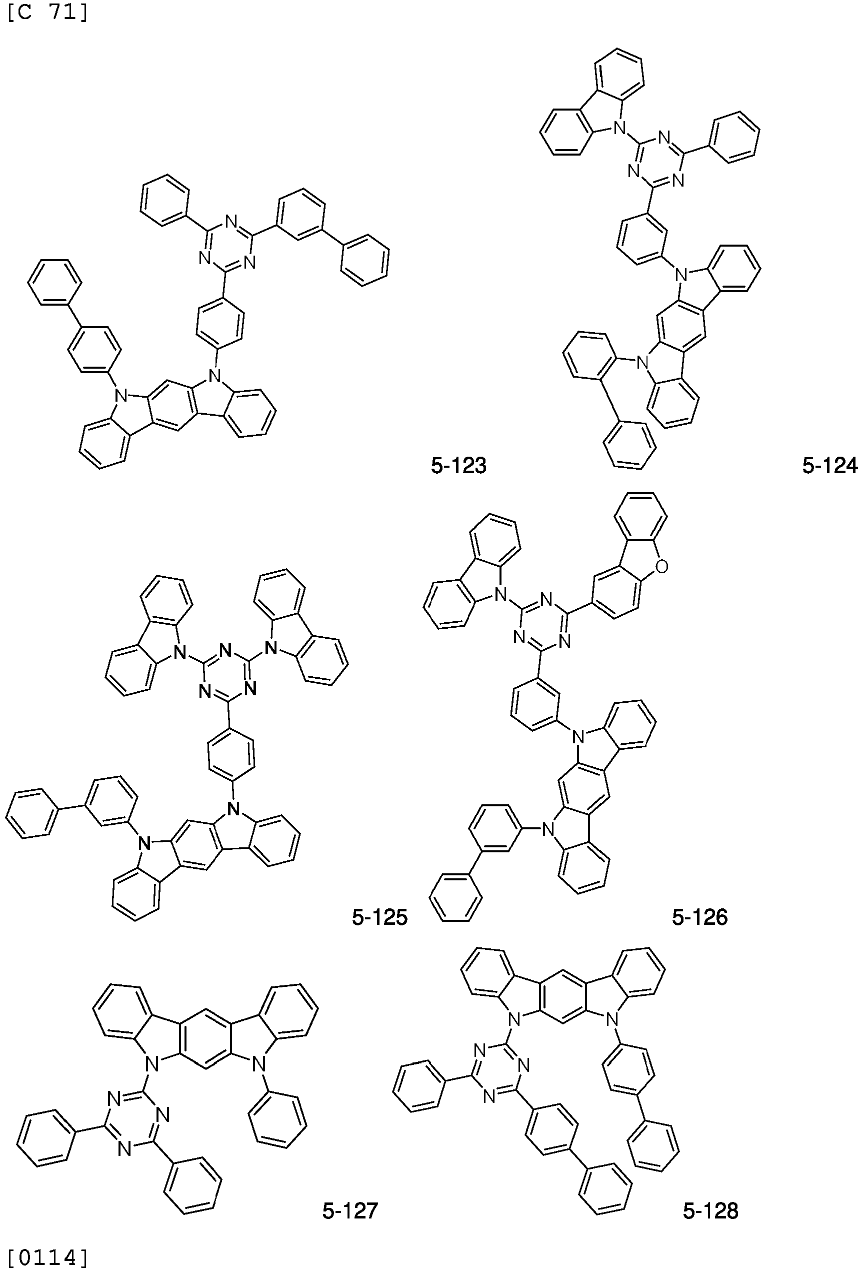

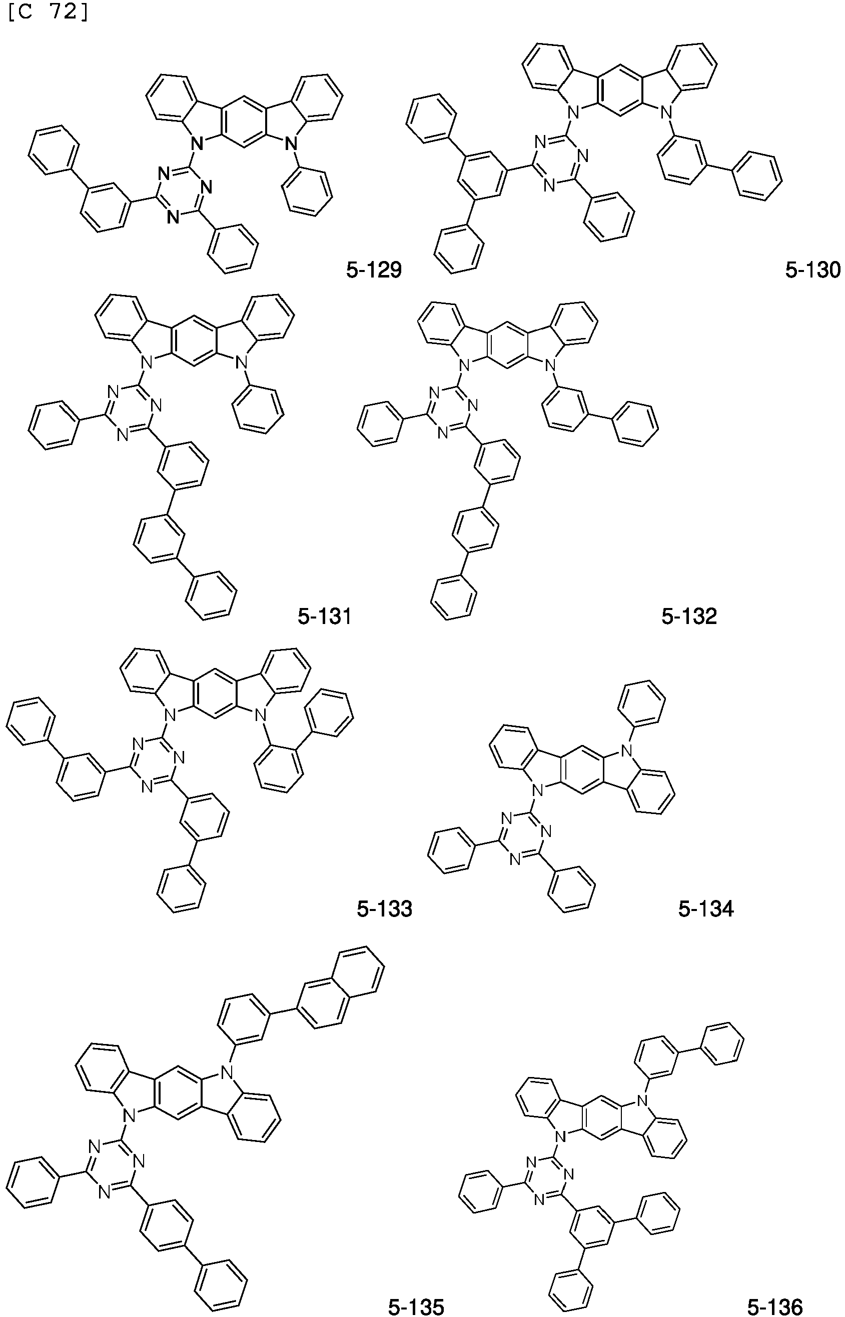

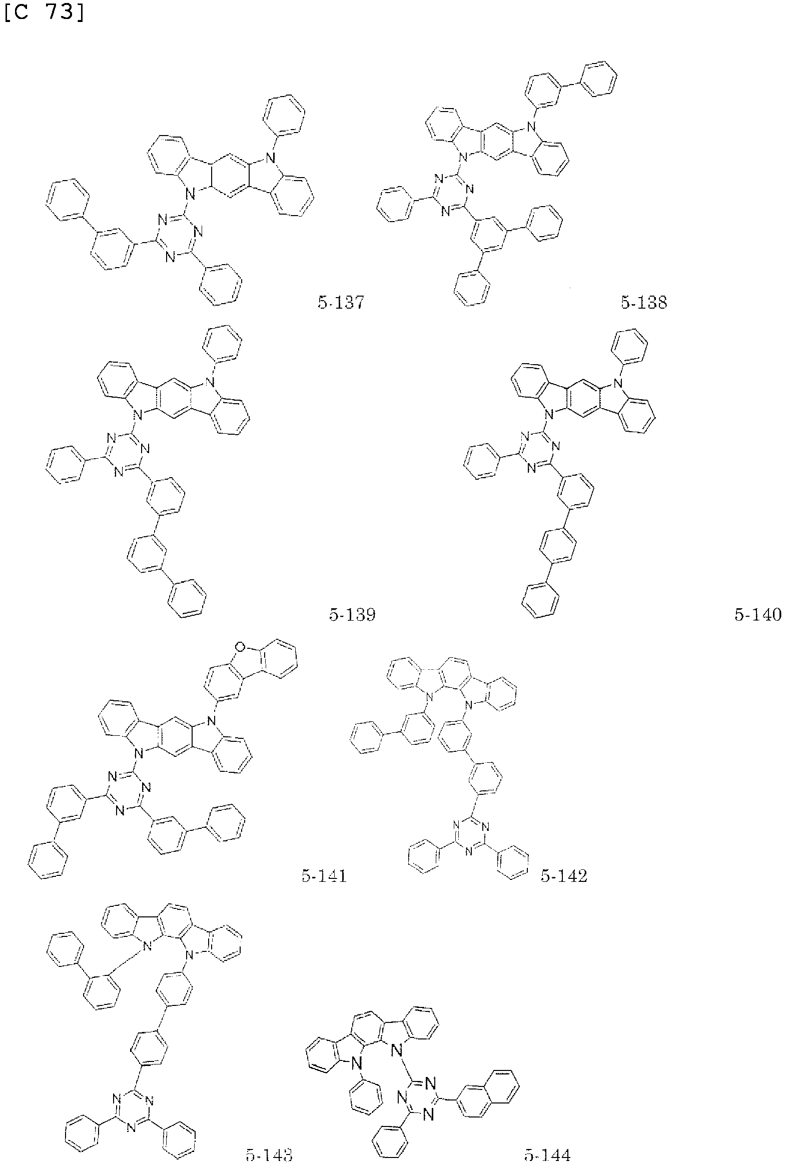

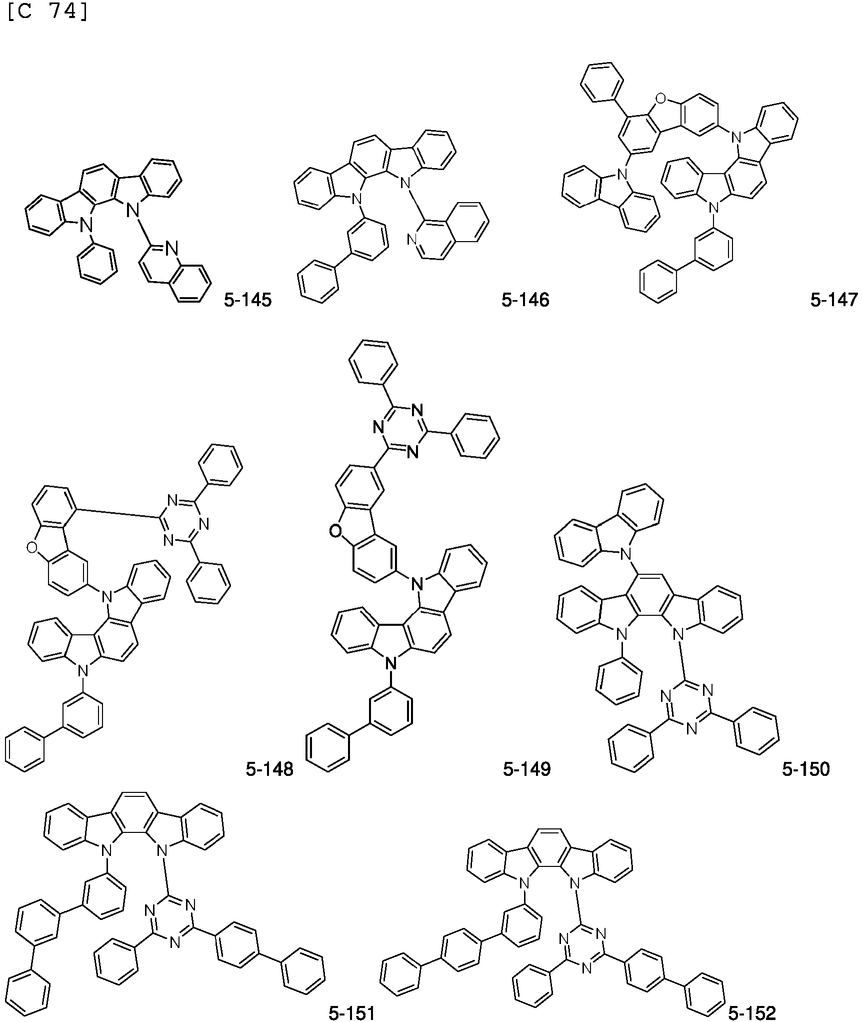

A method for producing an organic electroluminescent device of the present invention is a method for forming a light-emitting layer by vapor deposition of a host material composition including the first host material, the second host material and the third host material, and a light-emitting dopant material.

A host material composition of the present invention is a host material composition for use in the method for producing an organic electroluminescent device. The host material composition includes the first host material, the second host material and the third host material, and a dopant material.

The first host material, the second host material, the third host material and the light-emitting dopant material which are, for example, vapor deposited from different individual vapor deposition sources can be used; however, it is preferable to premix them as a host material composition (also referred to as "premixture".) before vapor deposition and to vapor deposit the premixture simultaneously from one vapor deposition source to thereby form a light-emitting layer. In this case, it is preferred to premix the host material, or the light-emitting dopant material and the host material may be mixed, and when there is a large difference in temperatures to provide desired vapor pressure, vapor deposition may be performed from another vapor deposition source.

The premixing method is desirably a method that can allow for mixing as uniformly as possible, and examples thereof include pulverization and mixing, a heating and melting method under reduced pressure or under an atmosphere of an inert gas such as nitrogen, and sublimation, but not limited thereto.

The first host material has a LUMO energy of -1.95 eV or less, and is a host material having the lowest LUMO energy among the first, second and third host materials. The third host material has a LUMO energy of -1.94 to - 1.77 eV, and is a material having the next lowest LUMO energy to the first host material. The second host material has a LUMO energy of -1.54 eV or more, and is a host material having the highest LUMO energy among the first, second and third host materials.

In addition, regarding the mixing ratio (weight ratio) among the first host material, the second host material and the third host material, the proportion of the first host material is larger than 1.0 wt% and less than 30 wt% and the proportion of the third host material is larger than 5.0 wt% and less than 80 wt% based on the first host material, the second host material and the third host material in total. It is preferred that the proportion of the first host material is 2 to 20 wt% and the proportion of the third host material is 10 to 70 wt%, and it is more preferred that the proportion of the first host material is 5 to 20 wt% and the proportion of the third host material is 10 to 60 wt%. The proportion of the second host material is a value obtained by subtracting the proportions of the first host material and the third host material from 100 wt%, and is preferably 19 to 80 wt%.

Each of the triplet excitation (T1) energies of the first host material, the second host material and the third host material is preferably 2.55 eV or more.

The LUMO energies and the T1 energies can be obtained by quantum chemical calculation. The LUMO energy prescribed herein is a value calculated by performing structure optimization calculation at the B3LYP/6-31G* level with molecular orbital method program Gaussian 03 according to the density functional theory (DFT).

Next, the structure of the organic electroluminescent device of the present invention will be described by referring to the drawing, but the structure of the organic electroluminescent device of the present invention is not limited thereto.

Figure 1 is a cross-sectional view showing a structure example of an organic electroluminescent device generally used for the present invention, in which there are indicated a substrate 1, an anode 2, a hole injection layer 3, a hole transport layer 4, a light-emitting layer 5, an electron transport layer 6, and a cathode 7. The organic electroluminescent device of the present invention may have an exciton blocking layer adjacent to the light-emitting layer and may have an electron blocking layer between the light-emitting layer and the hole injection layer. The exciton blocking layer can be inserted into either of the anode side and the cathode side of the light-emitting layer and inserted into both sides at the same time. The organic electroluminescent device of the present invention has the anode, the light-emitting layer, and the cathode as essential layers, and preferably has a hole injection transport layer and an electron injection transport layer in addition to the essential layers, and further preferably has a hole blocking layer between the light-emitting layer and the electron injection transport layer. Note that the hole injection transport layer refers to either or both of a hole injection layer and a hole transport layer, and the electron injection transport layer refers to either or both of an electron injection layer and an electron transport layer.

A structure reverse to that of Figure 1 is applicable, in which a cathode 7, an electron transport layer 6, a light-emitting layer 5, a hole transport layer 4, and an anode 2 are stacked on a substrate 1 in this order. In this case, layers may be added or omitted as necessary.

### -- Substrate --

The organic electroluminescent device of the present invention is preferably supported on a substrate. The substrate is not particularly limited, and those conventionally used in organic electroluminescent devices may be used, and substrates made of, for example, glass, a transparent plastic, or quartz may be used.

### -- Anode --

Regarding an anode material for an organic electroluminescent device, it is preferable to use a material selected from a metal, an alloy, an electrically conductive compound or a mixture thereof having a large work function (4 eV or more).

Specific examples of such an electrode material include a metal such as Au, and a conductive transparent material such as CuI, indium tin oxide (ITO), SnO₂, and ZnO. In addition, an amorphous material such as IDIXO (In₂O₃-ZnO), which is capable of forming a transparent conductive film, may be used. Regarding the anode, such an electrode material is used to form a thin film by, for example, a vapor-deposition or sputtering method, and a desired shape pattern may be formed by a photolithographic method; or if the pattern accuracy is not particularly required (about 100 µm or more), a pattern may be formed via a desired shape mask when the electrode material is vapor deposited or sputtered. Alternatively, when a coatable substance such as an organic conductive compound is used, a wet film formation method such as a printing method or a coating method may be used. For taking emitted light from the anode, it is desired to have a transmittance of more than 10%, and the sheet resistance for the anode is preferably several hundreds Ω/□ or less. The film thickness is selected usually within 10 to 1000 nm, preferably within 10 to 200 nm though depending on the material.

### -- Cathode --

Meanwhile, regarding a cathode material, preferable to a material selected from a metal (an electron injection metal), an alloy, an electrically conductive compound, or a mixture thereof having a small work function (4 eV or less) are used. Specific examples of such an electrode material include sodium, a sodium-potassium alloy, magnesium, lithium, a magnesium/copper mixture, a magnesium/silver mixture, a magnesium/aluminum mixture, a magnesium/indium mixture, an aluminum/aluminum oxide (Al₂O₃) mixture, indium, a lithium/aluminum mixture, and a rare earth metal. Among these, from the viewpoint of the electron injectability and the durability against oxidation and the like, a mixture of an electron injection metal and a second metal which is a stable metal having a larger work function value is suitable, and examples thereof include a magnesium/silver mixture, a magnesium/aluminum mixture, a magnesium/indium mixture, an aluminum/aluminum oxide mixture, a lithium/aluminum mixture and aluminum. The cathode can be produced by forming a thin film by a method such as vapor-depositing or sputtering of such a cathode material. In addition, the sheet resistance of cathode is preferably several hundreds Ω/□ or less. The film thickness is selected usually within 10 nm to 5 µm, preferably within 50 to 200 nm. Note that for transmission of emitted light, if either one of the anode and cathode of the organic electroluminescent device is transparent or translucent, emission luminance is improved, which is convenient.

In addition, formation of a film of the above metal with a thickness of 1 to 20 nm on the cathode, followed by formation of a conductive transparent material described in the description on the anode thereon, enables production of a transparent or translucent cathode, and application of this enables production of a device wherein an anode and a cathode both have transmittance.

### -- Light-emitting Layer --

The light-emitting layer is a layer that emits light after excitons are generated when holes and electrons injected from the anode and the cathode, respectively, are recombined. The light-emitting layer contains an organic light-emitting dopant material and a host material.

As a host material, the first host material, the second host material and the third host material are used.

Regarding the compound represented by the general formula (1) as the first host material, one kind thereof may be used, or two or more kinds thereof may be used. Likewise, regarding the carbazole compound or indolocarbazole compound represented by the general formulas (2) to (4) as the second host material, one kind thereof may be used, or two or more kinds thereof may be used. Regarding to the compound represented by the general formula (5), as the third host material, one kind thereof may be used, or two or more kinds thereof may be used.

If necessary, a material other than the first host material, the second host material and the third host material may be added.

When a plurality of the host materials, or the host materials and a dopant are premixed and used, it is desirable that a difference in 50% weight reduction temperature (T₅₀) be small in order to produce an organic electroluminescent device having favorable characteristics with high reproducibility. The 50% weight reduction temperature is a temperature at which the weight is reduced by 50% when the temperature is raised to 550°C from room temperature at a rate of 10°C/min in TG-DTA measurement under a nitrogen stream reduced pressure (1 Pa). It is considered that vaporization due to evaporation or sublimation the most vigorously occurs around this temperature.

The difference in 50% weight reduction temperatures of the materials for use in the premixture is preferably within 15°C and more preferably within 12°C.

The premixing method is desirably a method that can allow for mixing as uniformly as possible, and examples thereof include pulverization and mixing, a heating and melting method under reduced pressure or under an atmosphere of an inert gas such as nitrogen, and sublimation, but not limited thereto.

The forms of the hosts and the premixture thereof may be powdery, stick-shaped, or granular.

When a phosphorescent dopant is used as a light-emitting dopant material, preferred is a phosphorescent dopant including an organic metal complex containing at least one metal selected from ruthenium, rhodium, palladium, silver, rhenium, osmium, iridium, platinum and gold. Specifically, iridium complexes described in J. Am. Chem. Soc. 2001, 123, 4304 and JP2013-530515A are preferably used, but the phosphorescent dopant is not limited thereto.

Regarding the phosphorescent dopant material, only one kind thereof may be contained in the light-emitting layer, or two or more kinds thereof may be contained. A content of the phosphorescent dopant material is preferably 0.10 to 30 wt% and more preferably 1.0 to 20 wt% with respect to the host material.

The phosphorescent dopant material is not particularly limited, and specific examples thereof include the following.

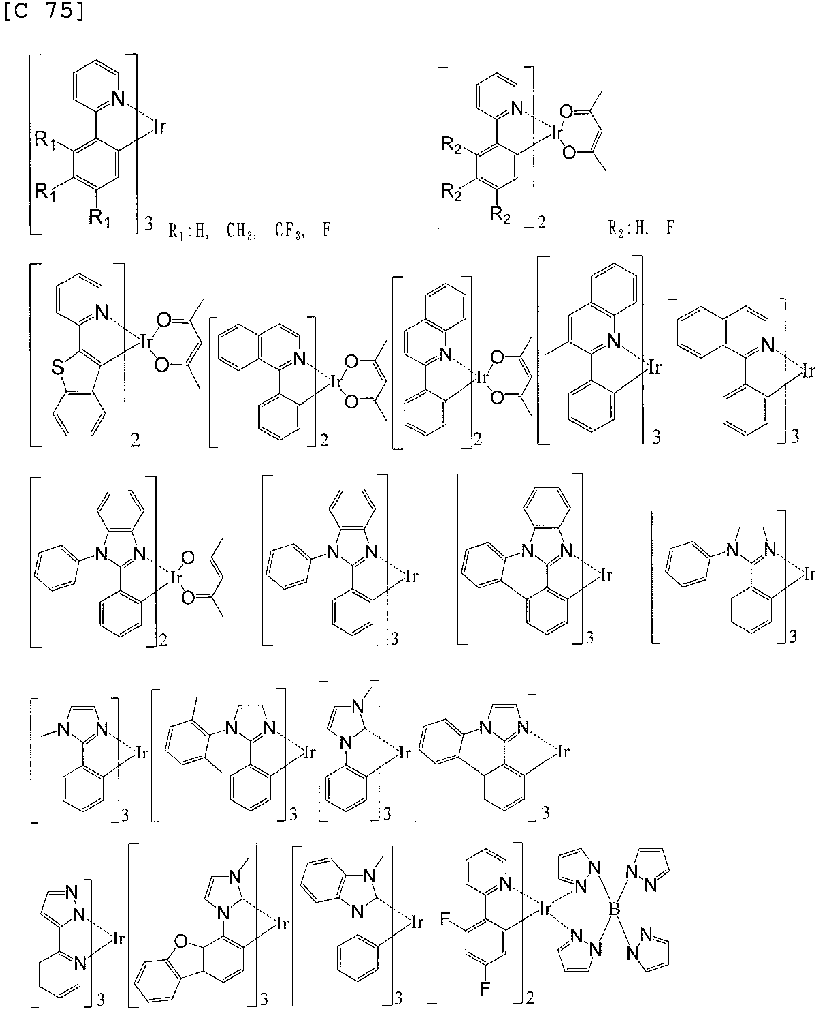

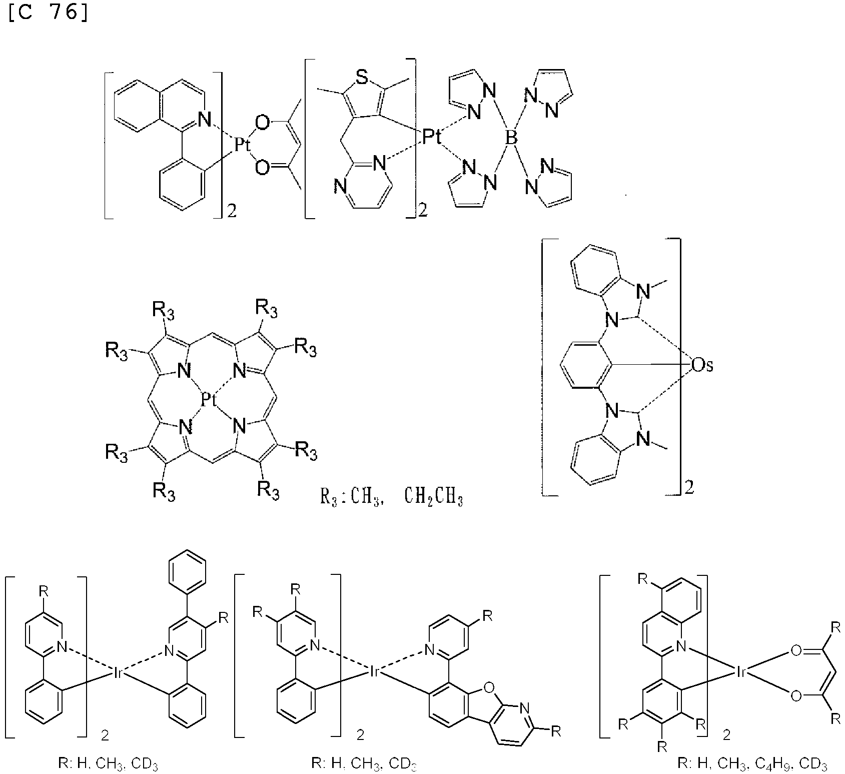

When a fluorescence-emitting dopant is used as the light-emitting dopant material, the fluorescence-emitting dopant is not particularly limited. Examples thereof include benzoxazole derivatives, benzothiazole derivatives, benzimidazole derivatives, styrylbenzene derivatives, polyphenyl derivatives, diphenylbutadiene derivatives, tetraphenyl butadiene derivatives, naphthalimido derivatives, coumarin derivatives, fused aromatic compounds, perinone derivatives, oxadiazole derivatives, oxazine derivatives, aldazine derivatives, pyrrolidine derivatives, cyclopentadiene derivatives, bisstyryl anthracene derivatives, quinacridone derivatives, pyrrolopyridine derivatives, thiadiazolopyridine derivatives, styrylamine derivatives, diketopyrrolopyrrole derivatives, aromatic dimethylidine compounds, metal complexes of 8-quinolinol derivatives or metal complexes of pyromethene derivatives, rare earth complexes, various metal complexes represented by transition metal complexes, polymer compounds such as polythiophene, polyphenylene, and polyphenylene vinylene, and organosilane derivatives. Preferred examples thereof include fused aromatic derivatives, styryl derivatives, diketopyrrolopyrrole derivatives, oxazine derivatives, pyromethene metal complexes, transition metal complexes, and lanthanoid complexes. More preferable examples thereof include naphthalene, pyrene, chrysene, triphenylene, benzo[c]phenanthrene, benzo[a]anthracene, pentacene, perylene, fluoranthene, acenaphthofluoranthene, dibenzo[a,j]anthracene, dibenzo[a,h]anthracene, benzo[a]naphthalene, hexacene, naphtho[2,1-f]isoquinoline, α-naphthaphenanthridine, phenanthrooxazole, quinolino[6,5-f]quinoline, and benzothiophanthrene. These may have an alkyl group, an aryl group, an aromatic heterocyclic group, or a diarylamino group as a substituent.

Regarding the fluorescence-emitting dopant material, only one kind thereof may be contained in the light-emitting layer, or two or more kinds thereof may be contained. A content of the fluorescence-emitting dopant material is preferably 0.1 % to 20 % and more preferably 1 % to 10 % with respect to the host material.

When a thermally activated delayed fluorescence-emitting dopant is used as the light-emitting dopant material, the thermally activated delayed fluorescence-emitting dopant is not particularly limited. Examples thereof include: metal complexes such as a tin complex and a copper complex; indolocarbazole derivatives described in WO2011/070963A; cyanobenzene derivatives and carbazole derivatives described in Nature 2012, 492, 234; and phenazine derivatives, oxadiazole derivatives, triazole derivatives, sulfone derivatives, phenoxazine derivatives, and acridine derivatives described in Nature Photonics 2014, 8,326.

The thermally activated delayed fluorescence-emitting dopant material is not particularly limited, and specific examples thereof include the following.

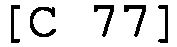

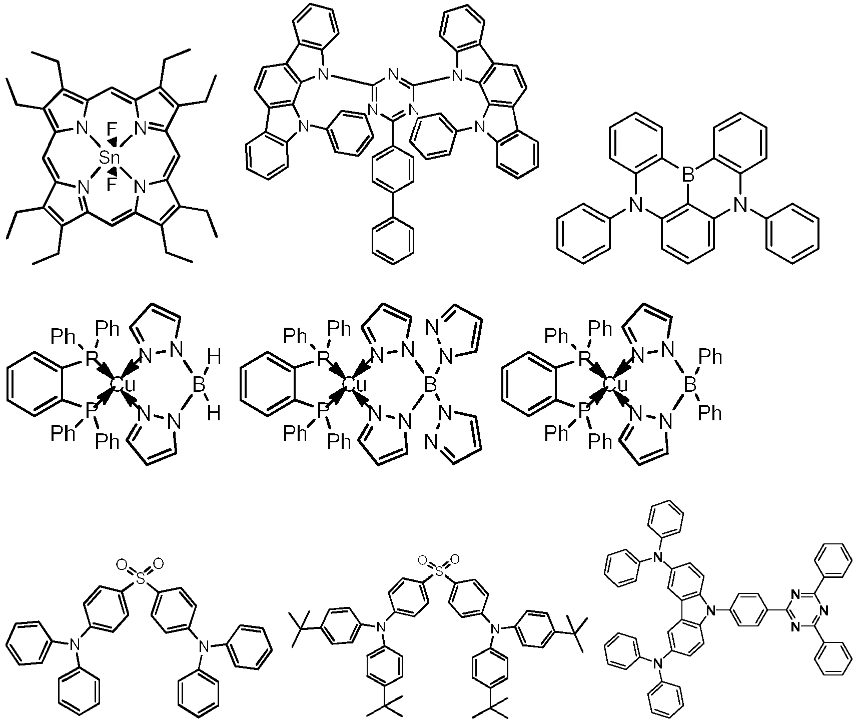

Regarding the thermally activated delayed fluorescence-emitting dopant material, only one kind thereof may be contained in the light-emitting layer, or two or more kinds thereof may be contained. In addition, the thermally activated delayed fluorescence-emitting dopant may be used by mixing with a phosphorescent dopant and a fluorescence-emitting dopant. A content of the thermally activated delayed fluorescence-emitting dopant material is preferably 0.10% to 50% and more preferably 1.0% to 30% with respect to the host material.

### -- Injection Layer --

The injection layer is a layer that is provided between an electrode and an organic layer in order to lower a driving voltage and improve emission luminance, and includes a hole injection layer and an electron injection layer, and may be present between the anode and the light-emitting layer or the hole transport layer, and between the cathode and the light-emitting layer or the electron transport layer. The injection layer can be provided as necessary.

### -- Hole Blocking Layer --

The hole blocking layer has a function of the electron transport layer in a broad sense, and is made of a hole blocking material having a function of transporting electrons and a significantly low ability to transport holes, and can block holes while transporting electrons, thereby improving a probability of recombining electrons and holes in the light-emitting layer.

For the hole blocking layer, a known hole blocking layer material can be used.

### -- Electron Blocking Layer --

The electron blocking layer has a function of a hole transport layer in a broad sense and blocks electrons while transporting holes, thereby enabling a probability of recombining electrons and holes in the light-emitting layer to be improved.

Regarding the material of the electron blocking layer, a known electron blocking layer material can be used and a material of the hole transport layer to be described below can be used as necessary. A film thickness of the electron blocking layer is preferably 3 to 100 nm, and more preferably 5 to 30 nm.

### -- Exciton Blocking Layer --

The exciton blocking layer is a layer for preventing excitons generated by recombination of holes and electrons in the light-emitting layer from being diffused in a charge transport layer, and insertion of this layer allows excitons to be efficiently confined in the light-emitting layer, enabling the luminous efficiency of the device to be improved. The exciton blocking layer can be inserted, in a device having two or more light-emitting layers adjacent to each other, between two adjacent light-emitting layers.

Regarding the material of the exciton blocking layer, a known exciton blocking layer material can be used. Examples thereof include 1,3-dicarbazolyl benzene (mCP) and bis(2-methyl-8-quinolinolato)-4-phenylphenolato aluminum (III) (BAlq).

### -- Hole Transport Layer --

The hole transport layer is made of a hole transport material having a function of transporting holes, and the hole transport layer can be provided as a single layer or a plurality of layers.

The hole transport material has either hole injection, transport properties or electron barrier properties, and may be an organic material or an inorganic material. For the hole transport layer, any one selected from conventionally known compounds can be used. Examples of such a hole transport material include porphyrin derivatives, arylamine derivatives, triazole derivatives, oxadiazole derivatives, imidazole derivatives, polyarylalkane derivatives, pyrazoline derivatives and pyrazolone derivatives, phenylenediamine derivatives, arylamine derivatives, amino-substituted chalcone derivatives, oxazole derivatives, styryl anthracene derivatives, fluorenone derivatives, hydrazone derivatives, stilbene derivatives, silazane derivatives, an aniline copolymer, and a conductive polymer oligomer, and particularly a thiophene oligomer. Use of porphyrin derivatives, arylamine derivatives, or styrylamine derivatives preferred. Use of arylamine compounds is more preferred.

### -- Electron Transport Layer --

The electron transport layer is made of a material having a function of transporting electrons, and the electron transport layer can be provided as a single layer or a plurality of layers.

The electron transport material (which may also serve as a hole blocking material) may have a function of transferring electrons injected from the cathode to the light-emitting layer. For the electron transport layer, any one selected from conventionally known compounds can be used, and examples thereof include polycyclic aromatic derivatives such as naphthalene, anthracene, and phenanthroline, tris(8-quinolinolato)aluminum(III) derivatives, phosphine oxide derivatives, nitro-substituted fluorene derivatives, diphenylquinone derivatives, thiopyrandioxide derivatives, carbodiimide, (fluorenylidene)methane derivatives, anthraquinodimethane and anthrone derivatives, bipyridine derivatives, quinoline derivatives, oxadiazole derivatives, benzimidazole derivatives, benzothiazole derivatives, and indolocarbazole derivatives. In addition, a polymer material in which the above material is introduced into a polymer chain or the above material is used for a main chain of a polymer can be used.

### Examples

Hereafter, the present invention will be described in detail by referring to Examples, but the present invention is not limited these Examples and can be implemented in various forms without departing from the gist thereof.

"%" expressing the compounding proportion and the concentration means "wt%".

### Example 1

The LUMO energy and the T1 energy of each compound used for a first host material, a second host material and a third host material were calculated by performing structure optimization calculation at the B3LYP/6-31G* level with molecular orbital method program Gaussian 03 according to the density functional theory (DFT). Results are shown in Table 1.

The LUMO energy and the T1 energy of each compound used in Comparative Examples are shown in Table 1.

**[Table 1]**

| Compound | | LUMO (eV) | T1 (eV) |
|---|---|---|---|
| 1-326 | | -2.01 | 2.68 |
| 1-327 | | -1.98 | 2.70 |
| 1-217 | | -2.00 | 2.66 |
| 1-6 | | -1.98 | 2.75 |
| 3-21 | | -0.94 | 2.97 |
| 3-33 | | -0.96 | 2.96 |
| 4-69 | | -1.32 | 3.16 |
| 2-3 | | -0.98 | 3.01 |
| 2-51 | | -1.26 | 2.65 |
| 2-2 | | -0.98 | 3.01 |
| 2-43 | | -0.99 | 3.00 |
| 5-2 | | -1.93 | 2.73 |
| 5-3 | | -1.88 | 2.81 |
| 5-38 | | -1.92 | 2.73 |
| 5-151 | | -1.93 | 2.73 |
| 5-152 | | -1.93 | 2.73 |
| A | | -2.02 | 2.38 |
| B | | -1.20 | 3.01 |
| C | | -2.15 | 2.49 |

### Example 2

On a glass substrate on which an anode made of ITO with a film thickness of 110 nm was formed, respective thin films were stacked by a vacuum evaporation method at a degree of vacuum of 4.0×10⁻⁵ Pa. First, HAT-CN was formed with a thickness of 25 nm as a hole injection layer on ITO, and next, NPD was formed with a thickness of 30 nm as a hole transport layer. Next, HT-1 was formed with a thickness of 10 nm as an electron blocking layer. Then, compound 1-326 as a first host material, compound 3-21 as a second host material, compound 5-2 as a third host material and Ir(ppy)₃ as a light-emitting dopant were co-vapor deposited from different vapor deposition sources, respectively, to form a light-emitting layer with a thickness of 40 nm. In this case, co-vapor deposition was performed under vapor deposition conditions such that the concentration of Ir(ppy)₃ was 10% and the concentration of the host material was 90% (the ratio among the first host material, the second host material and the third host material was 5%:70%:25%). Next, ET-1 was formed with a thickness of 20 nm as an electron transport layer. Further, LiF was formed with a thickness of 1 nm as an electron injection layer on the electron transport layer. Finally, Al was formed with a thickness of 70 nm as a cathode on the electron injection layer to produce an organic electroluminescent device.

### Examples 3 to 17

Organic electroluminescent devices were produced in the same manner as in Example 2 except that the first host material, the second host material and the third host material, and the proportions of the first host material, the second host material and the third host material were as shown in Table 2.

The luminance, driving voltage, luminous efficiency and lifetime characteristics of each of the organic electroluminescent devices produced in Examples 2 to 17 are shown in Table 2. In the table, the luminance, driving voltage, and luminous efficiency are values obtained at a driving current of 20 mA/cm², and exhibit initial characteristics. LT70 is a time period taken for a reduction in luminance to 70% of an initial luminance of 9000 cd/m², and represents lifetime characteristics. The same also applies to the following table.

The numbers in brackets in each column of the first host, the second host, and the third host are compounding proportions.

**[Table 2]**

| Example | First host | Second host | Third host | Luminance (cd/m²) | Voltage (V) | Power efficiency (lm/W) | LT70 (h) |
|---|---|---|---|---|---|---|---|
| 2 | 1-326 (5%) | 3-21 (70%) | 5-2 (25%) | 11,830 | 4.4 | 42.1 | 3,270 |
| 3 | 1-326 (10%) | 3-21 (70%) | 5-2 (20%) | 11,520 | 4.3 | 41.9 | 3,300 |
| 4 | 1-326 (20%) | 3-21 (70%) | 5-2 (10%) | 11,510 | 4.1 | 43.9 | 3,250 |
| 5 | 1-326 (10%) | 3-21 (80%) | 5-2 (10%) | 12,330 | 4.3 | 40.1 | 3,460 |
| 6 | 1-326 (10%) | 3-33 (70%) | 5-3 (20%) | 12,140 | 4.4 | 43.2 | 3,220 |
| 7 | 1-327 (10%) | 4-69 (70%) | 5-2 (20%) | 11,830 | 4.6 | 40.2 | 3,560 |
| 8 | 1-327 (20%) | 4-69 (70%) | 5-2 (10%) | 11,840 | 4.4 | 42.1 | 3,500 |
| 9 | 1-217 (10%) | 2-3 (70%) | 5-3 (20%) | 12,330 | 4.4 | 43.9 | 3,720 |
| 10 | 1-217 (20%) | 2-3 (70%) | 5-3 (10%) | 12,350 | 4.2 | 46.0 | 3,700 |
| 11 | 1-327 (10%) | 2-51 (70%) | 5-3 (20%) | 12,160 | 4.4 | 43.3 | 3,040 |
| 12 | 1-6 (10%) | 2-3 (70%) | 5-38 (20%) | 11,970 | 4.2 | 44.6 | 4,040 |
| 13 | 1-6 (10%) | 2-3 (80%) | 5-38 (10%) | 11,830 | 4.4 | 42.1 | 4,070 |
| 14 | 1-6 (10%) | 2-3 (70%) | 5-151 (20%) | 11,330 | 4.6 | 38.5 | 3,520 |
| 15 | 1-327 (10%) | 2-2 (70%) | 5-38 (20%) | 11,470 | 4.4 | 40.8 | 4,020 |
| 16 | 1-6 (10%) | 2-43 (70%) | 5-152 (20%) | 11,220 | 4.4 | 39.3 | 3,380 |
| 17 | 1-6 (3%) | 2-43 (70%) | 5-152 (27%) | 11,340 | 4.6 | 38.6 | 3,520 |

### Example 18

The 50% weight reduction temperatures (T₅₀) of compounds used as the first host material, the second host material and the third host material were measured. Results are shown in Table 3.

**[Table 3]**

| Compound | T₅₀ [°C] |
|---|---|
| 1-6 | 312 |
| 2-3 | 315 |
| 5-151 | 312 |
| 2-2 | 279 |
| 5-38 | 278 |
| 2-43 | 316 |
| 5-152 | 311 |
| 1-327 | 273 |

### Example 19

Premixture H1 was prepared by weighing first host material 1-6 (0.10 g), second host material 2-3 (0.70 g) and third host material 5-151 (0.20 g) and mixing them with grinding in a mortar.

On a glass substrate on which an anode made of ITO with a film thickness of 110 nm was formed, respective thin films were stacked by a vacuum evaporation method at a degree of vacuum of 4.0×10⁻⁵ Pa. First, HAT-CN was formed with a thickness of 25 nm as a hole injection layer on ITO, and next, NPD was formed with a thickness of 30 nm as a hole transport layer. Next, HT-1 was formed with a thickness of 10 nm as an electron blocking layer. Then, premixture H1 and Ir(ppy)₃ as a light-emitting dopant were co-vapor deposited from different vapor deposition sources, respectively, to form a light-emitting layer with a thickness of 40 nm. In this case, co-vapor deposition was performed under vapor deposition conditions such that the concentration of Ir(ppy)₃ was 10% and the concentration of the host material was 90%. Next, ET-1 was formed with a thickness of 20 nm as an electron transport layer. Further, LiF was formed with a thickness of 1 nm as an electron injection layer on the electron transport layer. Finally, Al was formed with a thickness of 70 nm as a cathode on the electron injection layer to produce an organic electroluminescent device.

### Examples 20 to 23

Premixtures H2 to H5 were prepared in the same manner as in Example 19 except that the types and the compounding proportions of the first host material, the second host material and the third host material were as shown in Table 4.

Organic electroluminescent devices were produced in the same manner as in Example 19 except that premixtures H2 to H5 were used.

**[Table 4]**

| Premixture | First host | Second host | Third host |
|---|---|---|---|
| H1 | 1-6 (10%) | 2-3 (70%) | 5-151 (20%) |
| H2 | 1-6 (20%) | 2-3 (70%) | 5-151 (10%) |
| H3 | 1-327 (10%) | 2-2 (70%) | 5-38 (20%) |
| H4 | 1-327 (20%) | 2-2 (70%) | 5-38 (10%) |
| H5 | 1-6 (10%) | 2-43 (70%) | 5-152 (20%) |

Evaluation results of the organic electroluminescent devices in produced Examples 19 to 23 are shown in Table 5.

**[Table 5]**

| Example | Premixture | Luminance (cd/m²) | Voltage (V) | Power efficiency (lm/W) | LT70 (h) |
|---|---|---|---|---|---|
| 19 | H1 | 11,300 | 4.4 | 40.2 | 3,790 |
| 20 | H2 | 11,230 | 4.2 | 41.8 | 3,850 |
| 21 | H3 | 11,480 | 4.2 | 42.8 | 4,050 |
| 22 | H4 | 11,450 | 4.0 | 44.8 | 4,040 |
| 23 | H5 | 11,280 | 4.3 | 41.1 | 3,830 |

### Comparative Examples 1 to 5

Organic electroluminescent devices were produced in the same manner as in Example 2 except that the types and the compounding proportions of the first host material, the second host material and the third host material were as shown in Table 6.

Evaluation results of the produced organic electroluminescent devices are shown in Table 6.

**[Table 6]**

| Comparative Example | First host | Second host | Third host | Luminance (cd/m²) | Voltage (V) | Power efficiency (lm/W) | LT70 (h) |
|---|---|---|---|---|---|---|---|
| 1 | - | 3-33 (70%) | 5-3 (30%) | 11,070 | 4.9 | 35.4 | 3,210 |
| 2 | - | 2-43 (70%) | 5-152 (30%) | 11,150 | 4.9 | 35.6 | 3,460 |
| 3 | 1-6 (30%) | 2-3 (70%) | - | 10,900 | 4.0 | 42.6 | 2,450 |
| 4 | A (10%) | B (70%) | C (20%) | 530 | 4.3 | 1.9 | 2,150 |
| 5 | - | 2-3 (70%) | 5-2 (10%) | 11,200 | 5.0 | 35.1 | 3,580 |
| | | | 5-151 (20%) | | | | |

### Comparative Examples 7 to 8

Premixture H6 to H7 were prepared in the same manner as in Example 19 except that the types and the compounding proportions of the host materials were as shown in Table 7.

**[Table 7]**

| Premixture | Second host | Third host |
|---|---|---|
| H6 | 2-3 (70%) | 5-151 (30%) |
| H7 | 2-3 (70%) | 5-152 (10%) |
| | | 5-151 (20%) |

Organic electroluminescent devices were produced in the same manner as in Example 19 except that premixtures H6 to H7 were used.

Evaluation results of the produced organic electroluminescent devices are shown in Table 8.

**[Table 8]**

| Compara tive Example | Premixture | Luminance (cd/m²) | Voltage (V) | Power efficiency (lm/W) | LT70 (h) |
|---|---|---|---|---|---|
| 8 | H6 | 11,230 | 4.9 | 35.9 | 3,600 |
| 9 | H7 | 11,250 | 4.9 | 35.9 | 3,550 |

It can be seen from the foregoing that Examples, while exhibit lifetime characteristics comparable with or more than those of Comparative Examples, exhibit improved driving voltage and power efficiency. In other words, the present invention realizes an organic electroluminescent device having simultaneously respective characteristics of lifetime characteristics, driving voltage, and power efficiency at favorable levels.

Compounds used in Examples and Comparative Examples are shown below.

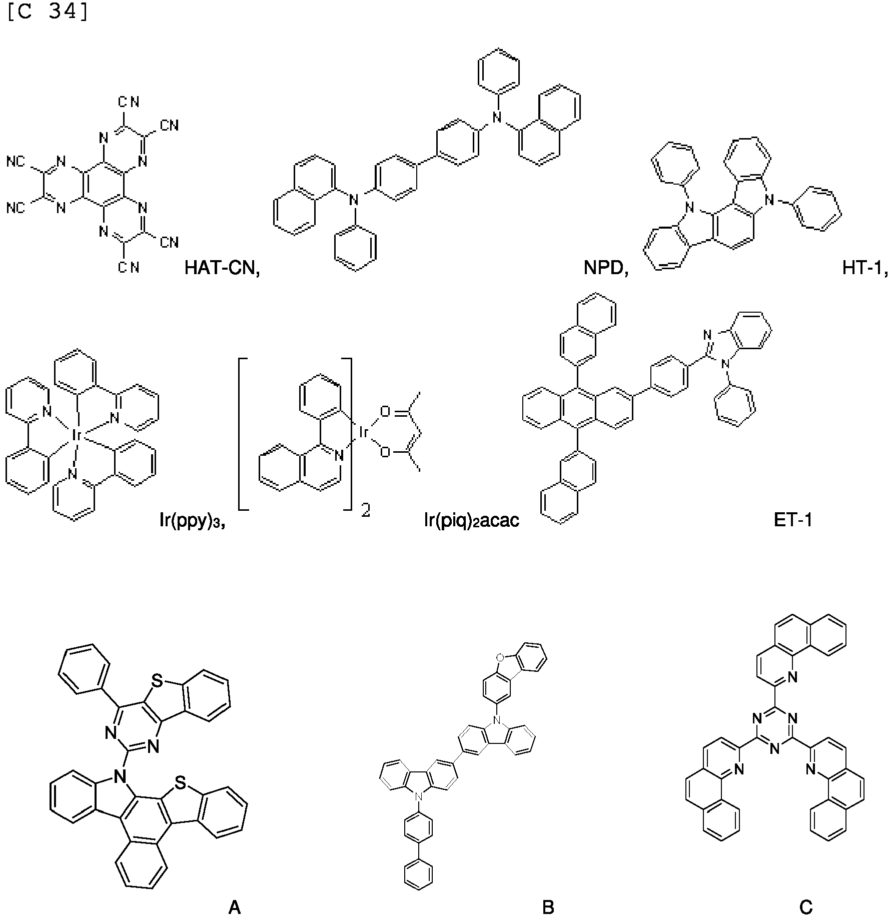

## Claims

1. An organic electroluminescent device comprising one or more light-emitting layers (5) between an anode (2) and a cathode (7) opposed to each other, wherein at least one of the light-emitting layers (5) contains a host material comprising a first host material, a second host material and a third host material, and a light-emitting dopant material, the LUMO energy of the first host material is -1.95 eV or less, the LUMO energy of the second host material is -1.54 eV or more, and LM2 ≥ LM3 ≥ LM1 is satisfied under the assumption that the LUMO energies of the first host material, the second host material and the third host material are LM1, LM2 and LM3, respectively, wherein the LUMO energy of the first host material, the second host material, and the third host material is calculated as set out in the examples of the description, **characterised in that** the LUMO energy of the third host material is -1.94 to -1.77 eV.

2. The organic electroluminescent device according to claim 1, wherein the triplet excitation (T1) energies of the first host material, the second host material and the third host material are each 2.55 eV or more.

3. The organic electroluminescent device according to claim 1 or 2, wherein the light-emitting dopant material is a material emitting fluorescence including a thermally activated delayed fluorescence material.

4. The organic electroluminescent device according to any one of claims 1 to 3, wherein the light-emitting dopant material is a phosphorescence-emitting material.

5. The organic electroluminescent device according to any one of claims 1 to 4, wherein the first host material is a compound represented by the following general formula (1): 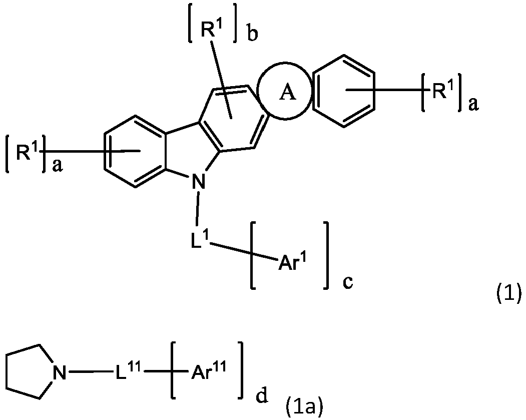
wherein a ring A is a heterocycle represented by formula (1a) and the ring A is fused to an adjacent ring at any position,
R¹ independently represents deuterium, an aliphatic hydrocarbon group having 1 to 10 carbon atoms, a substituted or unsubstituted aromatic hydrocarbon group having 6 to 30 carbon atoms, a substituted or unsubstituted aromatic heterocyclic group having 3 to 17 carbon atoms, or a substituted or unsubstituted linked aromatic group in which two to five of these aromatic rings are linked to each other,
a, c and d each independently represent an integer of 0 to 4, and b represents an integer of 0 to 2,
L¹ and L¹¹ independently represent a substituted or unsubstituted aromatic hydrocarbon group having 6 to 30 carbon atoms, or a substituted or unsubstituted aromatic heterocyclic group having 3 to 17 carbon atoms, and at least one thereof represents the aromatic heterocyclic group, and
Ar¹ and Ar¹¹ each independently represent a substituted or unsubstituted aromatic hydrocarbon group having 6 to 30 carbon atoms, a substituted or unsubstituted aromatic heterocyclic group having 3 to 17 carbon atoms, or a substituted or unsubstituted linked aromatic group in which two to seven of these aromatic rings are linked to each other.

6. The organic electroluminescent device according to claim 5, wherein at least one of L¹ and L¹¹ is a substituted or unsubstituted nitrogen-containing 6-membered ring group, or a substituted or unsubstituted ring-fused aromatic heterocyclic group containing a nitrogen-containing 6-membered ring.

7. The organic electroluminescent device according to claim 5, wherein the general formula (1) is the following formula (11): 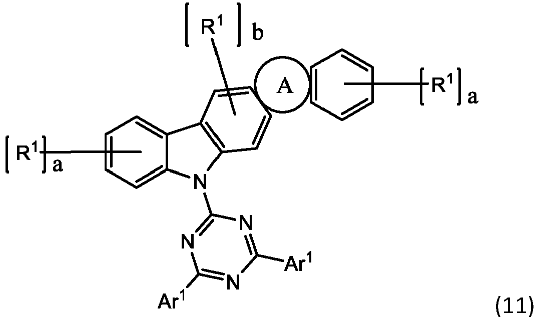 wherein R¹, ring A, Ar¹, a, and b are as defined for the general formula (1).

8. The organic electroluminescent device according to claim 7, wherein the formula (11) is the following formula (12) or formula (13): 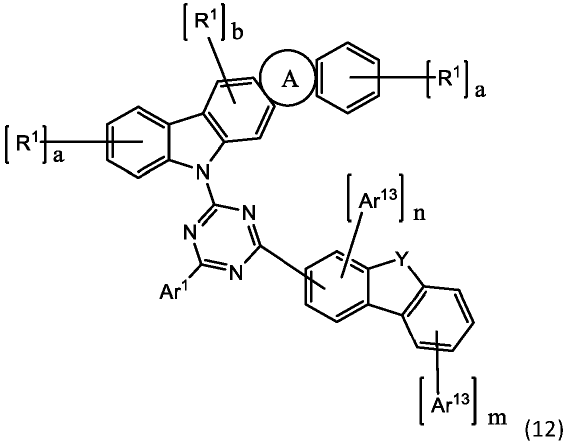 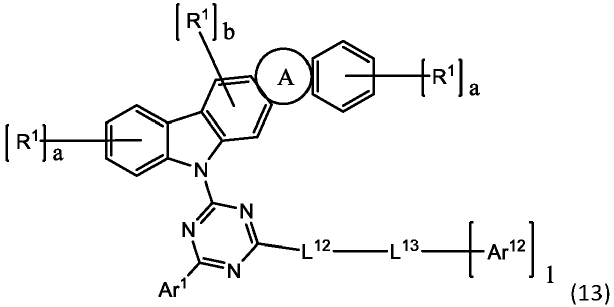
wherein R¹, ring A, Ar¹, a and b are as defined for the general formula (1),
Y represents O, S, NAr¹⁴, or CAr¹⁵Ar¹⁶,
Ar¹³, Ar¹⁴, Ar¹⁵, and Ar¹⁶ each independently represent an aliphatic hydrocarbon group having 1 to 10 carbon atoms, a substituted or unsubstituted aromatic hydrocarbon group having 6 to 30 carbon atoms, a substituted or unsubstituted aromatic heterocyclic group having 3 to 17 carbon atoms, or a substituted or unsubstituted linked aromatic group in which two to five of these aromatic rings are linked to each other,
m represents an integer of 0 to 4, n represents an integer of 0 to 3, and I represents an integer of 0 to 4,
L¹² represents a substituted or unsubstituted aromatic hydrocarbon group having 6 to 30 carbon atoms,
L¹³ represents a nitrogen-containing 6-membered ring group, and
Ar¹² independently represents a substituted or unsubstituted aromatic hydrocarbon group having 6 to 30 carbon atoms, a substituted or unsubstituted aromatic heterocyclic group having 3 to 17 carbon atoms, or a substituted or unsubstituted linked aromatic group in which two to five of these aromatic rings are linked to each other.

9. The organic electroluminescent device according to any one of claims 1 to 8, wherein the second host material is a compound represented by the following general formula (2), general formula (3), or general formula (4): 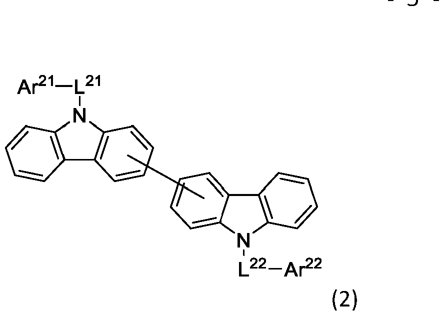
wherein Ar²¹ and Ar²² independently represent hydrogen, deuterium, a substituted or unsubstituted aromatic hydrocarbon group having 6 to 14 carbon atoms, a substituted or unsubstituted aromatic heterocyclic group having 3 to 17 carbon atoms, or a substituted or unsubstituted linked aromatic group in which two of the aromatic rings are linked to each other; and L²¹ and L²² independently represent a direct bond or a phenylene group; 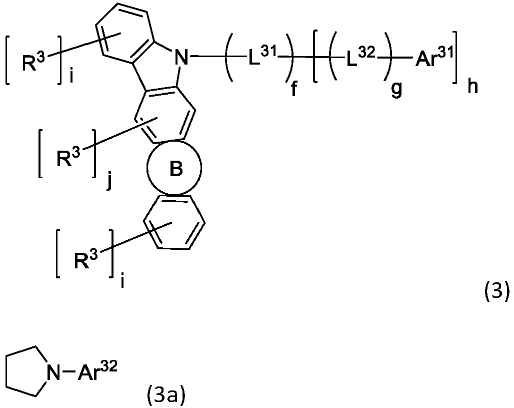
wherein a ring B is a heterocycle represented by formula (3a) and the ring B is fused to an adjacent ring at any position,
R³ independently represents deuterium, an aliphatic hydrocarbon group having 1 to 10 carbon atoms, a substituted or unsubstituted aromatic hydrocarbon group having 6 to 30 carbon atoms, a substituted or unsubstituted aromatic heterocyclic group having 3 to 17 carbon atoms, or a substituted or unsubstituted linked aromatic group in which two to five of these aromatic rings are linked to each other,
L³¹ independently represents a substituted or unsubstituted aromatic hydrocarbon group having 6 to 30 carbon atoms, or a substituted or unsubstituted aromatic heterocyclic group having 3 to 17 carbon atoms,
L³² independently represents a substituted or unsubstituted aromatic hydrocarbon group having 6 to 10 carbon atoms,
Ar³¹ represents a substituted or unsubstituted aromatic hydrocarbon group having 6 to 10 carbon atoms, a substituted or unsubstituted carbazolyl group, or a substituted or unsubstituted linked aromatic group in which two to five of these aromatic rings are linked to each other,
Ar³² represents a substituted or unsubstituted aromatic hydrocarbon group having 6 to 30 carbon atoms, a substituted or unsubstituted aromatic heterocyclic group having 3 to 17 carbon atoms, or a substituted or unsubstituted linked aromatic group in which two to five of these aromatic rings are linked to each other,
i represents an integer of 0 to 4, and j represents an integer of 0 to 2,
f is the number of repetitions, and represents an integer of 1 to 3, g is the number of repetitions and independently represents an integer of 0 to 3, and h represents the number of substitutions and represents an integer of 0 to 7; 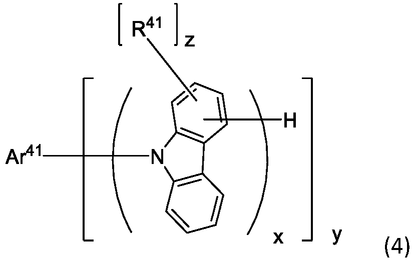
wherein Ar⁴¹ represents a substituted or unsubstituted aromatic hydrocarbon group having 6 to 30 carbon atoms, a substituted or unsubstituted aromatic heterocyclic group having 3 to 17 carbon atoms, or a linked aromatic group in which two to five of these aromatic rings are linked; when Ar⁴¹ has a hydrogen atom, the hydrogen atom may be replaced by deuterium,
R⁴¹ each independently represents deuterium, an aliphatic hydrocarbon group having 1 to 10 carbon atoms, a substituted or unsubstituted aromatic hydrocarbon group having 6 to 30 carbon atoms, or a substituted or unsubstituted aromatic heterocyclic group having 3 to 17 carbon atoms, provided that R⁴¹ is not a carbazolyl group,
x is the number of repetitions and independently represents an integer of 1 to 4, and at least one x is an integer of 2 to 4; y is the number of substitutions and independently represents an integer of 1 to 4; and when x and y is 2 or more, a plurality of carbazolyl groups may be the same or different; and z is an integer of 0 to 3.

10. The organic electroluminescent device according to any one of claims 1 to 9, wherein the third host material is a compound represented by general formula (5): 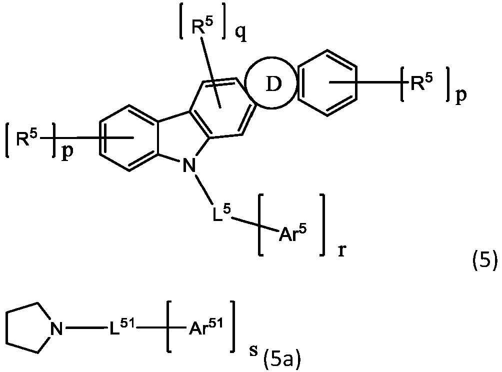
wherein a ring D is a heterocycle represented by formula (5a) and the ring D is fused to an adjacent ring at any position,
R⁵ independently represents deuterium, an aliphatic hydrocarbon group having 1 to 10 carbon atoms, a substituted or unsubstituted aromatic hydrocarbon group having 6 to 30 carbon atoms, a substituted or unsubstituted aromatic heterocyclic group having 3 to 17 carbon atoms, or a substituted or unsubstituted linked aromatic group in which two to five of these aromatic rings are linked to each other,
p, r and s each independently represent an integer of 0 to 4, and q represents an integer of 0 to 2,
L⁵ and L⁵¹ independently represent a substituted or unsubstituted aromatic hydrocarbon group having 6 to 30 carbon atoms, or a substituted or unsubstituted aromatic heterocyclic group having 3 to 17 carbon atoms, and at least one thereof represents the aromatic heterocyclic group, and
Ar⁵ and Ar⁵¹ each independently represent a substituted or unsubstituted aromatic hydrocarbon group having 6 to 30 carbon atoms, a substituted or unsubstituted aromatic heterocyclic group having 3 to 17 carbon atoms, or a substituted or unsubstituted linked aromatic group in which two to seven of these aromatic rings are linked to each other.

11. The organic electroluminescent device according to claim 10, wherein at least one of L⁵ and L⁵¹ represents a substituted or unsubstituted nitrogen-containing 6-membered ring or ring-fused aromatic heterocyclic group containing a nitrogen-containing 6-membered ring.

12. The organic electroluminescent device according to claim 10, wherein the third host material is a compound represented by the following formula (51): 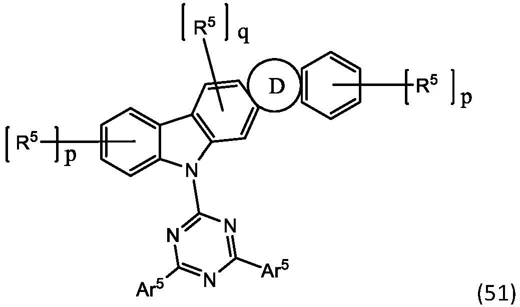 wherein R⁵, D, Ar⁵, p, and q are as defined for the general formula (5).

13. The organic electroluminescent device according to any one of claims 1 to 12, wherein a proportion of the first host material is larger than 1.0 wt% and less than 30 wt% and a proportion of the third host material is larger than 5.0 wt% and less than 80 wt% based on the first host material, the second host material and the third host material in total.

14. A method for producing an organic electroluminescent device comprising one or more light-emitting layers (5) between an anode (2) and a cathode (7) opposed to each other, at least one of the light-emitting layers (5) comprising a host material comprising a first host material, a second host material and a third host material, and a light-emitting dopant material, the method comprising forming each of the light-emitting layers (5) by vapor depositing the light-emitting dopant material and a host material composition in which the LUMO energy of the first host material is -1.95 eV or less, the LUMO energy of the second host material is -1.54 eV or more, and LM2 ≥ LM3 ≥ LM1 is satisfied under the assumption that the LUMO energies of the first host material, the second host material and the third host material are LM1, LM2 and LM3, respectively, wherein the LUMO energy of the first host material, the second host material, and the third host material is calculated according to the examples of the description, **characterised in that** the LUMO energy of the third host material is -1.94 to -1.77 eV.

15. A method for producing the organic electroluminescent device according to claim 14, which is an organic electroluminescent device comprising one or more light-emitting layers (5) between an anode (2) and a cathode (7) opposed to each other, wherein at least one of the light-emitting layers (5) contains a first host material, a second host material and a third host material, and a light-emitting dopant material, wherein at least three materials contained in the light-emitting layers (5) are vapor deposited from one vapor deposition source.

16. The method according to claim 15, wherein the first host material, the second host material and the third host material are vapor deposited from one vapor deposition source.

17. Use of a host material composition in the method for producing an organic electroluminescent device according to any one of claims 14 to 16, wherein the host material composition comprises a first host material, a second host material and a third host material, and a light-emitting dopant material, the LUMO energy of the first host material is -1.95 eV or less, the LUMO energy of the second host material is -1.54 eV or more, and LM2 ≥ LM3 ≥ LM1 is satisfied under the assumption that the LUMO energies of the first host material, the second host material and the third host material are LM1, LM2 and LM3, respectively, wherein the LUMO energy of the first host material, the second host material, and the third host material is calculated as set out in the examples of the description, **characterised in that** the LUMO energy of the third host material is -1.94 to -1.77 eV.

18. Use of a host material composition comprising a first host material, a second host material and a third host material, and a light-emitting dopant material, the LUMO energy of the first host material is -1.95 eV or less, the LUMO energy of the second host material is -1.54 eV or more, and LM2 ≥ LM3 ≥ LM1 is satisfied under the assumption that the LUMO energies of the first host material, the second host material and the third host material are LM1, LM2 and LM3, respectively, wherein the LUMO energy of the first host material, the second host material, and the third host material is calculated as set out in the examples of the description, in an organic electroluminescent device comprising one or more light-emitting layers (5) between an anode (2) and a cathode (7) opposed to each other, wherein at least one of the light-emitting layers (5) contains the host material, **characterised in that** the LUMO energy of the third host material is -1.94 to -1.77 eV.

## Patentansprüche

1. Eine organische Elektrolumineszenzvorrichtung, umfassend eine oder mehrere lichtemittierende Schichten (5) zwischen einer Anode (2) und einer Kathode (7), die einander gegenüberliegen, wobei mindestens eine der lichtemittierenden Schichten (5) ein Wirtsmaterial enthält, das ein erstes Wirtsmaterial, ein zweites Wirtsmaterial und ein drittes Wirtsmaterial sowie ein lichtemittierendes Dotierungsmaterial umfasst, wobei die LUMO-Energie des ersten Wirtsmaterials -1,95 eV oder weniger beträgt, die LUMO-Energie des zweiten Wirtsmaterials -1,54 eV oder mehr beträgt und LM2 ≥ LM3 ≥ LM1 erfüllt ist, unter der Annahme, dass die LUMO-Energien des ersten Wirtsmaterials, des zweiten Wirtsmaterials und des dritten Wirtsmaterials LM1, LM2 bzw. LM3 sind, wobei die LUMO-Energie des ersten Wirtsmaterials, des zweiten Wirtsmaterials und des dritten Wirtsmaterials wie in den Beispielen der Beschreibung dargelegt berechnet wird, **dadurch gekennzeichnet, dass** die LUMO-Energie des dritten Wirtsmaterials -1,94 bis -1,77 eV beträgt.

2. Die organische Elektrolumineszenzvorrichtung gemäß Anspruch 1, wobei die Triplett-Anregungsenergien (T1) des ersten Wirtsmaterials, des zweiten Wirtsmaterials und des dritten Wirtsmaterials jeweils 2,55 eV oder mehr betragen.

3. Die organische Elektrolumineszenzvorrichtung gemäß Anspruch 1 oder 2, wobei das lichtemittierende Dotierungsmaterial ein Material ist, das Fluoreszenz emittiert, einschließlich ein thermisch aktiviertes Material mit verzögerter Fluoreszenz.

4. Die organische Elektrolumineszenzvorrichtung gemäß einem der Ansprüche 1 bis 3, wobei das lichtemittierende Dotierungsmaterial ein phosphoreszenzemittierendes Material ist.

5. Die organische Elektrolumineszenzvorrichtung gemäß einem der Ansprüche 1 bis 4, wobei das erste Wirtsmaterial eine Verbindung ist, die durch die folgende allgemeine Formel (1) dargestellt ist: 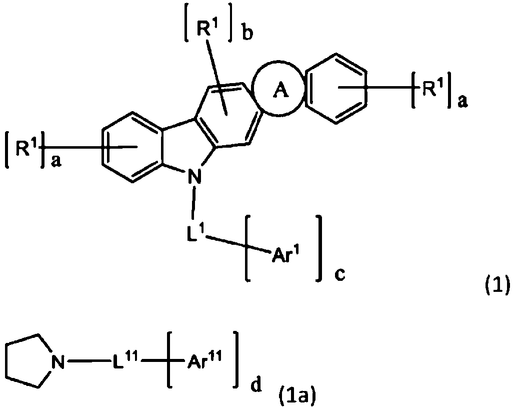
wobei ein Ring A ein durch Formel (1a) dargestellter Heterocyclus ist und der Ring A an einer beliebigen Position an einen benachbarten Ring anelliert ist,
R¹ unabhängig Deuterium, eine aliphatische Kohlenwasserstoffgruppe mit 1 bis 10 Kohlenstoffatomen, eine substituierte oder unsubstituierte aromatische Kohlenwasserstoffgruppe mit 6 bis 30 Kohlenstoffatomen, eine substituierte oder unsubstituierte aromatische heterocyclische Gruppe mit 3 bis 17 Kohlenstoffatomen oder eine substituierte oder unsubstituierte verknüpfte aromatische Gruppe, in der zwei bis fünf dieser aromatischen Ringe miteinander verknüpft sind,
a, c und d jeweils unabhängig voneinander eine ganze Zahl von 0 bis 4 darstellen und b eine ganze Zahl von 0 bis 2 darstellt,
L¹ und L¹¹ unabhängig eine substituierte oder unsubstituierte aromatische Kohlenwasserstoffgruppe mit 6 bis 30 Kohlenstoffatomen oder eine substituierte oder unsubstituierte aromatische heterocyclische Gruppe mit 3 bis 17 Kohlenstoffatomen darstellen und mindestens eine davon die aromatische heterocyclische Gruppe darstellt, und
Ar¹ und Ar¹¹ jeweils unabhängig eine substituierte oder unsubstituierte aromatische Kohlenwasserstoffgruppe mit 6 bis 30 Kohlenstoffatomen, eine substituierte oder unsubstituierte aromatische heterocyclische Gruppe mit 3 bis 17 Kohlenstoffatomen oder eine substituierte oder unsubstituierte verknüpfte aromatische Gruppe, in der zwei bis sieben dieser aromatischen Ringe miteinander verknüpft sind, darstellen.

6. Die organische Elektrolumineszenzvorrichtung gemäß Anspruch 5, wobei mindestens eines von L¹ und L¹¹ eine substituierte oder unsubstituierte stickstoffhaltige 6-gliedrige Ringgruppe oder eine substituierte oder unsubstituierte anellierte aromatische heterocyclische Gruppe ist, die einen stickstoffhaltigen 6-gliedrigen Ring enthält.

7. Die organische Elektrolumineszenzvorrichtung gemäß Anspruch 5, wobei die allgemeine Formel (1) die folgende Formel (11) ist: 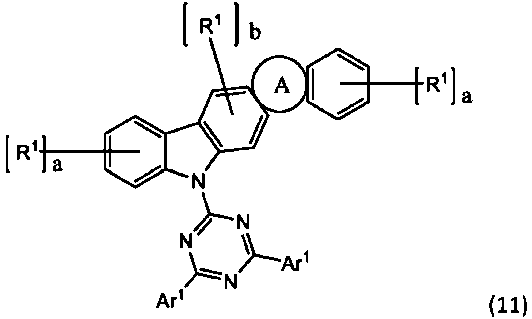 wobei R¹, Ring A, Ar¹, a und b wie für die allgemeine Formel (1) definiert sind.

8. Die organische Elektrolumineszenzvorrichtung gemäß Anspruch 7, wobei die Formel (11) die folgende Formel (12) oder Formel (13) ist: 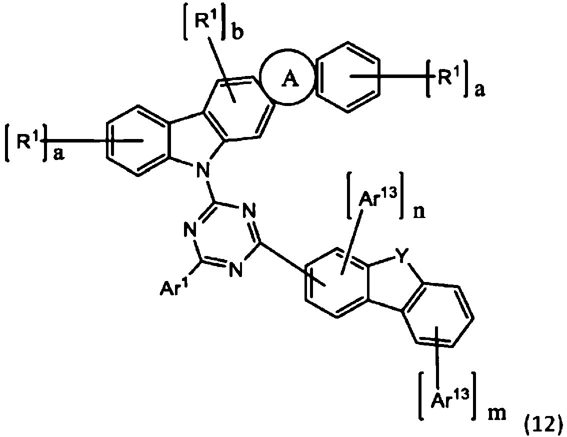 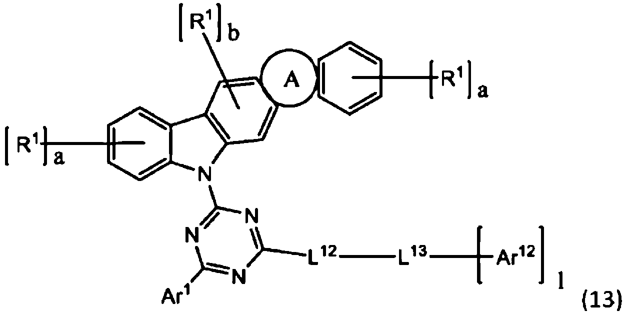
wobei R¹, Ring A, Ar¹, a und b wie für die allgemeine Formel (1) definiert sind,
Y für O, S, NAr¹⁴ oder CAr¹⁵Ar¹⁶ steht,
Ar¹³, Ar¹⁴, Ar¹⁵ und Ar¹⁶ jeweils unabhängig eine aliphatische Kohlenwasserstoffgruppe mit 1 bis 10 Kohlenstoffatomen, eine substituierte oder unsubstituierte aromatische Kohlenwasserstoffgruppe mit 6 bis 30 Kohlenstoffatomen, eine substituierte oder unsubstituierte aromatische heterocyclische Gruppe mit 3 bis 17 Kohlenstoffatomen oder eine substituierte oder unsubstituierte verknüpfte aromatische Gruppe, in der zwei bis fünf dieser aromatischen Ringe miteinander verknüpft sind, darstellen,
m eine ganze Zahl von 0 bis 4 darstellt, n eine ganze Zahl von 0 bis 3 darstellt und I eine ganze Zahl von 0 bis 4 darstellt,
L¹² eine substituierte oder unsubstituierte aromatische Kohlenwasserstoffgruppe mit 6 bis 30 Kohlenstoffatomen darstellt,
L¹³ eine stickstoffhaltige 6-gliedrige Ringgruppe darstellt und
Ar¹² unabhängig eine substituierte oder unsubstituierte aromatische Kohlenwasserstoffgruppe mit 6 bis 30 Kohlenstoffatomen, eine substituierte oder unsubstituierte aromatische heterocyclische Gruppe mit 3 bis 17 Kohlenstoffatomen oder eine substituierte oder unsubstituierte verknüpfte aromatische Gruppe, in der zwei bis fünf dieser aromatischen Ringe miteinander verknüpft sind, darstellt.

9. Die organische Elektrolumineszenzvorrichtung gemäß einem der Ansprüche 1 bis 8, wobei das zweite Wirtsmaterial eine Verbindung ist, die durch die folgende allgemeine Formel (2), allgemeine Formel (3) oder allgemeine Formel (4) dargestellt ist: 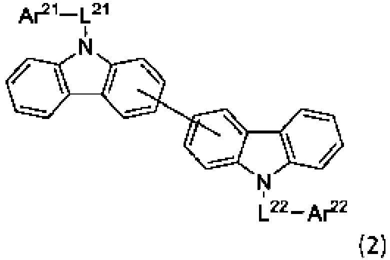
wobei Ar²¹ und Ar²² unabhängig Wasserstoff, Deuterium, eine substituierte oder unsubstituierte aromatische Kohlenwasserstoffgruppe mit 6 bis 14 Kohlenstoffatomen, eine substituierte oder unsubstituierte aromatische heterocyclische Gruppe mit 3 bis 17 Kohlenstoffatomen oder eine substituierte oder unsubstituierte verknüpfte aromatische Gruppe, in der zwei der aromatischen Ringe miteinander verknüpft sind, darstellen; und L²¹ und L²² unabhängig eine direkte Bindung oder eine Phenylengruppe darstellen; 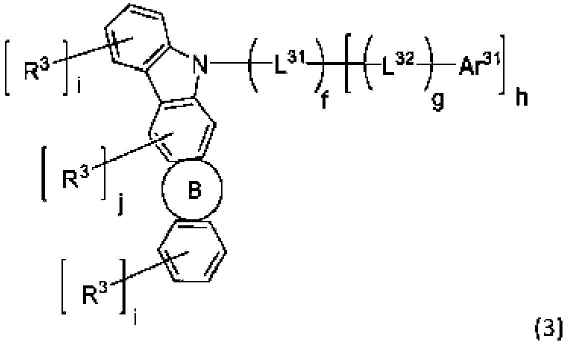 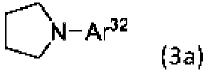
wobei ein Ring B ein durch Formel (3a) dargestellter Heterocyclus ist und der Ring B an einer beliebigen Position an einen benachbarten Ring anelliert ist,
R³ unabhängig Deuterium, eine aliphatische Kohlenwasserstoffgruppe mit 1 bis 10 Kohlenstoffatomen, eine substituierte oder unsubstituierte aromatische Kohlenwasserstoffgruppe mit 6 bis 30 Kohlenstoffatomen, eine substituierte oder unsubstituierte aromatische heterocyclische Gruppe mit 3 bis 17 Kohlenstoffatomen oder eine substituierte oder unsubstituierte verknüpfte aromatische Gruppe, in der zwei bis fünf dieser aromatischen Ringe miteinander verknüpft sind, darstellt,
L³¹ unabhängig eine substituierte oder unsubstituierte aromatische Kohlenwasserstoffgruppe mit 6 bis 30 Kohlenstoffatomen oder eine substituierte oder unsubstituierte aromatische heterocyclische Gruppe mit 3 bis 17 Kohlenstoffatomen darstellt,
L³² unabhängig eine substituierte oder unsubstituierte aromatische Kohlenwasserstoffgruppe mit 6 bis 10 Kohlenstoffatomen darstellt,
Ar³¹ eine substituierte oder unsubstituierte aromatische Kohlenwasserstoffgruppe mit 6 bis 10 Kohlenstoffatomen, eine substituierte oder unsubstituierte Carbazolylgruppe oder eine substituierte oder unsubstituierte verknüpfte aromatische Gruppe, in der zwei bis fünf dieser aromatischen Ringe miteinander verknüpft sind, darstellt,
Ar³² eine substituierte oder unsubstituierte aromatische Kohlenwasserstoffgruppe mit 6 bis 30 Kohlenstoffatomen, eine substituierte oder unsubstituierte aromatische heterocyclische Gruppe mit 3 bis 17 Kohlenstoffatomen oder eine substituierte oder unsubstituierte verknüpfte aromatische Gruppe, in der zwei bis fünf dieser aromatischen Ringe miteinander verknüpft sind, darstellt,
i eine ganze Zahl von 0 bis 4 darstellt und j eine ganze Zahl von 0 bis 2 darstellt,
f die Anzahl an Wiederholungen ist und eine ganze Zahl von 1 bis 3 darstellt, g die Anzahl an Wiederholungen ist und unabhängig eine ganze Zahl von 0 bis 3 darstellt und h die Anzahl an Substitutionen darstellt und eine ganze Zahl von 0 bis 7 darstellt; 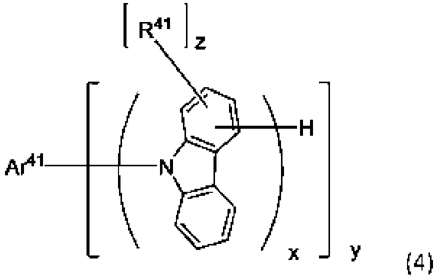
wobei Ar⁴¹ eine substituierte oder unsubstituierte aromatische Kohlenwasserstoffgruppe mit 6 bis 30 Kohlenstoffatomen, eine substituierte oder unsubstituierte aromatische heterocyclische Gruppe mit 3 bis 17 Kohlenstoffatomen oder eine verknüpfte aromatische Gruppe, in der zwei bis fünf dieser aromatischen Ringe verknüpft sind, darstellt; wenn Ar⁴¹ ein Wasserstoffatom aufweist, kann das Wasserstoffatom durch Deuterium ersetzt sein,
R⁴¹ jeweils unabhängig Deuterium, eine aliphatische Kohlenwasserstoffgruppe mit 1 bis 10 Kohlenstoffatomen, eine substituierte oder unsubstituierte aromatische Kohlenwasserstoffgruppe mit 6 bis 30 Kohlenstoffatomen oder eine substituierte oder unsubstituierte aromatische heterocyclische Gruppe mit 3 bis 17 Kohlenstoffatomen darstellt, mit der Maßgabe, dass R⁴¹ keine Carbazolylgruppe ist,
x die Anzahl an Wiederholungen ist und unabhängig eine ganze Zahl von 1 bis 4 darstellt, und mindestens ein x eine ganze Zahl von 2 bis 4 ist; y die Anzahl an Substitutionen ist und unabhängig eine ganze Zahl von 1 bis 4 darstellt; und wenn x und y 2 oder mehr ist, eine Mehrzahl an Carbazolylgruppen gleich oder verschieden sein kann; und z eine ganze Zahl von 0 bis 3 ist.

10. Die organische Elektrolumineszenzvorrichtung gemäß einem der Ansprüche 1 bis 9, wobei das dritte Wirtsmaterial eine Verbindung ist, die durch die allgemeine Formel (5) dargestellt ist: 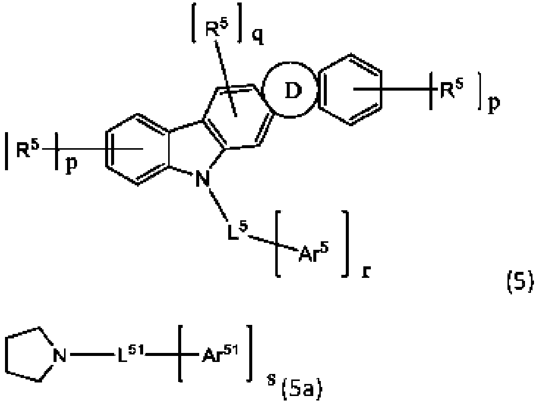
wobei ein Ring D ein Heterocyclus ist, der durch Formel (5a) dargestellt ist, und der Ring D an einer beliebigen Position an einen benachbarten Ring anelliert ist,
R⁵ unabhängig Deuterium, eine aliphatische Kohlenwasserstoffgruppe mit 1 bis 10 Kohlenstoffatomen, eine substituierte oder unsubstituierte aromatische Kohlenwasserstoffgruppe mit 6 bis 30 Kohlenstoffatomen, eine substituierte oder unsubstituierte aromatische heterocyclische Gruppe mit 3 bis 17 Kohlenstoffatomen oder eine substituierte oder unsubstituierte verknüpfte aromatische Gruppe, in der zwei bis fünf dieser aromatischen Ringe miteinander verknüpft sind, darstellt,
p, r und s jeweils unabhängig eine ganze Zahl von 0 bis 4 darstellen und q eine ganze Zahl von 0 bis 2 darstellt,
L⁵ und L⁵¹ unabhängig eine substituierte oder unsubstituierte aromatische Kohlenwasserstoffgruppe mit 6 bis 30 Kohlenstoffatomen oder eine substituierte oder unsubstituierte aromatische heterocyclische Gruppe mit 3 bis 17 Kohlenstoffatomen darstellen und mindestens eines davon die aromatische heterocyclische Gruppe darstellt, und
Ar⁵ und Ar⁵¹ jeweils unabhängig eine substituierte oder unsubstituierte aromatische Kohlenwasserstoffgruppe mit 6 bis 30 Kohlenstoffatomen, eine substituierte oder unsubstituierte aromatische heterocyclische Gruppe mit 3 bis 17 Kohlenstoffatomen oder eine substituierte oder unsubstituierte verknüpfte aromatische Gruppe, in der zwei bis sieben dieser aromatischen Ringe miteinander verknüpft sind, darstellen.

11. Die organische Elektrolumineszenzvorrichtung gemäß Anspruch 10, wobei mindestens eines von L⁵ und L⁵¹ einen substituierten oder unsubstituierten stickstoffhaltigen 6-gliedrigen Ring oder eine anellierte aromatische heterocyclische Gruppe, die einen stickstoffhaltigen 6-gliedrigen Ring enthält, darstellt.

12. Die organische Elektrolumineszenzvorrichtung gemäß Anspruch 10, wobei das dritte Wirtsmaterial eine Verbindung ist, die durch die folgende Formel (51) dargestellt ist: 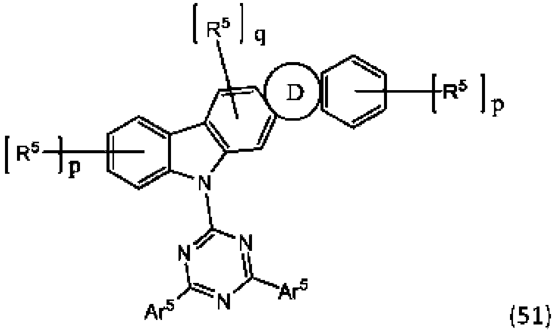 wobei R⁵, D, Ar⁵, p und q wie für die allgemeine Formel (5) definiert sind.

13. Die organische Elektrolumineszenzvorrichtung gemäß einem der Ansprüche 1 bis 12, wobei ein Anteil des ersten Wirtsmaterials größer als 1,0 Gew.-% und weniger als 30 Gew.-% ist und ein Anteil des dritten Wirtsmaterials größer als 5,0 Gew.-% und weniger als 80 Gew.-% ist, bezogen auf das erste Wirtsmaterial, das zweite Wirtsmaterial und das dritte Wirtsmaterial insgesamt.

14. Ein Verfahren zur Herstellung einer organischen Elektrolumineszenzvorrichtung, die eine oder mehrere lichtemittierende Schichten (5) zwischen einer Anode (2) und einer Kathode (7), die einander gegenüberliegen, umfasst, wobei mindestens eine der lichtemittierenden Schichten (5) ein Wirtsmaterial umfasst, das ein erstes Wirtsmaterial, ein zweites Wirtsmaterial und ein drittes Wirtsmaterial sowie ein lichtemittierendes Dotierungsmaterial umfasst, wobei das Verfahren Bilden jeder der lichtemittierenden Schichten (5) durch Abscheiden des lichtemittierenden Dotierungsmaterials und einer Wirtsmaterialzusammensetzung aus der Gasphase umfasst, in welchem die LUMO-Energie des ersten Wirtsmaterials -1,95 eV oder weniger beträgt, die LUMO-Energie des zweiten Wirtsmaterials -1,54 eV oder mehr beträgt und LM2 ≥ LM3 ≥ LM1 erfüllt ist, unter der Annahme, dass die LUMO-Energien des ersten Wirtsmaterials, des zweiten Wirtsmaterials und des dritten Wirtsmaterials LM1, LM2 bzw. LM3 sind, wobei die LUMO-Energie des ersten Wirtsmaterials, des zweiten Wirtsmaterials und des dritten Wirtsmaterials gemäß den Beispielen der Beschreibung berechnet wird, **dadurch gekennzeichnet, dass** die LUMO-Energie des dritten Wirtsmaterials -1,94 bis -1,77 eV beträgt.

15. Ein Verfahren zur Herstellung der organischen Elektrolumineszenzvorrichtung gemäß Anspruch 14, bei der es sich um eine organische Elektrolumineszenzvorrichtung handelt, die eine oder mehrere lichtemittierende Schichten (5) zwischen einer Anode (2) und einer Kathode (7), die einander gegenüberliegen, umfasst, wobei mindestens eine der lichtemittierenden Schichten (5) ein erstes Wirtsmaterial, ein zweites Wirtsmaterial und ein drittes Wirtsmaterial sowie ein lichtemittierendes Dotierungsmaterial umfasst, wobei mindestens drei in den lichtemittierenden Schichten (5) enthaltene Materialien aus einer Quelle für die Gasphasenabscheidung aus der Gasphase abgeschieden werden.

16. Das Verfahren gemäß Anspruch 15, wobei das erste Wirtsmaterial, das zweite Wirtsmaterial und das dritte Wirtsmaterial aus einer Quelle für die Gasphasenabscheidung aus der Gasphase abgeschieden werden.

17. Verwendung einer Wirtsmaterialzusammensetzung in dem Verfahren zur Herstellung einer organischen Elektrolumineszenzvorrichtung gemäß einem der Ansprüche 14 bis 16, wobei die Wirtsmaterialzusammensetzung ein erstes Wirtsmaterial, ein zweites Wirtsmaterial und ein drittes Wirtsmaterial sowie ein lichtemittierendes Dotierungsmaterial umfasst, die LUMO-Energie des ersten Wirtsmaterials -1,95 eV oder weniger beträgt, die LUMO-Energie des zweiten Wirtsmaterials -1,54 eV oder mehr beträgt und LM2 ≥ LM3 ≥ LM1 erfüllt ist, unter der Annahme, dass die LUMO-Energien des ersten Wirtsmaterials, des zweiten Wirtsmaterials und des dritten Wirtsmaterials LM1, LM2 bzw. LM3 sind, wobei die LUMO-Energie des ersten Wirtsmaterials, des zweiten Wirtsmaterials und des dritten Wirtsmaterials wie in den Beispielen der Beschreibung dargelegt berechnet wird, **dadurch gekennzeichnet, dass** die LUMO-Energie des dritten Wirtsmaterials -1,94 bis -1,77 eV beträgt.

18. Verwendung einer Wirtsmaterialzusammensetzung umfassend ein erstes Wirtsmaterial, ein zweites Wirtsmaterial und ein drittes Wirtsmaterial sowie ein lichtemittierendes Dotierungsmaterial, wobei die LUMO-Energie des ersten Wirtsmaterials -1,95 eV oder weniger beträgt, die LUMO-Energie des zweiten Wirtsmaterials -1,54 eV oder mehr beträgt und LM2 ≥ LM3 ≥ LM1 erfüllt ist, unter der Annahme, dass die LUMO-Energien des ersten Wirtsmaterials, des zweiten Wirtsmaterials und des dritten Wirtsmaterials LM1, LM2 bzw. LM3 sind, wobei die LUMO-Energie des ersten Wirtsmaterials, des zweiten Wirtsmaterials und des dritten Wirtsmaterials wie in den Beispielen der Beschreibung dargelegt berechnet wird, in einer organischen Elektrolumineszenzvorrichtung, die eine oder mehrere lichtemittierende Schichten (5) zwischen einer Anode (2) und einer Kathode (7), die einander gegenüberliegen, umfasst, wobei mindestens eine der lichtemittierenden Schichten (5) das Wirtsmaterial enthält, **dadurch gekennzeichnet, dass** die LUMO-Energie des dritten Wirtsmaterials -1,94 bis -1,77 eV beträgt.

## Revendications

1. Dispositif électroluminescent organique comprenant une ou plusieurs couches luminescentes (5) entre une anode (2) et une cathode (7) opposées l'une à l'autre, dans lequel au moins une des couches luminescentes (5) contient un matériau hôte comprenant un premier matériau hôte, un deuxième matériau hôte et un troisième matériau hôte, et un matériau dopant luminescent, l'énergie LUMO du premier matériau hôte est de -1,95 eV ou moins, l'énergie LUMO du deuxième matériau hôte est de -1,54 eV ou plus, et l'inégalité LM2 ≥ LM3 ≥ LM1 est satisfaite étant supposé que les énergies LUMO du premier matériau hôte, du deuxième matériau hôte et du troisième matériau hôte sont respectivement LM1, LM2 et LM3, dans lequel l'énergie LUMO du premier matériau hôte, du deuxième matériau hôte et du troisième matériau hôte est calculée comme indiqué dans les exemples de la description, **caractérisé en ce que** l'énergie LUMO du troisième matériau hôte est de -1,94 à -1,77 eV.

2. Dispositif électroluminescent organique selon la revendication 1, dans lequel les énergies d'excitation triplet (T1) du premier matériau hôte, du deuxième matériau hôte et du troisième matériau hôte sont chacune de 2,55 eV ou plus.

3. Dispositif électroluminescent organique selon la revendication 1 ou 2, dans lequel le matériau dopant luminescent est un matériau émettant de la fluorescence incluant un matériau à fluorescence retardée thermiquement activée.

4. Dispositif électroluminescent organique selon l'une quelconque des revendications 1 à 3, dans lequel le matériau dopant luminescent est un matériau phosphorescent.

5. Dispositif électroluminescent organique selon l'une quelconque des revendications 1 à 4, dans lequel le premier matériau hôte est un composé représenté par la formule générale (1) suivante : 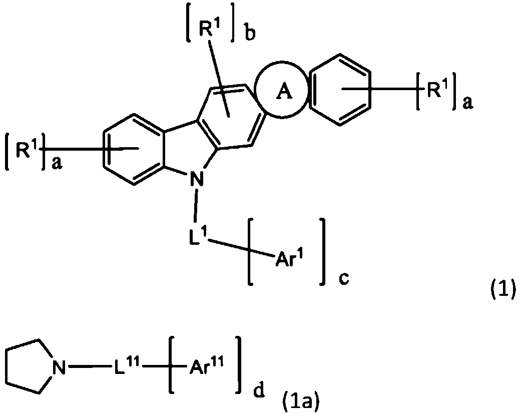
dans laquelle le cycle A est un hétérocycle représenté par la formule (1a) et le cycle A est condensé à un cycle adjacent en n'importe quelle position,
R¹ représente indépendamment un deutérium, un groupe hydrocarboné aliphatique ayant 1 à 10 atomes de carbone, un groupe hydrocarboné aromatique substitué ou non substitué ayant 6 à 30 atomes de carbone, un groupe hétérocyclique aromatique substitué ou non substitué ayant 3 à 17 atomes de carbone, ou un groupe aromatique lié substitué ou non substitué dans lequel deux à cinq de ces cycles aromatiques sont liés entre eux,
chacun de a, c et d représente indépendamment un entier de 0 à 4, et b représente un entier de 0 à 2,
L¹ et L¹¹ représentent indépendamment un groupe hydrocarboné aromatique substitué ou non substitué ayant 6 à 30 atomes de carbone, ou un groupe hétérocyclique aromatique substitué ou non substitué ayant 3 à 17 atomes de carbone, et au moins l'un d'eux représente le groupe hétérocyclique aromatique, et
chacun de Ar¹ et Ar¹¹ représente indépendamment un groupe hydrocarboné aromatique substitué ou non substitué ayant 6 à 30 atomes de carbone, un groupe hétérocyclique aromatique substitué ou non substitué ayant 3 à 17 atomes de carbone, ou un groupe aromatique lié substitué ou non substitué dans lequel deux à sept de ces cycles aromatiques sont liés entre eux.

6. Dispositif électroluminescent organique selon la revendication 5, dans lequel au moins l'un parmi L¹ et L¹¹ est un groupe cyclique azoté à 6 chaînons substitué ou non substitué, ou un groupe hétérocyclique aromatique à cycles condensés substitué ou non substitué contenant un cycle azoté à 6 chaînons.

7. Dispositif électroluminescent organique selon la revendication 5, dans lequel la formule générale (1) est la formule (11) suivante : 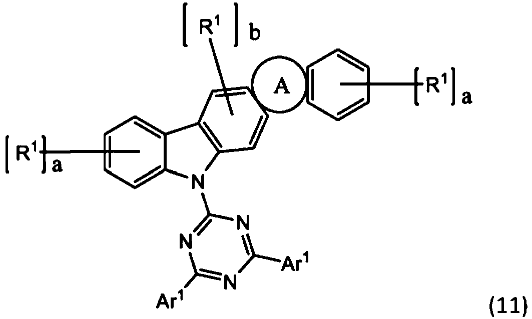 dans laquelle R¹, le cycle A, Ar¹, a et b sont tels que définis pour la formule générale (1).

8. Dispositif électroluminescent organique selon la revendication 7, dans lequel la formule (11) est l'une des formules (12) et (13) suivantes : 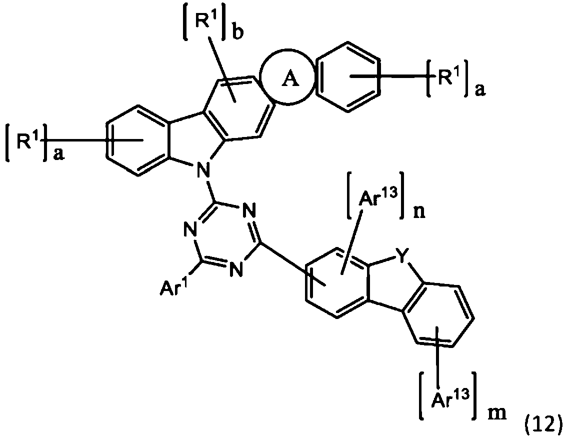 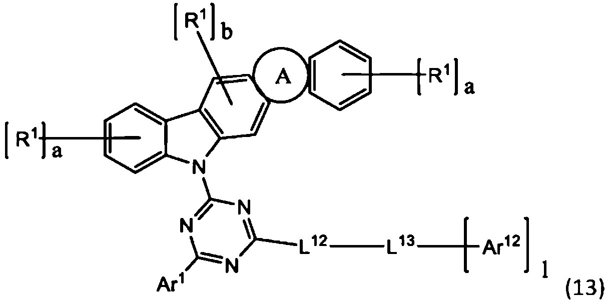
dans lesquelles R¹, le cycle A, Ar¹, a et b sont tels que définis pour la formule générale (1),
Y représente O, S, NAr¹⁴ ou CAr¹⁵Ar¹⁶,
chacun de Ar¹³, Ar¹⁴, Ar¹⁵ et Ar¹⁶ représente indépendamment un groupe hydrocarboné aliphatique ayant 1 à 10 atomes de carbone, un groupe hydrocarboné aromatique substitué ou non substitué ayant 6 à 30 atomes de carbone, un groupe hétérocyclique aromatique substitué ou non substitué ayant 3 à 17 atomes de carbone, ou un groupe aromatique lié substitué ou non substitué dans lequel deux à cinq de ces cycles aromatiques sont liés entre eux,
m représente un entier de 0 à 4, n représente un entier de 0 à 3 et l représente un entier de 0 à 4,
L¹² représente un groupe hydrocarboné aromatique substitué ou non substitué ayant 6 à 30 atomes de carbone,
L¹³ représente un groupe cyclique azoté à 6 chaînons, et
Ar¹² représente indépendamment un groupe hydrocarboné aromatique substitué ou non substitué ayant 6 à 30 atomes de carbone, un groupe hétérocyclique aromatique substitué ou non substitué ayant 3 à 17 atomes de carbone, ou un groupe aromatique lié substitué ou non substitué dans lequel deux à cinq de ces cycles aromatiques sont liés entre eux.

9. Dispositif électroluminescent organique selon l'une quelconque des revendications 1 à 8, dans lequel le deuxième matériau hôte est un composé représenté par la formule générale (2), la formule générale (3) ou la formule générale (4) suivantes : 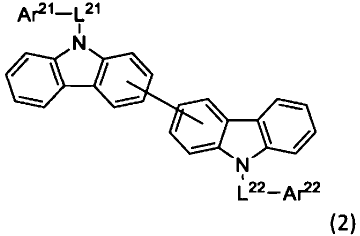
dans laquelle Ar²¹ et Ar²² représentent indépendamment un hydrogène, un deutérium, un groupe hydrocarboné aromatique substitué ou non substitué ayant 6 à 14 atomes de carbone, un groupe hétérocyclique aromatique substitué ou non substitué ayant 3 à 17 atomes de carbone, ou un groupe aromatique lié substitué ou non substitué dans lequel deux des cycles aromatiques sont liés entre eux ; et L²¹ et L²² représentent indépendamment une liaison directe ou un groupe phénylène ; 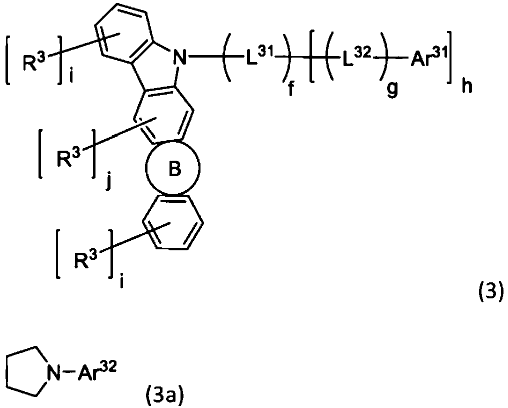
dans laquelle le cycle B est un hétérocycle représenté par la formule (3a) et le cycle B est condensé à un cycle adjacent en n'importe quelle position,
R³ représente indépendamment un deutérium, un groupe hydrocarboné aliphatique ayant 1 à 10 atomes de carbone, un groupe hydrocarboné aromatique substitué ou non substitué ayant 6 à 30 atomes de carbone, un groupe hétérocyclique aromatique substitué ou non substitué ayant 3 à 17 atomes de carbone, ou un groupe aromatique lié substitué ou non substitué dans lequel deux à cinq de ces cycles aromatiques sont liés entre eux,
L³¹ représente indépendamment un groupe hydrocarboné aromatique substitué ou non substitué ayant 6 à 30 atomes de carbone, ou un groupe hétérocyclique aromatique substitué ou non substitué ayant 3 à 17 atomes de carbone,
L³² représente indépendamment un groupe hydrocarboné aromatique substitué ou non substitué ayant 6 à 10 atomes de carbone,
Ar³¹ représente un groupe hydrocarboné aromatique substitué ou non substitué ayant 6 à 10 atomes de carbone, un groupe carbazolyle substitué ou non substitué, ou un groupe aromatique lié substitué ou non substitué dans lequel deux à cinq de ces cycles aromatiques sont liés entre eux,
Ar³² représente un groupe hydrocarboné aromatique substitué ou non substitué ayant 6 à 30 atomes de carbone, un groupe hétérocyclique aromatique substitué ou non substitué ayant 3 à 17 atomes de carbone, ou un groupe aromatique lié substitué ou non substitué dans lequel deux à cinq de ces cycles aromatiques sont liés entre eux,
i représente un entier de 0 à 4, et j représente un entier de 0 à 2,
f est le nombre de répétitions, et représente un entier de 1 à 3, g est le nombre de répétitions et représente indépendamment un entier de 0 à 3, et h représente le nombre de substitutions et représente un entier de 0 à 7 ; 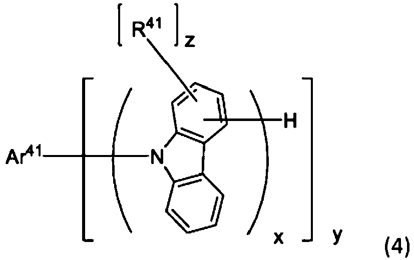
dans laquelle Ar⁴¹ représente un groupe hydrocarboné aromatique substitué ou non substitué ayant 6 à 30 atomes de carbone, un groupe hétérocyclique aromatique substitué ou non substitué ayant 3 à 17 atomes de carbone, ou un groupe aromatique lié dans lequel deux à cinq de ces cycles aromatiques sont liés ; quand Ar⁴¹ a un atome d'hydrogène, l'atome d'hydrogène peut être remplacé par du deutérium,
chaque R⁴¹ représente indépendamment un deutérium, un groupe hydrocarboné aliphatique ayant 1 à 10 atomes de carbone, un groupe hydrocarboné aromatique substitué ou non substitué ayant 6 à 30 atomes de carbone, ou un groupe hétérocyclique aromatique substitué ou non substitué ayant 3 à 17 atomes de carbone, sous réserve que R⁴¹ ne soit pas un groupe carbazolyle,
x est le nombre de répétitions et représente indépendamment un entier de 1 à 4, et au moins un x est un entier de 2 à 4 ; y est le nombre de substitutions et représente indépendamment un entier de 1 à 4 ; et quand x et y valent 2 ou plus, plusieurs groupes carbazolyle peuvent être identiques ou différents ; et z est un entier de 0 à 3.

10. Dispositif électroluminescent organique selon l'une quelconque des revendications 1 à 9, dans lequel le troisième matériau hôte est un composé représenté par la formule générale (5) : 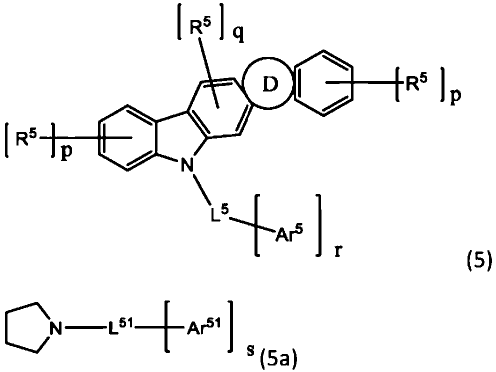
dans laquelle le cycle D est un hétérocycle représenté par la formule (5a) et le cycle D est condensé à un cycle adjacent en n'importe quelle position,
R⁵ représente indépendamment un deutérium, un groupe hydrocarboné aliphatique ayant 1 à 10 atomes de carbone, un groupe hydrocarboné aromatique substitué ou non substitué ayant 6 à 30 atomes de carbone, un groupe hétérocyclique aromatique substitué ou non substitué ayant 3 à 17 atomes de carbone, ou un groupe aromatique lié substitué ou non substitué dans lequel deux à cinq de ces cycles aromatiques sont liés entre eux,
chacun de p, r et s représente indépendamment un entier de 0 à 4, et q représente un entier de 0 à 2,
L⁵ et L⁵¹ représentent indépendamment un groupe hydrocarboné aromatique substitué ou non substitué ayant 6 à 30 atomes de carbone, ou un groupe hétérocyclique aromatique substitué ou non substitué ayant 3 à 17 atomes de carbone, et au moins l'un d'eux représente le groupe hétérocyclique aromatique, et
chacun de Ar⁵ et Ar⁵¹ représente indépendamment un groupe hydrocarboné aromatique substitué ou non substitué ayant 6 à 30 atomes de carbone, un groupe hétérocyclique aromatique substitué ou non substitué ayant 3 à 17 atomes de carbone, ou un groupe aromatique lié substitué ou non substitué dans lequel deux à sept de ces cycles aromatiques sont liés entre eux.

11. Dispositif électroluminescent organique selon la revendication 10, dans lequel au moins l'un parmi L⁵ et L⁵¹ représente un cycle azoté à 6 chaînons substitué ou non substitué ou un groupe hétérocyclique aromatique à cycles condensés , contenant un cycle azoté à 6 chaînons.

12. Dispositif électroluminescent organique selon la revendication 10, dans lequel le troisième matériau hôte est un composé représenté par la formule (51) suivante : 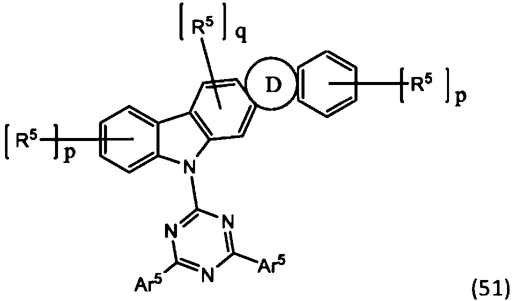 dans laquelle R⁵, D, Ar⁵, p et q sont tels que définis pour la formule générale (5).

13. Dispositif électroluminescent organique selon l'une quelconque des revendications 1 à 12, dans lequel la proportion du premier matériau hôte est supérieure à 1,0 % en poids et inférieure à 30 % en poids et la proportion du troisième matériau hôte est supérieure à 5,0 % en poids et inférieure à 80 % en poids par rapport au total du premier matériau hôte, du deuxième matériau hôte et du troisième matériau hôte.

14. Méthode pour produire un dispositif électroluminescent organique comprenant une ou plusieurs couches luminescentes (5) entre une anode (2) et une cathode (7) opposées l'une à l'autre, au moins une des couches luminescentes (5) comprenant un matériau hôte comprenant un premier matériau hôte, un deuxième matériau hôte et un troisième matériau hôte, et un matériau dopant luminescent, la méthode comprenant la formation de chacune des couches luminescentes (5) par dépôt en phase vapeur du matériau dopant luminescent et d'une composition de matériau hôte, dans laquelle l'énergie LUMO du premier matériau hôte est de -1,95 eV ou moins, l'énergie LUMO du deuxième matériau hôte est de -1,54 eV ou plus, et l'inégalité LM2 ≥ LM3 ≥ LM1 est satisfaite étant supposé que les énergies LUMO du premier matériau hôte, du deuxième matériau hôte et du troisième matériau hôte sont respectivement LM1, LM2 et LM3, dans laquelle l'énergie LUMO du premier matériau hôte, du deuxième matériau hôte et du troisième matériau hôte est calculée comme indiqué dans les exemples de la description, **caractérisée en ce que** l'énergie LUMO du troisième matériau hôte est de -1,94 à -1,77 eV.

15. Méthode pour produire un dispositif électroluminescent organique selon la revendication 14, dans laquelle il s'agit d'un dispositif électroluminescent organique comprenant une ou plusieurs une ou plusieurs couches luminescentes (5) entre une anode (2) et une cathode (7) opposées l'une à l'autre, dans laquelle au moins une des couches luminescentes (5) contient un premier matériau hôte, un deuxième matériau hôte et un troisième matériau hôte, et un matériau dopant luminescent, dans laquelle au moins trois matériaux contenus dans les couches luminescentes (5) sont déposés en phase vapeur à partir d'une seule source de dépôt en phase vapeur.

16. Méthode selon la revendication 15, dans laquelle le premier matériau hôte, le deuxième matériau hôte et le troisième matériau hôte sont déposés en phase vapeur à partir d'une seule source de dépôt en phase vapeur.

17. Utilisation d'une composition de matériau hôte dans la méthode pour produire un dispositif électroluminescent organique selon l'une quelconque des revendications 14 à 16, dans laquelle la composition de matériau hôte comprend un premier matériau hôte, un deuxième matériau hôte et un troisième matériau hôte, et un matériau dopant luminescent, l'énergie LUMO du premier matériau hôte est de -1,95 eV ou moins, l'énergie LUMO du deuxième matériau hôte est de -1,54 eV ou plus, et l'inégalité LM2 ≥ LM3 ≥ LM1 est satisfaite étant supposé que les énergies LUMO du premier matériau hôte, du deuxième matériau hôte et du troisième matériau hôte sont respectivement LM1, LM2 et LM3, dans laquelle l'énergie LUMO du premier matériau hôte, du deuxième matériau hôte et du troisième matériau hôte est calculée comme indiqué dans les exemples de la description, **caractérisée en ce que** l'énergie LUMO du troisième matériau hôte est de -1,94 à -1,77 eV.

18. Utilisation d'une composition de matériau hôte comprenant un premier matériau hôte, un deuxième matériau hôte et un troisième matériau hôte, et un matériau dopant luminescent, l'énergie LUMO du premier matériau hôte étant de -1,95 eV ou moins, l'énergie LUMO du deuxième matériau hôte étant de -1,54 eV ou plus, et l'inégalité LM2 ≥ LM3 ≥ LM1 étant satisfaite étant supposé que les énergies LUMO du premier matériau hôte, du deuxième matériau hôte et du troisième matériau hôte sont respectivement LM1, LM2 et LM3, dans laquelle l'énergie LUMO du premier matériau hôte, du deuxième matériau hôte et du troisième matériau hôte est calculée comme indiqué dans les exemples de la description, dans un dispositif électroluminescent organique comprenant une ou plusieurs couches luminescentes (5) entre une anode (2) et une cathode (7) opposées l'une à l'autre, dans laquelle au moins une des couches luminescentes (5) contient le matériau hôte, **caractérisée en ce que** l'énergie LUMO du troisième matériau hôte est de -1,94 à -1,77 eV.
